(19) Europäisches Patentamt
European Patent Office
Office européen des brevets

(11) **EP 4 484 378 A1**

(12) **EUROPEAN PATENT APPLICATION**
published in accordance with Art. 153(4) EPC

(43) Date of publication:
**01.01.2025 Bulletin 2025/01**

(21) Application number: **23759833.9**

(22) Date of filing: **15.02.2023**

(51) International Patent Classification (IPC):
**C01F 17/235** (2020.01)    **B82Y 30/00** (2011.01)
**B82Y 40/00** (2011.01)

(52) Cooperative Patent Classification (CPC):
**B82Y 30/00; B82Y 40/00; C01F 17/235;**
Y02P 20/54

(86) International application number:
**PCT/JP2023/005326**

(87) International publication number:
**WO 2023/162832 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.02.2022 JP 2022026912**

(71) Applicant: **Super Nano Design Co.,Ltd Sendai-shi Miyagi 980-8579 (JP)**

(72) Inventors:
• **AJIRI, Tadafumi**
  **Sendai-shi Miyagi 980-8577 (JP)**
• **TOMAI, Takaaki**
  **Sendai-shi Miyagi 980-8577 (JP)**
• **YOKO, Akira**
  **Sendai-shi Miyagi 980-8577 (JP)**
• **SEI, Kimei**
  **Sendai-shi Miyagi 980-8577 (JP)**

(74) Representative: **Hoffmann Eitle Patent- und Rechtsanwälte PartmbB Arabellastraße 30 81925 München (DE)**

(54) **METHOD FOR PRODUCING METAL OXIDE NANO-PARTICLES, AND METAL OXIDE NANO-PARTICLES**

(57)    The present invention enables us to achieve both further fine particle size reduction and uniformity of particle size distribution of metal oxide nanoparticles.

The present invention is a method for producing metal oxide nanoparticles that consists of a process for obtaining metal oxide nanoparticles by mixing a supercritical, subcritical, or gas phase aqueous material and an organometallic complex solution, wherein the mixing time is controllable within the range of 0.015 s to 380 s and the diameter of at least one of the average primary particle diameter or the crystallite diameter of the nanoparticles can be controlled within the range of 1.0 nm to 9.0 nm, and the coefficient of variation of the diameter can be controlled within 0.5 nm or less by controlling the mixing time. The resulting nanoparticles encompass metal elements capable of forming organometallic complexes. Additionally, the organic molecules are strongly bonded to the most unstable surface.

TEM images of cerium oxide (CeO$_2$) nanoparticles for Examples 1-1 to 1-8

Fig. 5

**EP 4 484 378 A1**

**Description**

TECHNICAL FIELD

The present invention relates to a method for producing metal oxide nanoparticles and metal oxide nanoparticles.

BACKGROUND ART

Fine particles, especially ones that are nanometer-sized (nanoparticles), exhibit a variety of unique and superior properties, characteristics, and functions and are expected to enable technologies that meet the current demand for higher precision, smaller size, and lighter weight in all materials and products. Thus, nanoparticles are attracting attention by enabling high functionality, high performance, high density, and high precision in industrial materials, such as nanostructure modification materials for ceramics, optical function coating materials, electromagnetic wave shielding materials, secondary battery materials, fluorescent materials, electronic component materials, magnetic recording materials, abrasive materials, and pharmaceutical and cosmetic materials. Recent basic research on nanoparticles has led to a series of discoveries of ultrahigh functionality, the expression of new physical properties, and the synthesis of new materials due to the quantum size effect of nanoparticles, which have attracted great interest from the industrial world.

Furthermore, lattice distortion below 10 nm has recently been found, especially when unstable surfaces are exposed, and ionic conduction and electronic state changes due to this distortion have also been discovered. However, the technology for synthesizing nanoparticles of 10 nm or less in large quantities with controlled exposure surfaces at industrially meaningful productivity levels has not been achieved by existing methods. The method of synthesizing organically modified nanoparticles in supercritical water is the only approach that enables the mass synthesis of nanoparticles with organic modification. However, with the conventional continuous synthesis method, it is not easy to achieve the strong organic modification of nanoparticles of 10 nm or less, and especially 5 nm or less, with uniform particle size and surface exposure control. The precise control of flow conditions and precursor formation in a distribution-type reaction apparatus is indispensable for achieving this and adding functions specific to fine particles, but optimal conditions have not been established. Under such optimized conditions, organic modification would be a convenient way to add functions to fine particles, especially nanoparticles, that could be used and utilized stably. In particular, modification through strong bonding is required.

Although not optimized for the synthesis of nanoparticles below 10 nm, and especially below 5 nm, the mass continuous synthesis of organically modified nanoparticles is an approach for recovering or collecting metal oxide nanoparticles using high temperature and high-pressure water as a reaction field. In this approach, metal oxide nanoparticle surfaces are reacted with an organic modifier to form covalent bonds-namely, ether bonds, bonds via N atoms and S atoms, and metal-C- bonds, which may be substituted or unsubstituted hydrocarbon groups. The surfaces of the nanoparticles are organically modified by being combined with the surfaces of the nanoparticles via a bond selected from the group consisting of a metal-C= bond and a metal-(C=O)- bond, precipitating the metal oxide nanoparticles dispersed in an aqueous solution and collecting them by transferring them into an organic solvent or collecting those at the interface between the organic solvent phase and the aqueous phase (Patent Document 1).

PRIOR ART DOCUMENT

PATENT DOCUMENT

Patent Document 1: JP3925936B

SUMMARY OF THE INVENTION

PROBLEM TO BE SOLVED BY THE INVENTION

In recent years, there has been growing demand for the further miniaturization of nanoparticles, and there have been calls for particles to have a uniform size distribution. In addition, there is a need to expose unstable surfaces for the purpose of reactivity and ion/electron conductivity.

In view of these problems, the present invention was designed to further reduce the fine particle size and achieve uniformity in the size distribution of metal oxide nanoparticles.

MEANS OF SOLVING THE PROBLEM

Through diligent research, the inventors established conditions for synthesis under a reaction rate by setting the mixing speed as greater than the reaction speed in a system that performs rapid temperature increases and reactions by mixing raw materials and heated water in a distribution-type reaction apparatus and using an organometallic complex as the reaction raw material for supercritical hydrothermal synthesis. Furthermore, by using an organometallic complex in this way and setting the reaction time to be within a specific range, at least one of the average primary particle diameter or the crystallite diameter of the generated metal oxide nanoparticles can be controlled within the range of 1.0 nm to 9.0 nm, and the coefficient of variation of the primary average particle diameter can be controlled at 0.5 or less, thus completing this invention. Specifically, the present invention provides the following.

According to a first embodiment, the invention provides a method of producing metal oxide nanoparticles comprising: a mixing process for obtaining metal oxide nanoparticles by mixing a supercritical, subcritical, or gas phase aqueous material and an organometallic complex solution, wherein the mixing time is controllable within a range from 0.015 seconds to 380 seconds, and at least one of the average primary particle diameter or the crystallite diameter of the nanoparticles can be controlled within the range of 1.0 nm to 9.0 nm, and the coefficient of variation of the diameter can be controlled at 0.5 nm or less by controlling the mixing time.

When a supercritical, subcritical, or vapor-phase aqueous material is mixed with an organometallic complex solution, the organometallic complex is hydrolyzed, and the hydrolyzed organometallic complex salt is instantly dehydrated without oxidation to produce metal oxide crystals within a short time period of between 0.015 and 380 s. Because the organometallic complex solution is homogeneous, uniform nanoparticles can be generated from the salt without forming bulk hydroxides.

According to the invention's first feature, at a synthesis concentration of 0.1 g/l or higher and a reaction rate of 0.8 or higher, it is possible to reduce the fine particle size and achieve uniformity in the size distribution of metal oxide nanoparticles.

According to a second embodiment, the invention provides a method wherein the diameter and the coefficient of variation of the nanoparticles can be controlled by controlling the molar ratio of the organometallic complex to the organic material with respect to the metal constituting the organometallic complex, according to the first embodiment.

According to the invention's second feature, by controlling the molar ratio, it is possible to achieve further microparticulation and uniformity in the size distribution of metal oxide nanoparticles.

According to a third embodiment, the invention provides a method wherein the mixing temperature is controllable within a range from 300°C to 450°C, and the diameter and the said coefficient of variation of the nanoparticles can be controlled by controlling the mixing temperature, according to the first or the second embodiment.

According to the invention's third feature, by controlling the temperature, it is possible to further reduce the size and achieve uniformity in the size distribution of metal oxide nanoparticles.

According to a fourth embodiment, the invention provides a method wherein when the metal elements comprising the organometallic complexes are metal elements that can take on multiple types of valence(s), the valence(s) of the metal elements comprising the organometallic complexes in said solution is controlled to be the same as that of the metal elements of the product, according to any of the first to the third embodiments.

According to the invention's fourth feature, by controlling the valence of the metal element constituting the organometallic complex that serves as the raw material, it is possible to achieve further microparticulation and a more uniform size distribution of the metal oxide nanoparticles with greater rigor.

According to a fifth embodiment, the invention provides a method wherein the mixing process is a mixing process using a continuous reactor, and the mixing time can be controlled within a range of 1 second or less by setting the Reynolds number (Re) to 3000 or more, according to any of the first to the fourth embodiments.

According to the fifth feature, the Re number of the mixing section (i.e., the mixing time) can be controlled to within 1 s by changing the volume of the continuous reactor and the flow rate of the mixed raw material-that is, the aqueous material and the organometallic complex solution-supplied to the continuous reactor. This makes it possible to further reduce the size and achieve uniformity in the size distribution of metal oxide nanoparticles in a continuous production system as well as in batch production.

The higher the Re number of the mixing section, the more the mixing rate increases and becomes the reaction rate, thereby enabling uniform particle synthesis with smaller particles. The mixing rate can be evaluated using Kolmogorov's theory. According to the invention's fifth feature, the reaction rate can be evaluated from a small number of experimental points, and a reactor that limits the reaction rate can be designed.

According to a sixth embodiment, the invention provides a method wherein a mixing speed $k_{mix}$ is obtained using Kolmogorov's theory, the true reaction rate $k$ is obtained from $1/k = 1/k_{app} - 1/k_{mix}$ using the mixing rate $k_{mix}$ and the apparent reaction rate $k_{app}$, and the mixing time can be controlled within the range of 1 second or less by setting the Damkeller number $Da = k/k_{mix} \ll 1$ and the Re to 3000 or more, according to the fifth embodiment.

According to the sixth feature, the reaction rate can be evaluated from a very small number of experimental points (i.e., 1-2 points), and a reactor can be designed to be reaction-rate limited.

According to a seventh embodiment, the invention provides a method further comprising a synthesis process for synthesizing the organometallic complexes, wherein the synthesis process and the mixing process are continuous processes, according to the fifth or sixth embodiment.

According to the invention's seventh feature, a series of processes from the synthesis of precursors to the synthesis of metal oxide nanoparticles can be realized in a continuous manufacturing system.

According to an eighth embodiment, the invention provides a method further comprising a washing process in which the organometallic complexes are removed by washing the mixed product using the mixing process, according to one of the first to the seventh embodiments.

According to the invention's eighth feature, metal oxide nanoparticles, which are free from residual organometallic complexes (unreacted substances), can be provided with a size of 9.0 nm or less and a uniform particle size distribution.

According to a ninth embodiment, the invention provides a metal oxide nanoparticle comprising: a diameter of at least one of the average primary particle diameter or the crystallite diameter being between 1.0 nm and 9.0 nm, and the coefficient of variation of the diameter being 0.5 or less, and the metal element constituting the metal oxide being a metal element capable of forming an organometallic complex.

According to a tenth embodiment, the invention provides an organically modified metal oxide nanoparticle comprising: a diameter of at least one of the average primary particle diameter or the crystallite diameter being between 1.0 nm and 9.0 nm, and the coefficient of variation of the diameter being 0.5 or less, and organic molecules being strongly bound to the most unstable surface.

EFFECT OF THE INVENTION

The present invention makes it possible to further refine metal oxide nanoparticles and achieve uniformity in their size distribution.

BRIEF DESCRIPTION OF DRAWINGS

Fig. 1 shows a schematic diagram of the method described in this embodiment when realized in a continuous reaction system.

Fig. 2 shows a schematic diagram of the preparation of organometallic complexes.

Fig. 3 shows a schematic diagram of a contact zone where a raw material solution containing an organometallic complex and an aqueous material in a supercritical state or the like are in contact.

Fig. 4 shows a schematic diagram of the recovery of products after the completion of the hydrothermal synthesis reaction in this test case.

Fig. 5 is a photograph of the nanoparticles in Example 1, as observed with a transmission electron microscope.

Fig. 6 shows a photograph of the nanoparticles in Comparative Example 1, observed with a transmission electron microscope.

Fig. 7 shows the time variation of the crystallite diameter measured from the XRD pattern of the synthesized nanoparticles.

Fig. 8 shows the results of the average volume of particles for each reaction time.

Fig. 9 shows the results of the HR-TEM magnification of the shape of the nanoparticles.

Fig. 10 illustrates the method used to calculate the aspect ratio of the particles from the HR-TEM images.

Fig. 11 shows the relationship between the particle length diameter and the aspect ratio for each reaction time.

Fig. 12 is a schematic diagram of the mechanistic change in particle diameter increase predicted using shape analysis for each reaction time.

Fig. 13A shows the relationship of collision time to particle radius. Fig. 13B shows the theoretical number of collisions at each reaction time plotted against the particle size at each reaction time.

Fig. 14 shows the change in the coefficient of variation of the TEM length diameter with the passage of the reaction time.

Fig. 15 shows the relationship between the molar ratio of octanoic acid to cerium and the average primary particle diameter of cerium oxide ($CeO_2$) nanoparticles produced in Test 2.

Fig. 16 shows a photograph of the nanoparticles in Examples 3-1 and 3-2, observed with a transmission electron microscope.

Fig. 17 shows the effects of the modifier concentration and reaction temperature on organically modified $CeO_2$.

Fig. 18 shows the change in the crystallite diameter versus the reaction time when the modifier concentration and reaction temperature are varied.

Fig. 19 shows a TEM image of the reaction time at 340°C and with OA = 0.52 mol/L.

Fig. 20 shows a TEM image at 300°C with OA = 0.12 mol/L.

Fig. 21A shows the results of calculating the collision frequency factor β for the particle size under different reaction conditions. Fig. 21B shows that the change in the number of particles N versus time t, dN/dt, was obtained using the number of particles before and after the reaction time, for which $N^2$ was obtained.

Fig. 22A shows a linear approximation of the graph in Fig. 21A. Fig. 22B shows an Arrhenius plot obtained using the collision frequency factor β obtained from Fig. 22A.

Fig. 23 shows the relationship between the reaction time to obtain cerium (IV) oxide nanoparticles and the conversion rate to cerium (IV) oxide nanoparticles for Examples 4-1 and 4-2 and Comparative Examples 4-1-4-3, respectively.

Fig. 24 shows the characterization of the precursor obtained in Test 5. Fig. 24A shows the Ce L3-Edge XANES spectra of cerium octanoate (III), cerium octanoate (IV), and cerium oxide (IV). Fig. 24B shows the XRD patterns of cerium octanoate (III), cerium octanoate (IV), and cerium oxide (IV).

Fig. 25 shows the XRD patterns of the products synthesized from cerium octanoate (III) and cerium octanoate (IV).

Fig. 26 shows the FT-IR spectra of the products synthesized from cerium (III) octanoate and cerium (IV) octanoate.

Fig. 27 shows a TEM image of the products. Fig. 27A shows a low-magnification image of the product synthesized from cerium octanoate (III). Fig. 27B shows a high-magnification image of the product synthesized from cerium (III) octanoate. Fig. 27C shows an image of the product synthesized from cerium octanoate (IV).

Fig. 28A shows the TGA pattern of the product synthesized from cerium octanoate (IV). Fig. 28B shows an image of the product dissolved in 1 wt.% cyclohexane.

Fig. 29 shows the XRD pattern of the product synthesized from cerium octanoate (III) with different reaction times.

Fig. 30 shows TEM images of the products synthesized from cerium (III) octanoate at different reaction times. Fig. 30A shows a reaction time of 5 min, Fig. 30B shows a reaction time of 10 min, Fig. 30C shows a reaction time of 60 min, and Fig. 30D shows a reaction time of 360 min.

Fig. 31 shows a schematic diagram of the formation route from cerium compounds to cerium oxide (IV) studied in Test 5.

Fig. 32 shows the effect of octanoic acid on the supercritical hydrothermal treatment of cerium octanoate (III). The Fig. without octanoic acid shows the case where 0.143 g cerium (III) octanoate and 2.5 mL water are added, and the Fig. with octanoic acid shows the case where 0.143 g cerium (III) octanoate, 1.25 mL water, and 1.25 mL octanoic acid are added.

Fig. 33 shows the FT-IR spectra of cerium (III) octanoate, cerium (IV) octanoate, and extracts.

Fig. 34 shows the particle size distribution of $CeO_2$ nanoparticles synthesized from cerium octanoate (IV) and $CeO_2$ nanoparticles synthesized from $Ce(OH)_4$.

Fig. 35 shows a TEM image of cerium oxide (IV) synthesized from $Ce(OH)_4$.

Fig. 36 shows the IR spectrum measurement results of the cerium carboxylate complex (IV) synthesized as a precursor in Test 6.

Fig. 37 shows the XANES spectra of the cerium carboxylate complex (IV) synthesized as a precursor in Test 6.

Fig. 38 shows the XRD pattern of the cerium-carboxylate complex (IV) synthesized as a precursor in Test 6.

Fig. 39 shows the XRD pattern of the cerium carboxylate complex (IV) after supercritical hydrothermal synthesis in Test 6.

Fig. 40 shows the XRD pattern of $C14-CeO_2$ (Example 6-4) after impurity separation.

Fig. 41 shows the variation of synthesized $CeO_2$ nanoparticles versus the reaction time.

Fig. 42 shows a schematic diagram of the effect of the modifier's molecular chain length on the interaction potential.

Fig. 43 is a TEM image of Example 6-1 (hexanoic acid-modified $CeO_2$, $C6-CeO_2$).

Fig. 44 is a TEM image of Example 6-3 (decanoic acid-modified $CeO_2$, $C10-CeO_2$).

Fig. 45 is a TEM image of Example 6-4 (myristic acid-modified $CeO_2$, $C14-CeO_2$).

Fig. 46 shows the results of the evaluation of the crystallite diameter by XRD in Test 7.

Fig. 47 shows a TEM image of nanoparticles at a reaction time of 0.04 s in Test 7.

Fig. 48 shows the relationship between the Reynolds number and the crystallite diameter during the supercritical hydrothermal synthesis reaction in Test 8.

Fig. 49 shows a TEM image of the synthesized particles in Test 8.

Fig. 50 shows the relationship between the lattice constant and the particle diameter in Test 9.

Fig. 51 shows the XRD pattern obtained in Test 9.

Fig. 52 shows the representative XPS spectrum in Test 9.

Fig. 53 shows the relationship between the ratio of $Ce^{3+}$ and the XRD crystallite diameter obtained from XPS in Test 9.

Fig. 54 shows the effect of the crystallite diameter on the X-ray emission spectrum in the O1s region in Test 9.

Fig. 55 shows the XRD patterns of the initial formation and after growth in Test 9.

Fig. 56 shows the variation in the 20° peak intensity ratio versus the crystallite diameter in Test 9.

Fig. 57 shows the relationship between the $Ce_2O_3$ ratio and the crystallite diameter determined by XRD pattern analysis.

Fig. 58 shows an overview of the apparatus used in Test 10.

Fig. 59 shows the reaction rate in $ZrO_2$ synthesis obtained from plasma atomic emission spectrometry (ICP) in Test 10.

Fig. 60 shows a TEM image of the synthesized $ZrO_2$ particles obtained in Test 10 (300°C, OA/Zr = 16).

Fig. 61 shows a TEM image (300°C, OA/Zr = 40) of the synthetic $ZrO_2$ particles obtained in Test 10.

Fig. 62 shows a TEM image (340°C, OA/Zr = 16) of the synthetic $ZrO_2$ particles obtained in Test 10.

Fig. 63 shows a TEM image (340°C, OA/Zr = 40) of the synthetic $ZrO_2$ particles obtained in Test 10.

Fig. 64 shows a TEM image (380°C, OA/Zr = 16) of the synthetic $ZrO_2$ particles obtained in Test 10.

Fig. 65 shows a TEM image (380°C, OA/Zr = 40) of the synthetic $ZrO_2$ particles obtained in Test 10.

Fig. 66 shows the change in particle size versus residence time (300°C) in Test 10.

Fig. 67 shows the change in particle size versus residence time (340°C) in Test 10.

Fig. 68 shows the change in grain size versus residence time (380°C) in Test 10.

Fig. 69 shows the XRD pattern of $ZrO_2$ in Test 10 (300°C, OA/Zr = 16).

Fig. 70 shows the XRD pattern of $ZrO_2$ in Test 10 (300°C, OA/Zr = 40).

Fig. 71 shows the XRD pattern of $ZrO_2$ in Test 10 (340°C, OA/Zr = 16).

Fig. 72 shows the XRD pattern of $ZrO_2$ in Test 10 (340°C, OA/Zr = 40).

Fig. 73 shows the XRD pattern of $ZrO_2$ in Test 10 (380°C, OA/Zr = 16).

Fig. 74 shows the XRD pattern of $ZrO_2$ in Test 10 (380°C, OA/Zr = 40).

Fig. 75 shows the crystallite size change of $ZrO_2$ versus the residence time in Test 10 (300°C).

Fig. 76 shows the crystallite size change of $ZrO_2$ versus the residence time in Test 10 (340°C).

Fig. 77 shows the crystallite size change of $ZrO_2$ with respect to the residence time (380°C) in Test 10.

Fig. 78 shows the percentage of the $mZrO_2$ phase in the synthesized particles as a function of the residence time (300°C) in Test 10.

Fig. 79 shows the percentage of the $mZrO_2$ phase in the synthetic particles as a function of the residence time (340°C) in Test 10.

Fig. 80 shows the ratio of the $mZrO_2$ phase in the synthetic particles to the residence time in Test 10 (380°C).

Fig. 81 shows the ratio of the $mZrO_2$ phase in the synthetic particles to the crystallite size in Test 10 (300°C).

Fig. 82 shows the ratio of the $mZrO_2$ phase in the synthetic particles to the crystallite size in Test 10 (340°C).

Fig. 83 shows the ratio of the $mZrO_2$ phase in the synthetic particles to the crystallite size in Test 10 (380°C).

Fig. 84 shows the XPS spectrum of Zr3d.

Fig. 85 shows the IR spectrum of $ZrO_2$.

Fig. 86 shows the change in the surface modification ratio of $ZrO_2$ with respect to the grain size (300°C) in Test 10.

Fig. 87 shows the change in the surface modification ratio of $ZrO_2$ with the grain size in Test 10 (340°C).

Fig. 88 shows the change in the surface modification ratio of $ZrO_2$ with respect to the grain size in Test 10 (380°C).

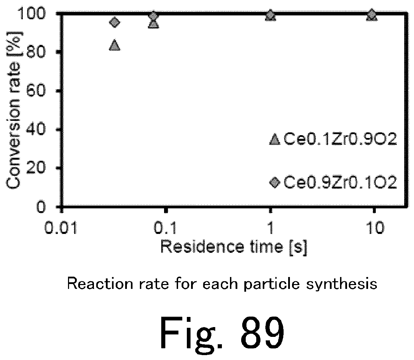

Fig. 89 shows the reaction rate of each particle in the synthesis of octanoic acid-modified $Ce_xZr_{1-x}O_2$ obtained from the ICP analysis in Test 10.

Fig. 90 shows a TEM image of the synthesized $Ce_{0.9}Zr_{0.1}O_2$ particles obtained in Test 10.

Fig. 91 shows a TEM image of the synthetic $Ce_{0.1}Zr_{0.9}O_2$ particles obtained in Test 10.

Fig. 92 shows the change in the particle size of each particle with respect to the residence time in Test 10.

Fig. 93 shows the XRD pattern of $Ce_{0.9}Zr_{0.1}O_2$.

Fig. 94 shows the XRD pattern of $Ce_{0.1}Zr_{0.9}O_2$.

Fig. 95 shows the crystallite size of $Ce_{0.9}Zr_{0.1}O_2$ versus the residence time.

Fig. 96 shows the crystallite size of $Ce_{0.1}Zr_{0.9}O_2$ versus the residence time.

Fig. 97 shows the percentage of $mZrO_2$ in $Ce_{0.1}Zr_{0.9}O_2$.

Fig. 98 shows the XPS spectrum (Ce3d) of $Ce_{0.9}Zr_{0.1}O_2$.

Fig. 99 shows the percentage of $Ce^{3+}$ on the surface of $Ce_{0.9}Zr_{0.1}O_2$ particles.

Fig. 100 shows the XPS spectrum (Zr3d) of $Ce_{0.1}Zr_{0.9}O_2$.

Fig. 101 shows the percentage of surface modification as a function of particle size for each particle in Test 10.

Fig. 102 shows the crystallite size change of $CeO_2$ in the initial stage of the reaction in Test 11.

Fig. 103 shows the relationship between the crystallite size of each particle and $\beta$ at a reaction temperature of 340°C.

Fig. 104 shows the relationship between the crystallite size and the unit lattice volume of $CeO_2$.

Fig. 105 shows the change in the crystallite size of $ZrO_2$ in the initial stage of the reaction.

Fig. 106 shows the relationship between the crystallite size of each particle and the collision frequency factor $\beta$.

Fig. 107 shows the relationship between the percentage of $mZrO_2$ in each particle and the collision frequency factor $\beta$.

Fig. 108 shows the relationship between the crystallite size and the unit lattice volume of $ZrO_2$.

Fig. 109 shows the relationship between the unit lattice volume and the collision frequency factor $\beta$ for $tZrO_2$.

Fig. 110 shows the relationship between the unit lattice volume and the collision frequency factor $\beta$ for $mZrO_2$.

MODE FOR CARRYING OUT THE INVENTION

Specific embodiments of the present invention are described in detail below. However, the invention is not at all limited to the following embodiments but can be conducted with modifications as appropriate within the scope of its purpose.

<Production method for metal oxide nanoparticles

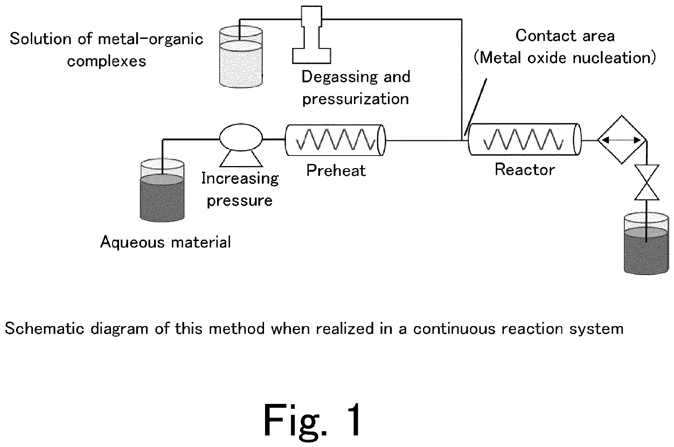

Fig. 1 is a schematic diagram of the method described in this embodiment when realized in a continuous reaction system. The method described in this embodiment includes a mixing process in which metal oxide nanoparticles are obtained by mixing a supercritical, subcritical, or gas-phase aqueous material with a an organometallic complex solution.

[Aqueous material]

The type of aqueous materials is not limited. Aqueous materials encompass water, polar organic solvents, or mixtures of water and polar organic solvents. Examples of aqueous materials include water, alcohols, carboxylic acids, ketones, ethers, esters, amides, amines, and sulfur compounds and mixtures thereof.
Alcohols include methanol, ethanol, isopropyl alcohol, t-butyl alcohol, propylene glycol, and phenol.
Carboxylic acids include lower carboxylic acids, such as formic acid, acetic acid, propionic acid, butyric acid, valeric acid, and caproic acid.
Ketones include acetone, methyl ethyl ketone, and methyl isobutyl ketone.
Ethers include ethylene glycol monobutyl ether, ethylene glycol monoethyl ether, tetrahydrofuran, dioxane, and methyl cellosolve.
Ethyl acetate and butyl acetate are examples of esters.
Amides include formamide, dimethylformamide, acetamide, dimethylacetamide, nitromethane, and acetonitrile.
Amines include methylamine, ethylamine, trimethylamine, triethylamine, monoethanolamine, diethanolamine, triethanolamine, pyridine, ethylenediamine, and hexamethylenediamine.
Dimethyl sulfoxide and the like are examples of sulfur compounds.
When the aqueous material is water, the treatment under supercritical or other conditions is called hydrothermal treatment, and when the aqueous material is alcohol, the treatment under supercritical or other conditions is called solvothermal treatment. The principles of hydrothermal and solvothermal treatment differ. Hydrothermal treatment is realized by a hydrolysis reaction, while solvothermal treatment is realized by a solvolysis-solvothermal reaction (alkalosis) when alcohols are used. In the case of solvothermal treatment using a nonpolar organic solvent, particle synthesis occurs due to the thermal decomposition of the raw material.
Even if the liquid medium (organometallic complex in this embodiment) has a hydrated structure, the hydrolysis reaction cannot occur without a large amount of water. Moreover, the reactivity of the hydrothermal treatment is determined by the in situ dielectric constant, which requires a large amount of bulk $H_2O$.
Therefore, even if the liquid medium is, for example, an alcohol, the hydrolysis reaction cannot occur because alcohols are different from water.
In this embodiment, the aqueous material can be from either a hydrolysis reaction or a solvolysis-solvent reaction, but it is preferable that the aqueous material be water that can be hydrothermally treated since it is easier to handle.
. However, in a high-temperature and high-pressure field, water and organic solvents form a homogeneous phase, so a mixed solvent can be used as the reaction field. In this case, hydrolysis also occurs, and the reaction can be controlled by controlling the polarity and dielectric constant of the mixed solvent.
. In the following, we discuss the case of water, including water-organic mixed solvents, since the principle is similar.

[Pressurization of aqueous materials]

Aqueous materials are pressurized in a pressurizing section. For example, a pressurizing pump is used as the pressurizing unit. By pressurizing the aqueous material and preheating it, supercritical, subcritical, or vapor-phase aqueous material can be continuously supplied.
The pressure of the aqueous material after pressurization is above the saturated vapor pressure. If the pressure is below the saturation vapor pressure, the nucleation of metal oxide nanoparticles cannot be realized, even if the aqueous material is brought into contact with a raw material solution containing organometallic complexes, which is not desirable.
To suitably realize the nucleation of metal oxide nanoparticles, the pressure of the aqueous material after pressurization should be 0.5 MPa or higher-10 MPa or higher is preferred, and 20 MPa or higher is even more preferred.

The pressure of the aqueous material after pressurization is 40 MPa or less; if the pressure exceeds 40 MPa, the cost of increasing the pressure resistance of the continuous manufacturing equipment will increase significantly, and the contact area between the aqueous material and the organometallic complex will easily deteriorate, which is not desirable.

From the viewpoint of reducing costs related to the pressure resistance of the continuous manufacturing device, it is preferable that the pressure of the aqueous material after pressurization be 37 MPa or less. From the viewpoint of reducing the deterioration of the contact area, it is preferable that the pressure of the aqueous material after pressurization be 35 MPa or less. This reduces the costs related to the pressure resistance of the continuous manufacturing equipment and also reduces the deterioration of the contact area.

[Preheating aqueous material]

The preheating section is not limited to heating aqueous material. Examples of preheating parts include a heating device that irradiates microwaves onto the aqueous material and a heating device that heats the aqueous material by heat conduction from a heating element, such as a heater. Preheating heats the aqueous material and can make it subcritical.

The temperature of the aqueous material after preheating is 300°C or higher; if the temperature is less than 300°C, in many cases, the aqueous material and the organometallic complex will not be able to form a homogeneous phase, which is undesirable. This is also undesirable because secondary changes occur after particle formation, such as agglomeration and collision, and stable bond formation by dehydration reaction is not expected.

The temperature of the aqueous material after preheating is 450°C or less. If the temperature of the aqueous material is too high, uniform particle size control within the range of 1.0 nm to 9.0 nm can be hindered, which is not desirable.

The preheating section is, for example, sloped forward and downward. When the flow velocity of the aqueous material is low, the aqueous material, which has been preheated to a low density, tries to rise, and the high-temperature, high-pressure water may flow backward through the preheating section, making it impossible to control the temperature. To prevent such backflow, aperture sections should be provided between the multiple heating sections. The aperture section is not limited as long as it prevents backflow. For example, a narrow tube or spiral tube can be used as an aperture section.

[Solution of organometallic complexes]

The raw material solution in this embodiment contains an organometallic complex. Even if the raw material solution contains salts of the same type of metal (including inorganic metal complexes), the diameter of the metal oxide nanoparticles produced is at least one of the average primary particle diameter or the crystallite diameter. (Hereinafter, unless otherwise specified, "diameter" shall mean the diameter of at least one of the average primary particle diameter or the crystallite diameter.) The coefficient of variation of the diameter cannot be controlled to be within the range of 1.0-9.0 nm; moreover, the coefficient of variation of the diameter cannot be controlled to be less than 1.0.

The form of the raw material liquid is not limited as long as it is fluid, and it can be an aqueous solution, slurry, paste, or suspension containing the raw material ingredients.

If it is difficult to prepare an aqueous slurry, the raw materials can be dispersed into an aqueous material, such as ethanol, to make a slurry.

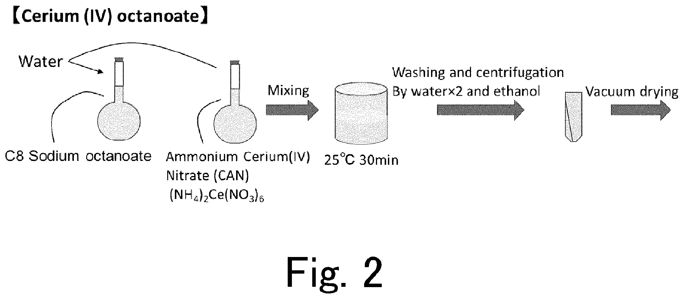

Organometallic complexes can be prepared by mixing metal salts with organomodified salts. Fig. 2 shows a schematic diagram of mixing cerium (IV) ammonium nitrate ($(NH_4)_2Ce(NO_3)_6$) and sodium octanoate to obtain cerium (IV) octanoate. First, cerium (IV) ammonium nitrate solution is added to sodium octanoate solution and stirred. Then, the solid phase is collected by filtration, washed with water and ethanol, and dried. Cerium (IV) octanoate can thus be obtained.

It is preferred that the molar ratio of the organic material to the metal comprising the organometallic complex be controllable. When the metal element constituting the organometallic complex is a metal element that can take on multiple types of valence, the valence of the metal element constituting the organometallic complex in the solution should be controlled to a relatively large valence of the same metal element. This allows the diameter and coefficient of variation of the nanoparticles to be more tightly controlled.

The following is an example of the effect of cerium valence on the precursor of cerium-carboxylate complexes in batch synthesis. When Ce (III) octanoate is used as a precursor, rod-like particles of $Ce(OH)_3$ are obtained as an intermediate, and oxidation proceeds over a long time to form $CeO_2$. Under the present conditions, $Ce(OH)_3$ is formed rapidly because a high concentration of octanoic acid Ce (III) is used as the raw material, and the redissolution rate is low because the carboxylic acid concentration in relation to $Ce(OH)_3$ is low. Therefore, $Ce(OH)_3$ crystal formation and growth proceed more preferentially than the Ce oxidation reaction.

In contrast, when Ce (IV) octanoate is used as a precursor, more homogeneous organically modified $CeO_2$ nanoparticles can be obtained; the use of a tetravalent Ce complex as a precursor does not require an oxidation reaction, and hydrolysis and dehydration occur immediately, forming uniform $CeO_2$ crystal nuclei.

In the distribution-type reaction using the Ce (IV) octanoate precursor, more uniform nanoparticles can be obtained than with the Ce (III) octanoate or cerium salt precursor. This is thought to be due to the fact that the surface is already modified during nucleation.

The conventional distribution method has been unable to obtain uniform nanoparticles, but the synthesis method of the present invention makes it possible to obtain uniform and controlled nanoparticles of 9.0 nm or less in a short time, even in the distribution method.

[Degassing and pressurization]

The raw material degassing section is not limited to those that degas the raw material solution containing organometallic complexes. For example, the raw material degassing section may include a degassing device that uses ultrasonic waves, one that reduces pressure, one that sends rare gases into the raw material solution, one that uses a permeation membrane or other existing technology, or one that combines these existing technologies. By degassing the feed solution, fluctuations in the feed volume of the feed solution caused, for example, by bubbles generated by dissolved gases can be suppressed. In addition, the corrosion of various components due to dissolved oxygen can be avoided.

The raw material pressurizing section is not limited to pressurizing a raw material solution containing an organometallic complex. For example, a pressurizing pump can be cited as a raw material pressurizing unit. By pressurizing the raw material solution, it can be brought into contact with the aqueous material under high pressure.

[Contact section]

The contacting section is not particularly limited, as long as the raw material solution containing organometallic complexes is mixed with the aqueous material in a supercritical state, etc. The contacting section is not particularly limited, as long as the raw material solution contains organometallic complexes and the aqueous material is in a supercritical state.

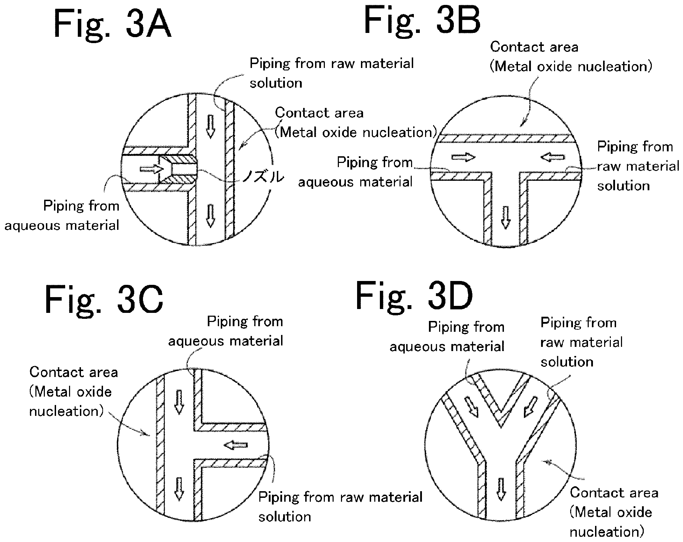

Fig. 3 is a schematic diagram showing an example of the contacting section. As shown in Fig. 3A, the contacting section preferably includes a structure in which the tip of a nozzle formed at the end, and from this tip is jetted a raw material liquid containing an organometallic complex, bringing it into contact with a high-temperature and high-pressure aqueous material. By being jetted from the tip of the nozzle, the raw material liquid is mixed with the aqueous material at a high speed to promote reaction efficiency. In addition, for phase-separated raw material liquids, the raw material liquid is atomized and dispersed in the aqueous material by being jetted from the tip of the nozzle, and the raw materials it contains are formed into a fine emulsion in the aqueous material at a high temperature and pressure, which further promotes reaction efficiency.

The contact section is not limited to mixing the aqueous material and the raw material liquid, and it may be of any other configuration. For example, as shown in Figure 3(B), a structure may be used where the aqueous material guided from an approximately horizontal direction is mixed with the raw material solution guided from an approximately horizontal direction. Alternatively, as shown in Figure 3(C), the structure may involve mixing the aqueous material guided from an approximately horizontal direction with the raw material solution guided from an approximately vertical direction. Furthermore, as shown in Figure 3(D), the structure may involve mixing the aqueous material guided from an upward diagonal direction with the raw material solution guided from an upward diagonal direction.

In the contact section, the source liquid is instantaneously heated to a subcritical temperature by the heat capacity possessed by the aqueous material, and the reaction between the source liquid and the aqueous material is initiated. This reaction initiates the surface treatment reaction of the organometallic complex.

The contact section is not an essential component. Although a contact section may be provided to achieve rapid mixing, it may not be provided. In this case, as shown in Fig. 3, the raw slurry passes directly through the preheating section shown in Fig. 1.

[Reaction processing section]

Return to Fig. 1. The reaction processing section (reactor) is not limited as long as supercritical, subcritical, or gas phase conditions are maintained for a predetermined time. A reactor covered with a thermostatic layer such as a spiral tube wound several times inside a heating cylinder, a molten salt bath jacket, or a fluidized sand bath are examples of reaction processing sections.

By making the reaction processing section a spiral tube wound several times inside the heating cylinder or a reactor covered with a thermostatic layer, the temperature change and unevenness of the mixture of raw material liquid and aqueous material due to heat conduction through the wall surface from the contact zone can be prevented, and the precise temperature control required for particle synthesis under supercritical, subcritical, or gas phase conditions can be achieved. This enables precise temperature control required for particle synthesis under supercritical, subcritical, or gas-phase conditions.

A hydrocyclone can also be installed downstream of the reaction processing section. This separates the reaction products from the fluid, and the reactor temperature can be controlled by the pressure of the fluid. The configuration with a hydrocyclone is preferred because it can improve the recovery yield of reaction products and also improve the controllability of the reactor temperature. The configuration in which the reaction treatment section is formed in a vertical direction so that the mixture of raw material liquid and aqueous material flows from the upper contact section to the lower hydrocyclone is preferred because it is easier to maintain a uniform temperature in the reaction treatment section.

The reaction temperature should be controllable between 300°C and 450°C. By controlling the reaction temperature, the diameter and coefficient of variation of the metal oxide nanoparticles produced can be controlled.

When the reaction product of the raw material solution and aqueous material passes through the reaction processing section, a high-temperature and high-pressure fluid containing metal oxide nanoparticles is emitted from the outlet of the reaction processing section.

The reaction time should be controllable in the range of 0.015 s to 380 s. When supercritical, subcritical, or vapor phase aqueous material is mixed with the solution of the organometallic complex, the organometallic complex is hydrolyzed, and the hydrolyzed organometallic complex salt is instantly dehydrated without oxidation to form metal oxide crystals in a short time of between 0.015 and 380 s. Because the organometallic complex solution is homogeneous, uniform nanoparticles can be generated from the salt of the organometallic complex without forming bulk hydroxides.

If the reaction rate is X, the reaction rate constant is k, and the residence time is $\tau$, then k can be obtained by using the equation $X = 1 - \exp(-ki)$ and measuring X with different residence time $\tau$. Once k is obtained, the relationship between residence time $\tau$ and reaction conversion rate X can be predicted.

According to the examples shown later, reaction conversion ratios of 99.33% and 99.97% can be obtained at reaction times of 0.04 and 95 s, respectively. From these results, it can be said that a practically meaningful reaction conversion rate of 85% can be achieved if the reaction time is 0.015 s or longer.

If the reactor is a continuous reaction system, the reaction time can be controlled by changing the volume of the continuous reactor and the mixing time by changing the flow rate of the mixed raw materials of the aqueous material and the organometallic complex solution fed to the continuous reactor. As a result, at a synthesis concentration of 0.1 g/l or higher and a reaction rate of 0.8 or higher, further metal oxide nanoparticle size reduction and uniformity of distribution can be achieved in continuous and batch production systems.

When the reactor is a continuous reaction system, it is preferable to set the Re number to 3000 or higher so that the mixing time can be controlled in the range of 1 s or less. A Re number of 6000 or higher is preferable, and one of 1000 or higher is even more preferable. By varying the volume of the continuous reactor and the flow rate of the mixed raw materials of the aqueous material and the solution of the organometallic complex supplied to the continuous reactor, the Re number of the mixing section or the mixing time can be controlled in the range of 1 s or less. This makes it possible to achieve both further size reduction of metal oxide nanoparticles and uniformity in particle size distribution in a continuous production system, as well as in batch production.

The higher the Re number in the reaction processing section, the higher the mixing rate increases and becomes the reaction rate, thereby enabling uniform particle synthesis with smaller particles (Non-patent Documents 1, 2). The mixing rate can also be evaluated using Kolmogorov's theory (Non-patent Document 1). In other words, the reaction rate can be evaluated from a small number of experiments, and a reactor can be designed for a rate-limiting reaction.

Here, the mixing rate is explained in more detail. The mixing rate, kmix, can be evaluated as a function of the Re number from Kolmogorov's theory. Then, the true reaction rate, k, can be evaluated from $1/k = 1/k_{app} - 1/k_{mix}$, using the apparent reaction rate, $k_{app}$, obtained from a small number of experimental points (1 or 2 points).

Synthesis below the reaction rate is necessary to obtain uniform and smaller particles. In other words, $1/k \gg 1/k_{mix}$, or $Da = k/k_{mix} \ll 1$.

. The Da number should be 1/5 or less, with 1/10 or less being preferable and 1/100 or less being even more preferable.

Compared to the hydrolysis rate of metal salts as raw materials, the hydrolysis rate of metal complexes is slower; therefore, this condition can be satisfied even with relatively slow mixing (i.e., relatively low Re numbers). However, to make the mixing time controllable within 1 s or less in a continuous reaction system, it is preferable that the Re number be 3000 or more, more preferable that it be 6000 or more, and even more preferable that it be 1000 or more.

[Recovery of metal oxide nanoparticles]

The recovery of metal oxide nanoparticles can be achieved by cooling the particle-containing high-temperature, high-pressure fluid from the reaction processing section and recovering the particles from the cooled fluid using, for example, an inline filter.

In doing so, a washing process may be included in which the mixed product is washed to remove organometallic complexes. This allows for the provision of metal oxide nanoparticles, free from residual organometallic complexes (unreacted substances), with a size of 9.0 nm or less and a uniform particle size distribution.

[Product]

The product (metal oxide nanoparticles) generated by the method described in this embodiment has an average primary particle size and/or crystallite size of 1.0 nm to 9.0 nm, with a coefficient of variation of 1.0 or less for these sizes. The metal element constituting the metal oxide is capable of forming an organometallic complex. Additionally, the product (metal oxide nanoparticles) has an average primary particle size and/or crystallite size of 1.0 nm to 9.0 nm, with a coefficient of variation of 1.0 or less for these sizes, and organic molecules are strongly bonded to the most unstable surface. According to the method described in this embodiment, extremely fine nanoparticles with a size of 9.0 nm or less can be provided with a uniform particle size distribution.

Whether the nanoparticles are strongly bonded can be determined by whether they are observed to desorb or decompose at a temperature higher than the boiling point of the adsorbent when thermogravimetry (TG) is performed. If the temperature of desorption or decomposition is higher than the boiling point of the adsorbent, the bond can be said to be strong, and if it is lower, it is not a strong bond.

The reverse micellar and hot soap methods are also known as methods for obtaining nanoparticles. However, in these methods, the organic modifier groups are physically adsorbed, and it cannot be said that the organic molecules are strongly bonded to the most unstable surface, as in the invention described in this form.

The smaller the coefficient of variation of diameter, the more desirable it is. In this embodiment, the coefficient of variation is less than 0.5, preferably less than 0.3, and more preferably less than 0.2.

The coefficient of variation of the average primary particle diameter can be obtained by dividing the standard deviation of the average primary particle diameter by the average primary particle diameter. In other words, the coefficient of variation is equal to the standard deviation of the average primary particle diameter divided by the average primary particle diameter.

Similarly, the coefficient of variation of the crystallite diameter can be obtained by dividing the standard deviation of the crystallite diameter by the crystallite diameter. In other words, the coefficient of variation is equal to the standard deviation of the crystallite diameter divided by the crystallite diameter.

In this embodiment, the average primary particle diameter of nanoparticles is assumed to be the arithmetic mean value obtained from the diagonal of a square on one side by capturing images of particles using a transmission electron microscope (TEM) and analyzing the TEM images of 50 particles using image analysis and image measurement software. In such cases, when the particle size distribution is wide in this particle size range, such as when the coefficient of variation exceeds 2.0, it is necessary to pay attention to whether the particles in the field of view are representative of all the particles.

In this method, the crystallite size of nanoparticles is determined by X-ray diffraction (XRD). CuK$\alpha$ ray ($\lambda$ = 1.5418Å) is used as the X-ray source. The 2$\theta$ scanning speed is 3°/min. The crystallite size calculation uses the half width of the X-ray diffraction peak (FWHM). The following Scherrer equation is used as the method (Equation [1]):

$$D = \frac{K\lambda}{\beta \cos\theta} \tag{1}$$

where D is the crystallite diameter, K is the shape factor (generally 0.9), and $\beta$ is the peak half width.

On the other hand, diffraction peak broadening can be attributed to the size effect or the strain effect. The Halder-Wagner method (Equation [2]) is used to separate and calculate the size effect.

$$\left(\frac{\beta^{\bullet}}{d^{\bullet}}\right)^2 = \frac{K}{D}\frac{\beta^{\bullet}}{(d^{\bullet})^2} + (2\varepsilon)^2 \tag{2}$$

$$\beta^{\bullet} = \frac{\beta' \cos\theta}{\lambda} \tag{3}$$

$$d^{\bullet} = \frac{2\sin\theta}{\lambda} \tag{4}$$

where $\beta'$ is the peak integration width and $\varepsilon$ is the microstrain. From Equations (3) and (4), Equation (2) can be expressed as in Equation (5):

$$\left(\frac{\beta'}{\tan\theta}\right)^2 = \frac{K\lambda}{D}\frac{\beta'}{\tan\theta\sin\theta} + 16\varepsilon^2 \qquad (5)$$

From Formula (5), the slope of $(\beta'/\tan\theta)^2$ plotted against $\beta'/(\tan\theta\sin\theta)$ is $K\lambda/D$, and the crystallite diameter, D, is obtained from this slope.

In this invention, in the synthesis of organically modified metal oxide nanoparticles by the supercritical hydrothermal method, the formation process of nanoparticles is clarified by controlling the reaction time on a second scale through a distribution-type reaction. In particular, the initial formation mechanism, which has not been clarified for ultrafine metal oxide particles, is elucidated.

In the initial stage of formation, uniform nucleation is achieved through the rapid reaction of precursors. After homogeneous nucleation, there is a nonclassical nucleation stage due to the aggregation and coalescence of micronuclei. In the nonclassical nucleation stage, by aggregation and coalescence, there is an initial stage in which the nuclei remain spherical after coalescence and a stage in which the ellipsoid shape increases due to the coalescence of the two particles. This suggests that the crystallinity of the nanoparticles in the reaction field changes as the particle size increases.

The aggregation and coalescence rates of the nonclassical nucleation stage vary with the reaction temperature and modifier, which significantly affect the final particle size. The particle size decreases with a decreasing reaction temperature, increasing the modifier concentration and the molecular chain length of the modifier. These changes in particle size are thought to be due to changes in the probability of coalescence of the aggregated particles, suggesting that nonclassical nucleation can be controlled by controlling the probability of particle coalescence.

The present invention makes it possible to obtain cubic-shaped particles with a crystallite size of 4-7 nm in a controlled manner. In addition, by obtaining nanoparticles in the nonclassical nucleation stage, the size of the nanoparticles can be controlled and synthesized from 1.5 nm, a region that has up to now been uncontrollable for metal oxide nanoparticles.

Taking as an example the case where the metal oxide nanoparticles are $CeO_2$ nanoparticles, in the initial stage of formation, these nanoparticles show a characteristic peak at 20° in the XRD diffraction pattern, in addition to increased strain and vacancies. This is due to stacking defects, which are thought to originate from particle aggregation and coalescence in the early stages of formation.

EXAMPLES

The invention is described in detail in the examples below, although it is not limited to these examples.

<Materials>

In this study, cerium nitrate hexahydrate $[Ce(NO_3)_3\text{-}6H_2O]$, cerium (IV) hydroxide $(Ce[OH]_4)$, sodium octanoate, octanoic acid, benzene, methanol, ethanol, and cyclohexane were purchased from Fujifilm Wako Pure Chemicals Co. Cerium (IV) ammonium nitrate $[(NH_4)_2(Ce[NO_3]_6)]$ (CAN) was purchased from Sigma-Aldrich. All materials were used as received without further purification.

<Test 1> Stable supply of fine nanoparticles

[Example 1] When the raw material solution contains an organometallic complex

[Synthesis of cerium octanoate (IV)]

Cerium octanoate (IV) was synthesized by a metathesis reaction. First, 0.1 M ammonium cerium (IV) nitrate solution and 0.3 M sodium octanoate solution were prepared separately. Next, 500 mL of cerium (IV) ammonium nitrate solution was added to 500 mL of sodium octanoate solution and stirred at 25°C for 60 min. The cerium (IV) octanoate product was collected by pressure filtration through a polytetrafluoroethylene (PTFE) filter with a pore size of 0.1 $\mu$m, washed with water and ethanol, and dried at 70°C overnight.

[Supercritical hydrothermal synthesis]

Supercritical hydrothermal synthesis was performed using a distribution-type hydrothermal apparatus. Cerium octanoate (IV) and octanoic acid were dissolved in a prescribed amount of benzene and mixed with heated water, and the reaction was carried out. The cerium concentration was 0.03 mol/L. The flow rate of the water was 24 mL/min and that of the benzene solution was 8 mL/min. The water was pumped by a plunger-type pump (Nihon Seimitsu), and the benzene solution was pumped by a syringe pump (ISCO). Reactions were performed at 340°C and 30 MPa using reaction tubes made of SUS. By changing the tube volume, the reaction was performed for each specified time (0.038, 0.1, 0.2, 1, 3, 6.8, 95, and 380 s).

[Product recovery]

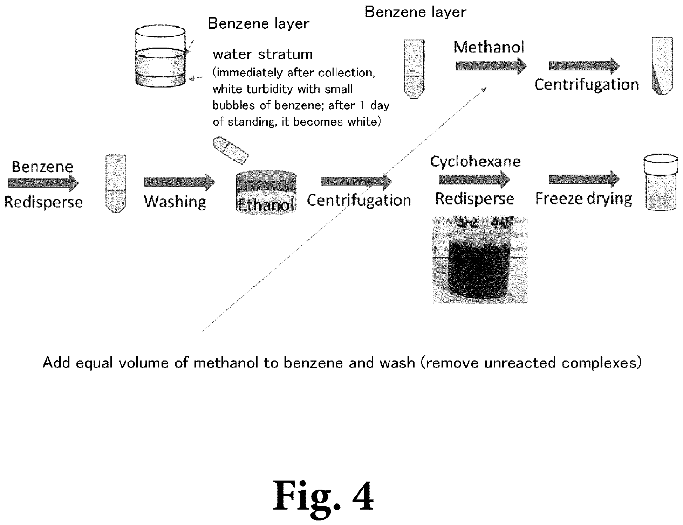

Fig. 4 shows a schematic diagram of the recovery of the products after the reaction is completed. First, after the reaction is completed, the vessel is quenched in a water bath at room temperature, and the products are recovered using benzene. After removing the water, methanol is added to the benzene solution. The volume ratio of benzene to methanol is 1:1. The solid product is washed twice with ethanol, centrifuged, and decanted. The solid product is redispersed in cyclohexane and lyophilized.

[Comparative example 1] When the raw material solution contains an inorganic metal complex

[Supercritical hydrothermal synthesis]

Cerium (IV) ammonium nitrate solution and octanoic acid were reacted in a distribution system. The cerium concentration was 0.03 mol/L, and the concentrations of octanoic acid to cerium were 16 and 69 in molar ratio, respectively. The reaction was carried out using the above electric furnace at 340°C and 30 MPa for 95 s.

[Evaluation]

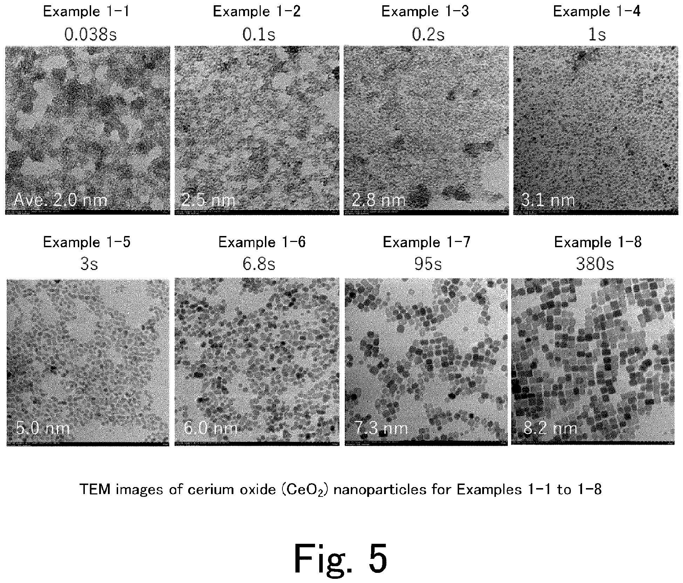

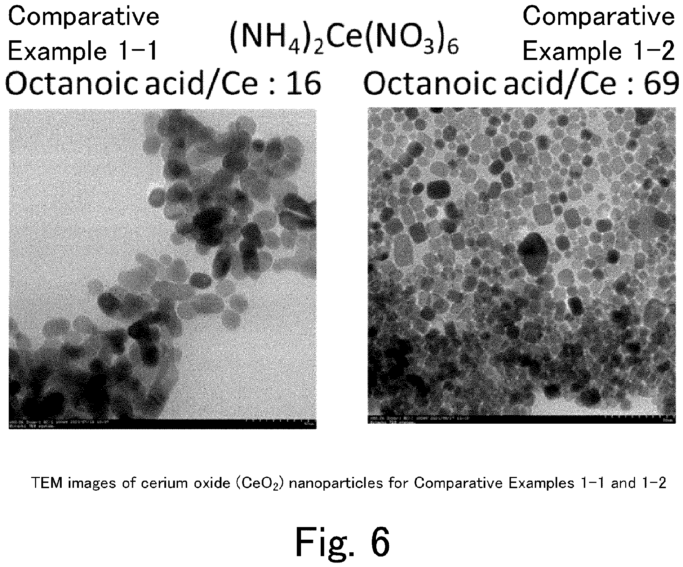

The morphology of the cerium (IV) oxide nanoparticles obtained for Example 1 and Comparative Example 1 was observed using a transmission electron microscope (TEM, H-7650, Hitachi High-Technologies Corporation). The results for Example 1 are shown in Fig. 5 and Table 1, and the results for Comparative Example 1 are shown in Fig. 6 and Table 1.

TABLE 1

| | Raw material solution | Reaction time | Cerium octanoate | Average primary particle diameter | Coefficient of variation | Recovery factor |
|---|---|---|---|---|---|---|
| Example 1-1 | Organic metal complexes | 0.038 sec | 16 | 2.0 nm | 0.17 | 80% |
| Example 1-2 | Organic metal complexes | 0.1 sec | 16 | 2.5 nm | 0.23 | - |
| Example 1-3 | Organic metal complexes | 0.2 sec | 16 | 2.8 nm | 0.18 | - |
| Example 1-4 | Organic metal complexes | 1 sec | 16 | 3.1 nm | 0.25 | 68% |
| Example 1-5 | Organic metal complexes | 3 sec | 16 | 5.0 nm | 0.25 | 79% |
| Example 1-6 | Organic metal complexes | 6.8 sec | 16 | 6.0 nm | 0.25 | 71% |
| Example 1-7 | Organic metal complexes | 95 sec | 16 | 7.3 nm | 0.23 | 60% |
| Example 1-8 | Organic metal complexes | 380 sec | 16 | 8.2 nm | 0.2 | 51% |
| Comparative example 1-1 | Inorganic metal complexes | 95 sec | 16 | 14.7 nm | 0.22 | - |
| Comparative example 1-2 | Inorganic metal complexes | 95 sec | 69 | 10.0 nm | 0.27 | - |

From Example 1, it was confirmed that the average primary particle diameter of the nanoparticles could be controlled within the range of 1.0 nm to 9.0 nm and that the coefficient of variation of the average primary particle diameter could be controlled within 0.25 nm or less by controlling the mixing time by making the target metal an organometallic complex and reacting it with a supercritical, subcritical, or gas-phase water-based material. The coefficient of variation of the primary average particle size was controlled to be within 0.25. It was also possible to obtain nanoparticles while controlling the size to less than 9.0 nm. We believe this is because the organometallic complexes were bound to the modified molecules from the precursor state, which was partially retained during nucleation at high temperatures. The layer of surface modifier lowered the surface energy and suppressed the aggregation of particles.

The recovery rates for Examples 1-1 to 1-8 exceeded 50%, indicating that nanoparticles with different reaction times can be efficiently obtained by this method. On the other hand, the recovery rate tended to decrease as the reaction time increased. This is presumed to be due to the effect of wall adhesion to the reaction zone.

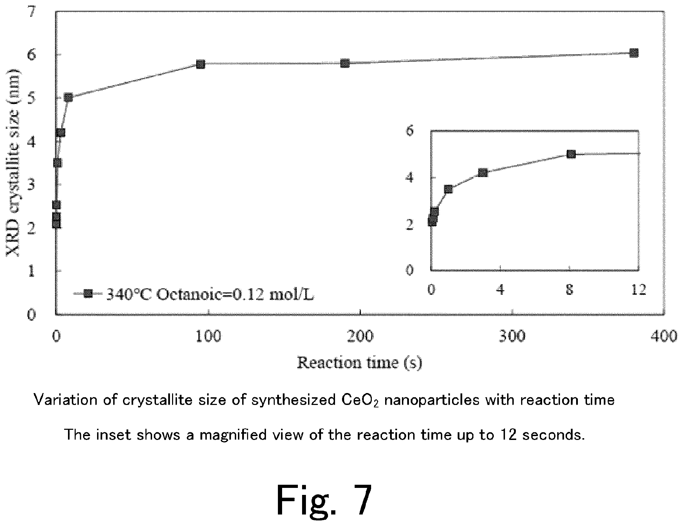

Fig. 7 shows the time variation of the crystallite diameter measured from the XRD pattern of the synthesized nanoparticles. The XRD pattern was measured using SmartLab 9MTP equipment manufactured by the Rigaku Corporation. By measuring the actual particles obtained, it was possible to capture the change in the crystallite diameter over the reaction time.

The crystallite diameter at the initial stage of the reaction was 2 nm. In the synthesis of metal nanoparticles, it is known that primary nuclei of 1 to 2 nm in size can be observed in the initial stage of nucleation. The nanoparticles in this test case were close to the size of the initial nucleus, and we believe that we were able to obtain particles close to the size of the nucleus immediately after nucleation. The precursor reaction rate was calculated using inductively coupled plasma atomic emission spectroscopy (ICP-AES) from the residual Ce concentration.

ICP-AES (ARCOS FHM22 MV130, AMETEK Inc.) was performed as follows. The cerium ion concentration in the supernatant liquid after centrifugation of the benzene-methanol solution was measured. The organic component was pyrolyzed by heating with acid (HNO3, H2SO4). The organic component was thermally decomposed by adding acid (HNO3, H2SO4), and then water was added and evaluated as a Ce solution.

Table 2 shows the ICP-AES results.

TABLE 2
Reaction time and rate of the precursor

| Reaction time (s) | 0.04 | 95 |
|---|---|---|
| Reaction rate (%) | 99.33 | 99.97 |

The reaction rate exceeded 99% at the reaction time of 0.04s, confirming that Ce (IV) octanoate reacts rapidly. We believe that this reaction led to rapid nucleation and the formation of uniform micronuclei. Thus, it was found that uniform nucleation by the rapid reaction of precursors occurred in the initial stage of the formation of surface-modified nanoparticles.

In the post-nucleation stage, the crystallite diameter increased rapidly during the reaction time up to 6.8 s. Thereafter, it increased slowly. Such a change in growth rate was considered to indicate a change in the particle growth mechanism. The change in the growth mechanism can be inferred from the particle shapes in the TEM images for each time period, as shown in Fig. 5. Until the reaction time was 6.8 s, the particles were spherical or ellipsoidal in shape. As the reaction time increased, the nanoparticles approached a cubic shape. This indicates that the carboxylic acid preferentially modified the (100) surface of $CeO_2$ as it matured and grew. On the other hand, the particles were nearly spherical in shape for a short time before 6.8 s, suggesting that the particle size increased by a mechanism other than ripening.

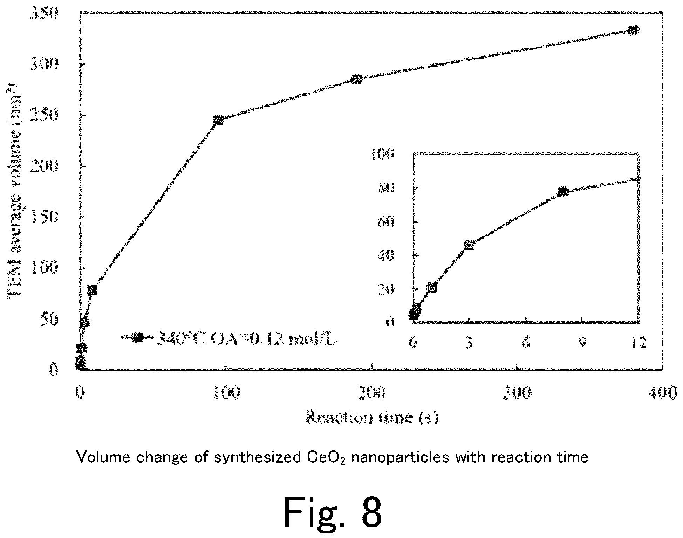

The change in particle growth rate with reaction time was confirmed not only by the crystallite diameter determined by XRD but also by the particle volume calculated from the TEM images. The volumes of 50 particles were calculated from the images shown in Fig. 5 to obtain the arithmetic mean volume. At reaction times of 0.04 s and 0.1 s, it was difficult to distinguish individual primary particles because they aggregated before the synthesized nanoparticles had been collected and dried on the TEM grid. Therefore, particles at 0.04 s and 0.1 s were converted to sphere equivalent volumes from the XRD diameter. Particles with reaction times of 0.02 s to 7 s had many spherical and ellipsoidal particles. Therefore, the particles were considered ellipsoids with a long diameter × a short diameter × a short diameter, and their long and short diameters were measured to calculate their volumes. The particles with reaction times from 95 s to 380 s were cubic and rectangular in shape. Therefore, the particles were considered hexahedrons of long × short × short diameter; their long and short diameters were measured, and their volumes were calculated. The results of the average volume of the particles for each reaction time are shown in Fig. 8. Even when the change in particle shape is taken into account, the acceleration of the particle diameter increase varied and slowed down with the reaction time.

Here, the mechanism of the initial increase in particle size up to a reaction time of 6.8 s is clarified. In this test, the reaction rate of the precursor was close to 100% at a reaction time of 0.04 s. Therefore, it can be said that almost all the precursors are consumed at the time of homogeneous nucleation, and the growth at this stage was not due to the reaction of the complexes. Nonclassical nucleation due to the aggregation of micronuclei has been reported to occur in the initial stage of metal nanoparticle growth. However, the process of metal oxide nanoparticle crystal formation smaller than 10 nm is difficult to elucidate, and the nonclassical nucleation stage has not been fully investigated.

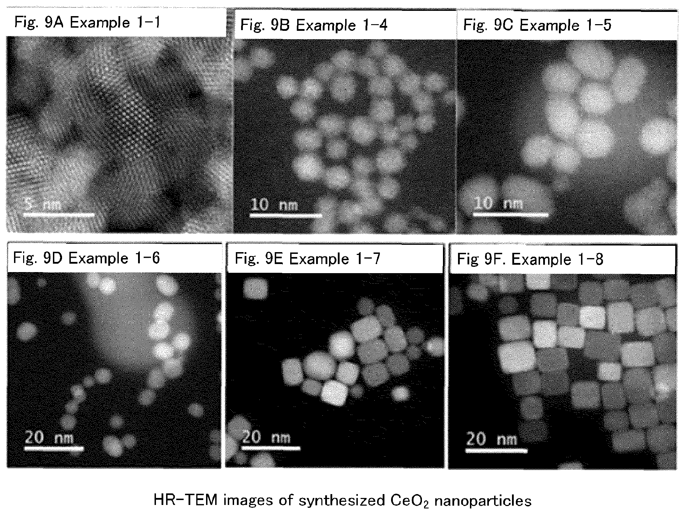

In this study, nanoparticles in the formation stage were successfully synthesized, and the formation process was clarified by analyzing the particle shapes. Fig. 9 shows the results of the HR-TEM magnification of the nanoparticle shapes. At a reaction time of 3 s, there were many elliptical particles. This is because the shape of the two spherical particles was maintained when they were agglomerated and coalesced. This indicates that the initial few seconds of the reaction phase were marked by nonclassical nucleation due to the agglomeration and coalescence of the micronuclei. In this document, the nonclassical nucleation stage is defined as the growth region in which $CeO_2$ nuclei or particles are used as the unit instead of Ce atoms or molecules.

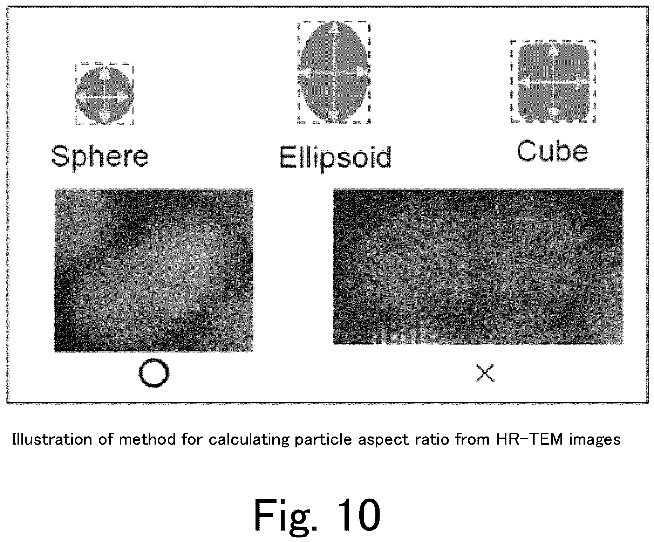

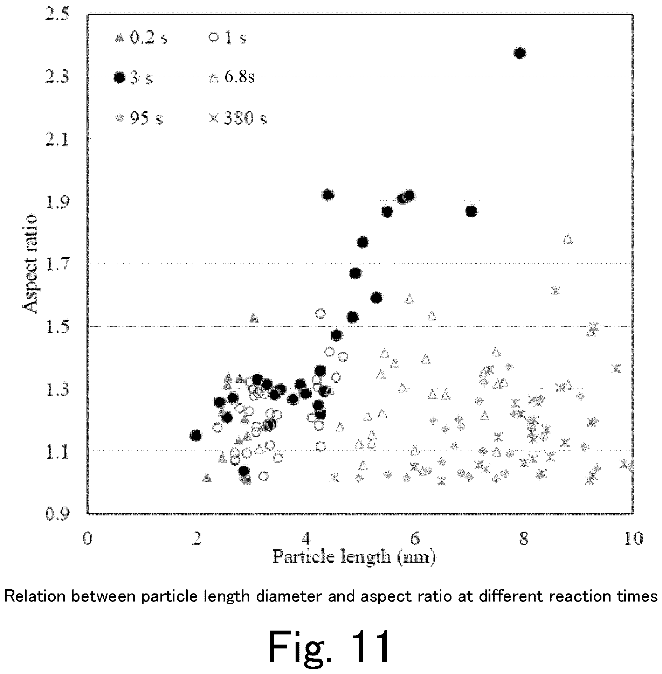

The aspect ratio of the particles was evaluated to quantify the change in particle shape due to the coalescence of the particles. Short and long diameters equivalent to a rectangle were evaluated for the particles in the image (Fig. 10). If the particles were spherical or cubic, the ratio of the short diameter to the long diameter was 1. In contrast, the aspect ratio increased for ellipsoids. From the HR-TEM images, the long diameter, short diameter, and aspect ratio of 30 particles were determined for each reaction time, and the aspect ratio was plotted against the particle-long diameter. Only single crystalized particles with an aligned lattice within them were measured (Fig. 10). The results are shown in Fig. 11.

The relationship between the particle length diameter and the aspect ratio, which changes with reaction time, is described below. At short reaction times of 1 s or less, there was almost no correlation between the particle length diameter and the aspect ratio. At a reaction time of 3 s, most of the particles were ellipsoidal in shape, and those before that time were close to spherical in shape, despite their cohesive growth. We attribute this to the fact that particles in the early stages of formation are smaller and less crystalline. Therefore, the coalesced particles were easily deformed, and their shape approached the most stable spherical shape. Thus, in nonclassical nucleation, there existed a region that maintained a spherical shape despite aggregation and coalescence. In contrast, when the reaction time reached 3 s, the number of elliptically shaped particles with a high aspect ratio increased rapidly. The larger the particle size, the higher the aspect ratio, indicating that there is a region where two spherical particles coalesce during the particle growth stage and retain their shape. We believe that this is because when the particle diameter increases to a certain degree, the crystallinity of the particles increases, and the ellipsoid-shaped particles retain their shape at the moment of coalescence.

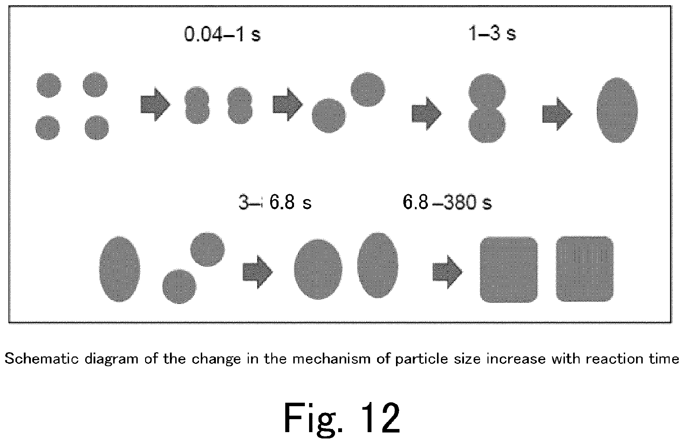

Furthermore, as the reaction time elapsed after 3 s, the aspect ratio decreased as the particle size increased. In addition, the number of particles with a difference in diameter between the long and short diameters, such as those with an aspect ratio close to 2, decreased. It is thought that the reason for the aspect ratio not increasing beyond a certain level is due to the merging of large particles not occurring as the particle diameter increases. The particles thus formed were finally shape-controlled to expose the (100) surface by Ostwald ripening. During the Ostwald ripening process, the ellipsoidal particles changed so that the curvature difference was resolved, and they approached a more stable shape, resulting in a lower aspect ratio. Fig. 12 summarizes the schematic diagram of the mechanism of change in particle size increase predicted from the shape analysis at each reaction time.

The lack of agglomeration and coalescence with increasing particle size can possibly be explained by the collision frequency decreasing with increasing particle size. The diffusion coefficient decreases with increasing particle size. In addition, the number density of particles decreases, and the distance between particles increases. As a result, the number of collisions decreases. To confirm whether the cessation of coalescence growth could be explained by a decrease in collision frequency, the number of collisions was measured based on the particle mean free path and diffusion constant.

From the formula for diffusion in three dimensions, due to diffusion between particles, the collision time t' can be calculated from Equation (6).

$$t' = \frac{L^2}{6D}$$

$$(6)$$

In Equation (6), L is the mean free path between particles (m), and D is the diffusion constant ($m^2$/s). The number of collisions per unit of time is expressed as 1/t'. The mean free path is expressed in Equation (7) from the particle density and the effective cross-sectional area of the particles.

$$L = \frac{1}{\sqrt{2}AN}$$

$$(7)$$

In Equation (7), A is the effective cross-sectional area ($m^2$), and the cross-sectional area of the organic-modified nanoparticle core is used. The number density of particles, N ($m^{-3}$), can be calculated using Equation (8).

$$N = \frac{MC}{\rho V} \tag{8}$$

In Equation (8), M is the particle molar mass (kg/mol), C is the raw material concentration (mol/m$^3$), and $\rho$ is the solid density (kg/m$^3$). Due to the Brownian motion of particles, the diffusion coefficient was calculated using the Stokes-Einstein equation in Equation (9).

$$D = \frac{k_B T}{6\pi \eta r} \tag{9}$$

In Equation (9), $k_B$ is Boltzmann's constant (m$^2$kgs$^{-1}$K$^{-1}$), T is temperature (K), $\eta$ is solvent viscosity (Pa·s), and r is the particle radius (m). This equation represents the motion of particles in a stationary fluid, and the actual diffusion constant and number of collisions will be larger.

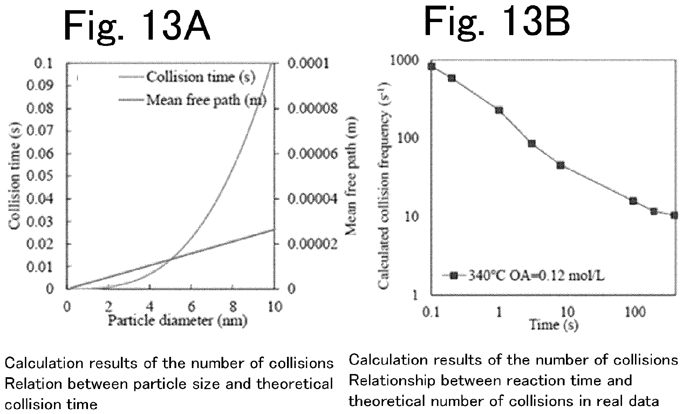

The relationship between collision time and particle radius obtained by Equations (6)-(9) is shown in Fig. 13A. The collision time at a particle diameter of 10 nm was less than 0.1 s, which was sufficiently short for particles with a long diameter of 10 nm or less in this experiment, even after the reaction time. The theoretical number of collisions at that time was obtained from the particle diameter at each reaction time and plotted for each reaction time in Fig. 13B. Although the theoretical number of collisions decreased as the reaction time elapsed, and the particle density and diffusion coefficient decreased, the number of collisions was more than 10. This indicates that particle agglomeration and coalescence end due to factors other than a simple decrease in the number of collisions.

When particles come into contact, they must exceed the energy barrier of coalescence to coalesce. This energy includes, for example, reaction energy to release the surface modifier before coalescence, reaction energy for dehydration and condensation of the surface hydroxyl groups, the repulsive force between particles, and the driving force for aligning their surfaces and orienting and adhering to each other. An increase in particle size increases crystallinity, and an increase in repulsive force between particles may decrease the probability of adhesion. It is also possible that an increase in crystallinity causes the particles to deform less at the moment of collision, resulting in a decrease in the collision area relative to the particle diameter and making coalescence by dehydration-condensation less likely. The increase in surface area also increases the total amount of surface modifier, and the energy required to desorb the modifier increases. We believe that these multiple factors increase the energy barrier to coalescence as particle size increases, resulting in a decrease in the aggregation and coalescence rates of the particles.

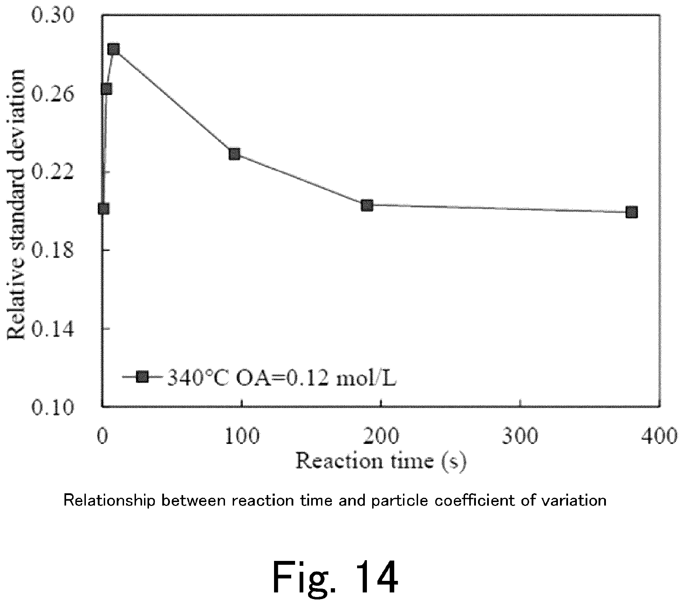

This decrease in the frequency of particle coalescence with increasing particle size can be inferred from the particle size distribution. Fig. 14 shows the change in the coefficient of variation of the TEM length diameter with reaction time. The coefficient of variation increased once between 1 and 7 s of reaction time, after which it gradually decreased, and the particles became more uniform. The reason why the particle size distribution initially became wider is that the frequency of coalescence between particles decreases during the growth of particle agglomeration, and the distribution of particles that have coalesced and those that have not coalesced occurs at that time. The particles that have increased in size do not coalesce. The coefficient of variation was reduced by the coalescence of smaller particles, not by the coalescence of larger particles. In the Ostwald ripening process, particles with inhomogeneous shapes, such as ellipsoids and spheres, were further reduced to cubes with exposed (100) surfaces, and the coefficient of variation was further lowered.

This experiment revealed the formation mechanism of homogeneous nucleation followed by nonclassical nucleation in the formation process of organically modified nanoparticles of 10 nm or less. Furthermore, the particle shape changes during the nonclassical nucleation stage, suggesting a change in crystallinity during this stage. The decrease in the nonclassical nucleation stage velocity with increasing particle size cannot be explained by a decrease in the number of collisions alone, and the effect of the coalescence energy barrier was found to be significant.

When supercritical, subcritical, or vapor-phase aqueous materials are mixed with solutions of organometallic complexes, the organometallic complexes are hydrolyzed, and these hydrolyzed organometallic complex salts are instantly dehydrated without oxidation to form metal oxide crystals within a short time period of 0.04-380 s. Because the solution of organometallic complexes is homogeneous, it is thought that uniform nanoparticles could be generated from the salts of organometallic complexes without forming bulk hydroxides.

According to this study, uniform diameter and shape-controlled nanoparticles can be obtained without any classification or other operations, not only by the batch method but also by the distribution method of synthesis.

It was also confirmed that the mixing time could be controlled within the range of 0.04-1 s by setting the Re number to 3000 or more.

From Comparative Example 1, even if one attempted to synthesize metal oxide nanoparticles from metal salts (inorganic metal complexes) by adding the same amount of modified molecules, it would not be possible to refine the particles to between 1.0 nm and 9.0 nm, as in Example 1. In addition, the particle size distribution was wider than in Example 1, and the shape could not be controlled to a cubic form. In the case of the inorganic metal complex as a precursor, the particles were not surface modified at the nucleation stage, and it is considered that they aggregated rapidly because the speed at which particles aggregate is faster than the speed at which they are surface modified. The snarled shape formed by agglomeration in the initial stage of the reaction remained even after the reaction time had elapsed.

<Test 2> Control of average primary particle diameter by shifting the amount of modification molecules added to the raw material for organometallic complexes [Example 2] When the raw material solution contains an organometallic complex

[Synthesis of cerium (IV) octanoate]

The product of cerium octanoate (IV) was recovered using the method described in Example 1.

[Supercritical hydrothermal synthesis]

In addition to cerium (IV) octanoate and water, octanoic acid was added as the composition of the feed solution supplied to the distribution-type hydrothermal apparatus. The ratios (molar ratio) of octanoic acid to cerium (IV) were 0, 16, 32, and 69, respectively. The reaction temperature was set at 380°C. The supercritical hydrothermal synthesis was carried out using the method described in Example 1.

[Recovery of products]

Cerium (IV) oxide nanoparticles were recovered using the method described in Example 1.

[Reference Example 2] When the raw material solution contains inorganic metal complexes

[Supercritical hydrothermal synthesis]

Cerium nitrate, cerium (IV) ammonium nitrate, and cerium (IV) hydroxide were used instead of cerium (IV) octanoate as the composition of the raw material solution fed into the reaction vessel. These cerium molar concentrations were the same as in Example 2. The ratios of octanoic acid to cerium (IV) (molar ratio) were 16 and 69, respectively. Otherwise, supercritical hydrothermal synthesis was performed using the same method described in Example 2.

[Recovery of products]

Cerium (IV) oxide nanoparticles were recovered using the method described in Example 2.

[Evaluation]

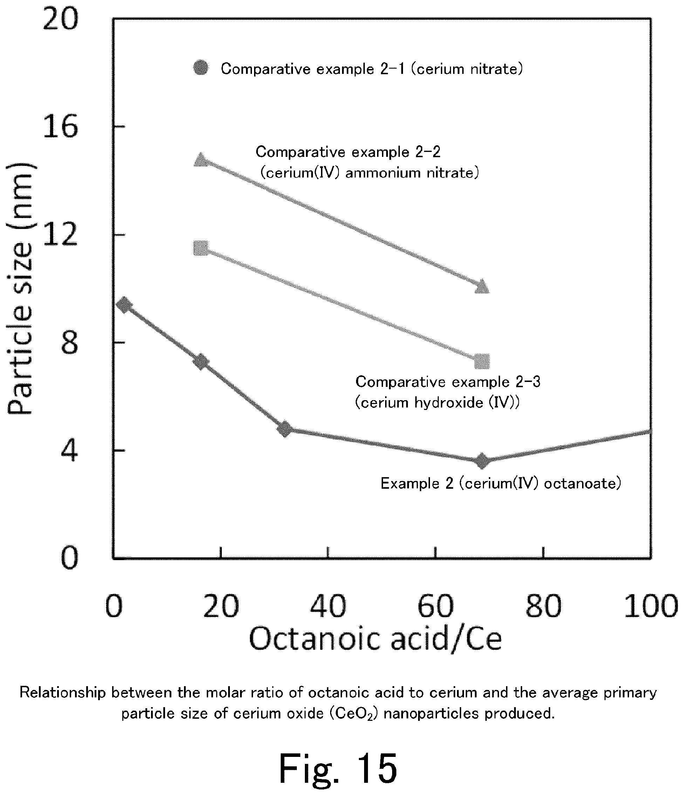

For Example 2 and Reference Example 2, the average primary particle diameter of the cerium oxide (IV) nanoparticles obtained was calculated from the images taken using the TEM described above. The results are shown in Fig. 15.

From Example 2, it was confirmed that the average primary particle diameter could be further refined by increasing the amount of modifier molecules added to the organometallic complex raw material. It was also confirmed that the correlation between the amount of modifying molecules and the amount of modifying molecules was the same for Reference Example 2. This is assumed to be because the reaction speed could be controlled by the number of modifying molecules.

<Test 3> Control of average primary particle size by shifting the reaction temperature [Example 3-1] Reaction temperature: 340°C

[Synthesis of cerium (IV) octanoate]

The product of cerium octanoate (IV) was recovered using the method described in Example 1.

[Supercritical hydrothermal synthesis]

In addition to cerium (IV) octanoate and water, octanoic acid was added as the composition of the feed solution supplied to the distribution-type hydrothermal apparatus. The ratios (molar ratio) of octanoic acid to cerium (IV) were 16 (octanoic acid concentration 0.12 mol/L) and 69 (octanoic acid concentration 0.52 mol/L). The reaction temperature was set at 340°C. Otherwise, supercritical hydrothermal synthesis was performed using the method described in Example 1.

[Recovery of products]

Cerium (IV) oxide nanoparticles were recovered using the method described in Example 1.

[Example 3-2] Reaction temperature: 300°C

Cerium (IV) oxide nanoparticles were recovered using the method described in Example 3-1, except that the reaction temperature in the supercritical hydrothermal synthesis was set to 300°C.

[Evaluation]

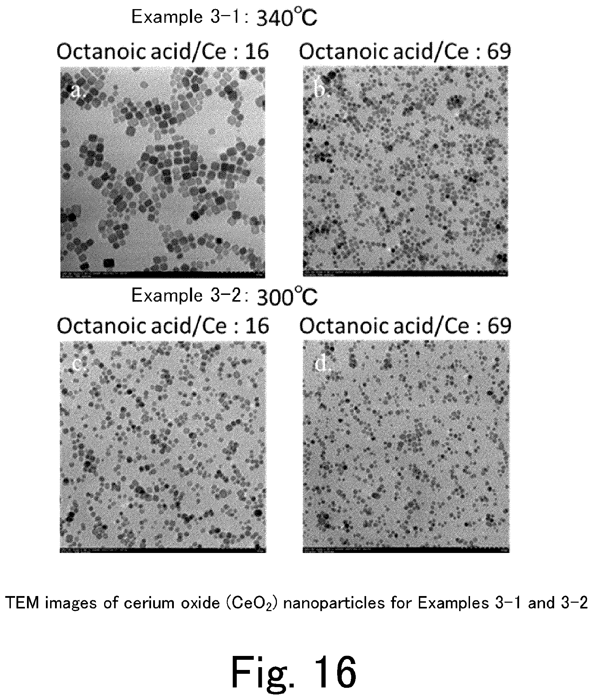

The morphology of the cerium (IV) oxide nanoparticles obtained for Examples 3-1 and 3-2 was observed using the TEM described above. The results are shown in Fig. 16, which confirms that the growth rate can be increased by increasing the temperature and that secondary changes after particle formation, such as agglomeration and collisions, can be suppressed by adjusting the temperature.

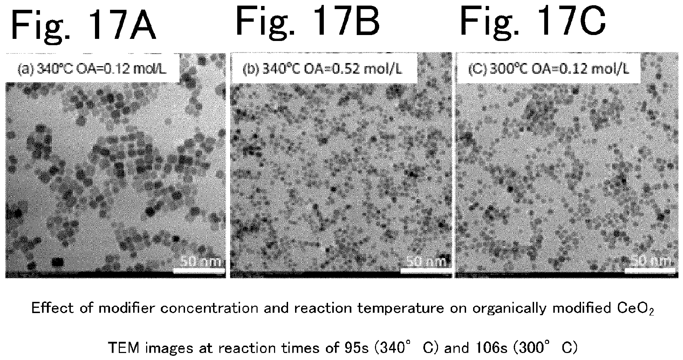

From Test 2, the particle size decreased as the modifier concentration increased. From Test 3, the crystallite diameter decreased as the reaction temperature decreased, even at the same modifier concentration. In the following, we show that the decrease in particle size with increasing modifier concentration and decreasing temperature is due to the nonclassical nucleation step. (a) Example 3-1: 340°C, octanoic acid concentration OA = 0.12 mol/L, OA/Ce = 16, (b) Example 3-2: 300°C, octanoic acid concentration OA = 0.12 mol/L, OA/Ce = 16 (temperature reduction), (c) Example 3-1: 340°C, octanoic acid concentration OA = 0.52 mol/L, OA/Ce = 64 (modifier increase). Under these conditions, nanoparticles were obtained by varying the reaction time, and the factors affecting the change in particle size are clarified. Fig. 17 shows an image after growth.

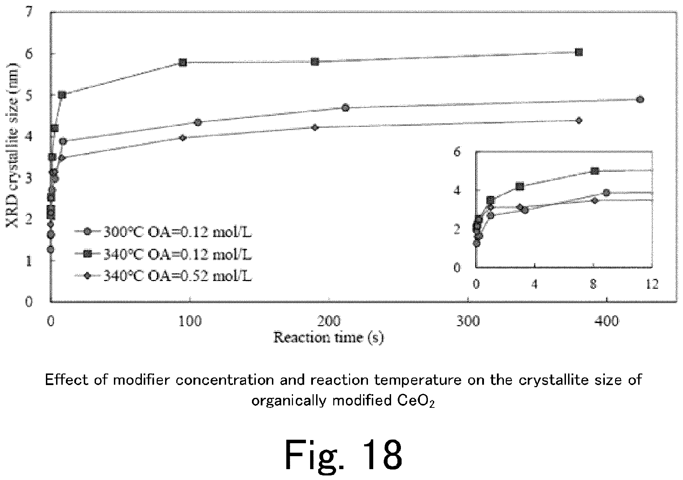

Fig. 18 shows the change in crystallite size versus reaction time when the modifier concentration and reaction temperature were varied. Under all conditions, the crystallite diameter increased rapidly in the initial stage a few seconds from the start of the reaction. On the other hand, the crystallite diameter at which the rapid increase ended was different. The final crystallite diameter also changed accordingly. We believe that reaction temperature and modifier concentration affected the increase in crystallite diameter during the nonclassical nucleation stage, which in turn determined the final particle size.

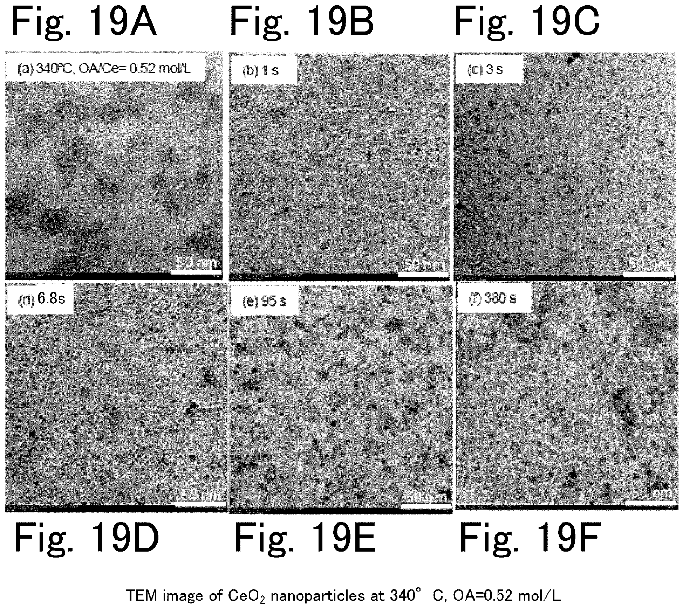

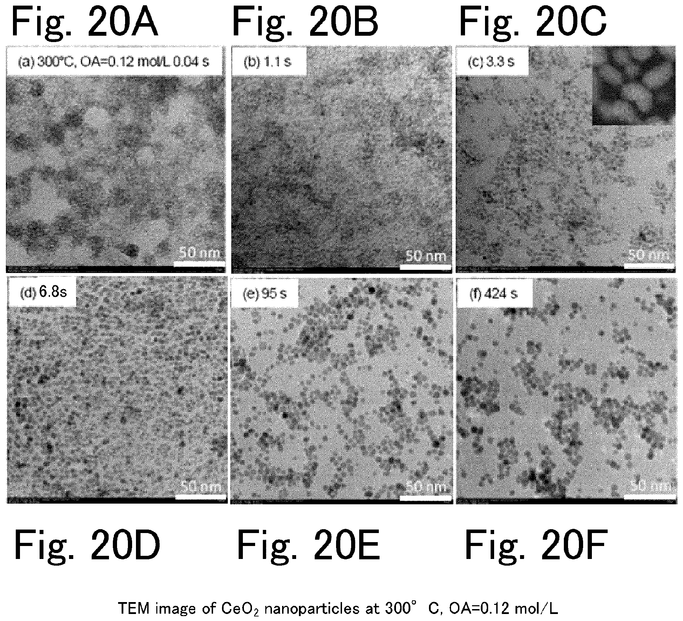

Fig. 19 shows TEM images versus the reaction time at 340°C (OA= 0.52 mol/L). Fig. 20 shows TEM images at 300°C (OA= 0.12 mol/L). In both conditions, the particles are nearly spherical in the initial stage of the reaction and become more cubic and aligned as the reaction time progresses. In particular, at 300°C (OA = 0.12 mol/L), there were many ellipsoidal particles at the reaction time of 3.3 s (Fig. 20C), and we believe that the shape was maintained at that stage due to the coalescence of the two particles. The increase in ellipsoids at smaller particle diameters compared to 340°C (OA = 0.12 mol/L) may be due to a slower deformation rate after particle coalescence at smaller particle diameters due to the lower reaction temperature. We believe this is because particle agglomeration was terminated at the smaller particle size stage, and the agglomerated particles remained spherical. The final particle diameter was also the smallest under this condition.

The evaluation of particle size and particle shape suggested that the growth rate in the nonclassical nucleation stage varies depending on the reaction conditions of temperature and modifier concentration. Kinetic analysis of the aggregation and coalescence growth process was carried out to discuss the control mechanism of nonclassical nucleation. When particles grow by aggregation, the change in the number of particles with respect to time is described as follows:

$$\frac{dN}{dt} = -\left(\frac{1}{2}\right)\beta N^2 \qquad\qquad (10)$$

where $\beta$ is the collision frequency factor (m$^3$/s). By plotting dN/dt against $N^2$ at a certain reaction time, the collision frequency factor $\beta$ at a certain reaction time (particle size) can be obtained.

Smoluchowski calculated the collision frequency factor using a model that assumes a case where a particle collides with another particle and agglomerates due to diffusion by Brownian motion (Non-patent Document 3). When there are i-order clusters of radius $a_i$ (m) and j-order clusters of radius $a_j$ (m), the collision frequency factor is derived as a diffusion process from the i-order cluster to the j-order cluster. The collision frequency factor is expressed by the diffusion constant of the particle and the collision radius, and is derived as follows (Non-patent Document 4):

$$\beta\left(a_i, a_j\right) = 4\pi R_{ij} D_{ij} \tag{11}$$

If the Brownian motion of the two particles is independent, the relative diffusion coefficient is expressed as the sum of the particles' respective diffusion coefficients. Therefore, by substituting the Stokes-Einstein equation for the diffusion coefficients, the following is obtained:

$$\beta\left(a_i, a_j\right) = 4\pi\left(a_i + a_j\right)\left(\frac{kT}{6\pi a_i \eta} + \frac{kT}{6\pi a_j \eta}\right)$$
$$= \frac{2kT}{3\mu}\left(a_i + a_j\right)\left(\frac{1}{a_i} + \frac{1}{a_j}\right) \tag{12}$$

If $a_i = a_j$, then Equation (12) becomes Equation (13), and $\beta$ is constant with respect to changes in particle size (Smoluchowski approximation).

$$\beta\left(a_i, a_j\right) = \frac{8kT}{3\mu} \tag{13}$$

In equation (13), $\beta$ is proportional to temperature, and when applied to this test, $\beta = 2.8 \times 10^{-16}$ at 340°C and $\beta = 2.6 \times 10^{-16}$ at 300°C. The Smoluchowski equation assumes Brownian motion in a stationary fluid, but in reality, the particle size dependence of the diffusion coefficient varies depending on the flow in the reaction field. In turbulent flow, the diffusion coefficient does not decrease with increasing particle size, but the collision frequency factor is said to increase with increasing particle size (Non-patent Document 5). In this test, the Re number of the reaction field at 340°C is 3100, which is between laminar and turbulent flow, but the Re number of the mixing zone is 19000, and turbulent eddies are generated. Therefore, the collision frequency factor could increase with an increase in particle size.

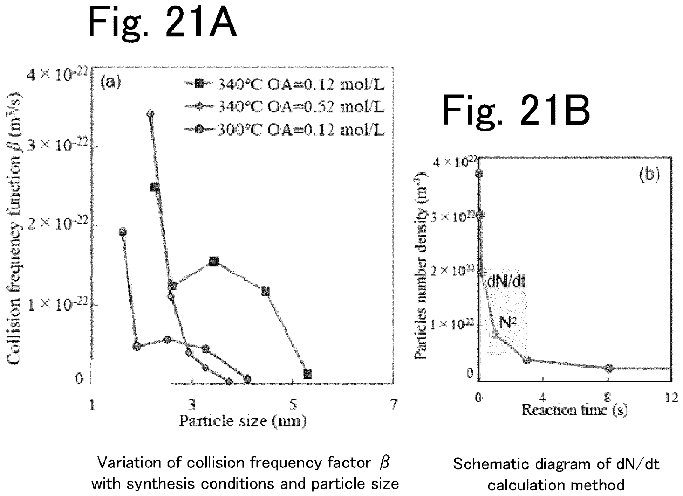

Fig. 21A shows the results of $\beta$ calculated for particle diameters under different reaction conditions. The dN/dt was calculated using the number of particles before and after the reaction time, for which $N^2$ was calculated, as illustrated in Fig. 21B.

In all conditions, $\beta$ decreased significantly with increasing particle size. Cohesion theory states that $\beta$ does not change, or rather increases, with increasing particle size; thus, it cannot be explained solely by cohesion theory. In addition, the initial collision frequency factor was at most $3.5 \times 10^{-22}$, which is less than 1/100,000 of the collision frequency factor, assuming Brownian motion. This indicates that, as shown in Test 1, agglomerated particles need to cross an energy barrier to actually coalesce with each other, and the effect of this is significant. The fact that the collision frequency factor decreased significantly as the particle size increased supports the assumption in Example 1 that the energy barrier to coalescence increases as the particle size increases.

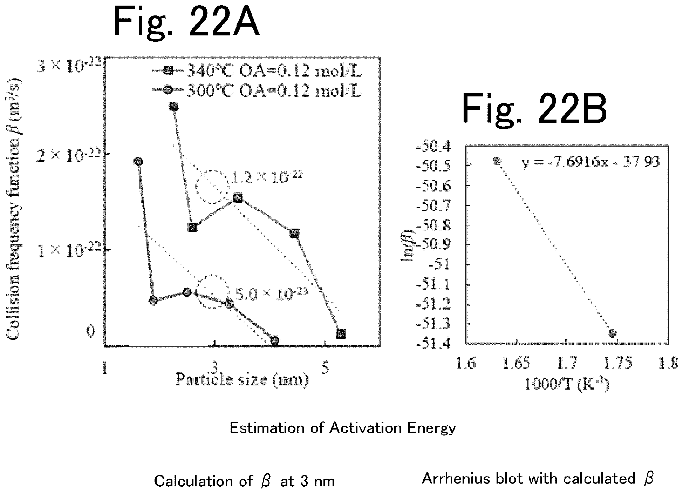

Decreasing the reaction temperature decreased $\beta$ at the same particle size. This result also suggests that the energy barrier influences coalescence. For particles to aggregate, it is necessary for them to undergo a molecular chain desorption reaction and hydroxide dehydration condensation at the moment they approach each other. In such a case, the reaction rate may affect the frequency of coalescence, and the dependence of $\beta$ on the reaction temperature may follow the Arrhenius type. In fact, the graph in Fig. 21A was linearly approximated (Fig. 22A), the activation energy was estimated from $\beta$ at a particle size of 3 nm, and the activation energy was calculated to be 64 kJ/mol (see Fig. 22B). This is close to the activation energy when the coalescence rate is not the diffusion rate but the reaction rate. The evaluation of the effect of temperature makes it clearer that the collision frequency factor affects the probability that the agglomerated particles will actually coalesce.

As the octanoic acid concentration increased, $\beta$ decreased more rapidly with respect to particle size. In addition, when the particle size exceeded 3 nm, $\beta$ decreased more than when the temperature was lowered to 300°C, and the particle size remained smaller. On the other hand, the initial stage, when the particle size was below 2 nm, showed a specific behavior of high $\beta$. The decrease in $\beta$ with the increasing modifier concentration was due to the decrease in the probability of desorption of octane molecules during collision. Considering that the desorption of octanoic acid molecules modified on the surface is necessary for the coalescence process of particles, the high octanoic acid concentration in the surrounding area decreases the desorption rate, resulting in a decrease in the probability of coalescence.

<Example 4> Relationship between reaction time and conversion ratio

[Example 4-1] Reaction temperature: 300°C

Cerium (IV) oxide nanoparticles were recovered using the method described in Example 3-2.

[Example 4-2] Reaction temperature: 340°C

Cerium (IV) oxide nanoparticles were recovered using the method described in Example 3-1.

[Comparative Example 4-1] When the raw material solution contains cerium nitrate

[Supercritical hydrothermal synthesis]

Cerium nitrate was used instead of cerium octanoate (IV) as the composition of the feed solution supplied to the distribution-type hydrothermal apparatus. These cerium molar concentrations were the same as in Example 2. The ratio of octanoic acid to cerium (IV) (molar ratio) was set to 16, the reaction temperature was set to 340°C, and the reaction times for supercritical hydrothermal synthesis were 0.038 s and 95 s. Other than the above, supercritical hydrothermal synthesis was performed using the method described in Example 2.

[Recovery of products]

Cerium (IV) oxide nanoparticles were recovered using the method described in Example 2.

[Comparative Example 4-2] When the raw material solution contains cerium (IV) ammonium nitrate

Cerium (IV) oxide nanoparticles were recovered using the method described in Comparative Example 4-1, except that cerium (IV) ammonium nitrate was used instead of cerium nitrate as the composition of the feed solution supplied to the distribution-type hydrothermal apparatus.

[Comparative Example 4-3] When the raw material solution contains cerium (IV) hydroxide

Cerium (IV) hydroxide was used instead of cerium nitrate as the composition of the feed solution supplied to the circulation-type hydrothermal apparatus.

[Evaluation]

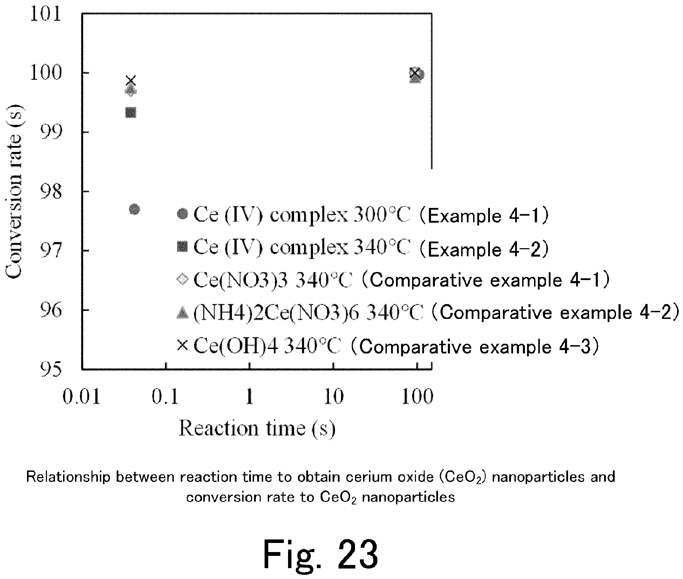

The relationship between the reaction time to obtain cerium (IV) oxide nanoparticles and the conversion rate to cerium (IV) oxide nanoparticles was plotted for each of Examples 4-1 and 4-2 and Comparative Examples 4-1 through 4-3. The results are shown in Fig. 23, which confirms that even when the reaction time was 0.038 s, the conversion ratio was approximately 98% or higher.

<Test 5> Control of average primary particle diameter by the valence of metal constituting organometallic complex

[Test 5-1] Relatively large valence (in the case of cerium, tetravalent)

[Synthesis of cerium (IV) octanoate]

The product of cerium octanoate (IV) was recovered using the method described in Example 1.

[Supercritical hydrothermal synthesis]

Supercritical hydrothermal synthesis was performed using the method described in Example 1, except that the reaction time was 10 min.

[Recovery of products]

Cerium (IV) oxide nanoparticles were recovered using the method described in Example 1.

[Test 5-2] Relatively small valence (trivalent in the case of cerium)

[Synthesis of cerium (III) octanoate]

Cerium (III) octanoate product was recovered using the method described in Test 5-1, except that 0.1 M cerium (III) nitrate solution was used instead of cerium (IV) ammonium nitrate solution.

[Supercritical hydrothermal synthesis]

Supercritical hydrothermal synthesis was carried out using the method described in Test 5-1, except that 143 mg of cerium (III) octanoate was used instead of 112 mg of cerium (IV) octanoate.

[Recovery of products]

Cerium (III) oxide nanoparticles were recovered using the method described in Test 5-1.

[Characteristic evaluation method]

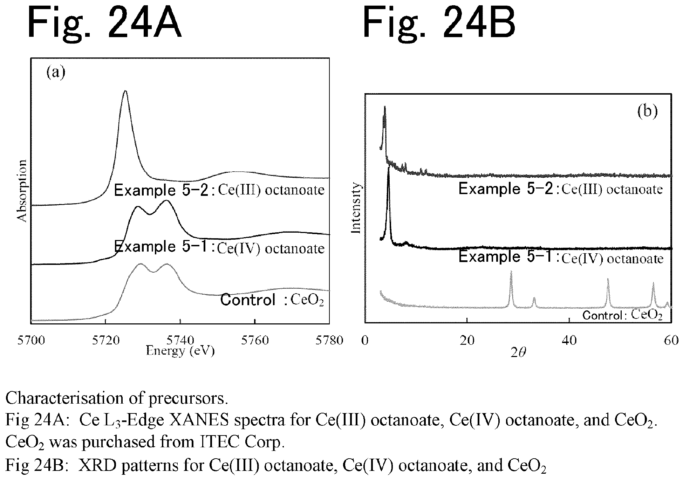

Ce L3-edge XANES spectra were collected using the transmission method with a BN binder on the pellet at SPring-8 beamline BL14B2. Incident X-rays were monochromatized using Si (111) crystals. The spectrum of the precursor obtained in Test 5 is shown in Fig. 24A.

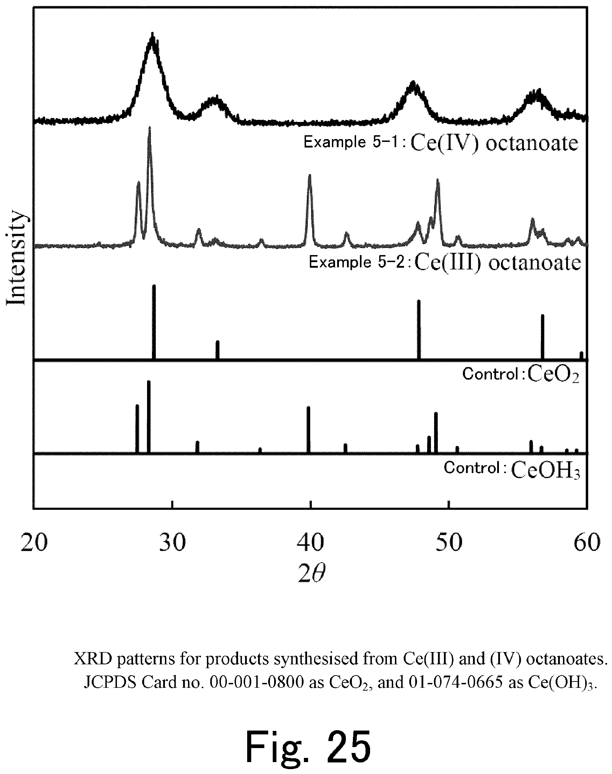

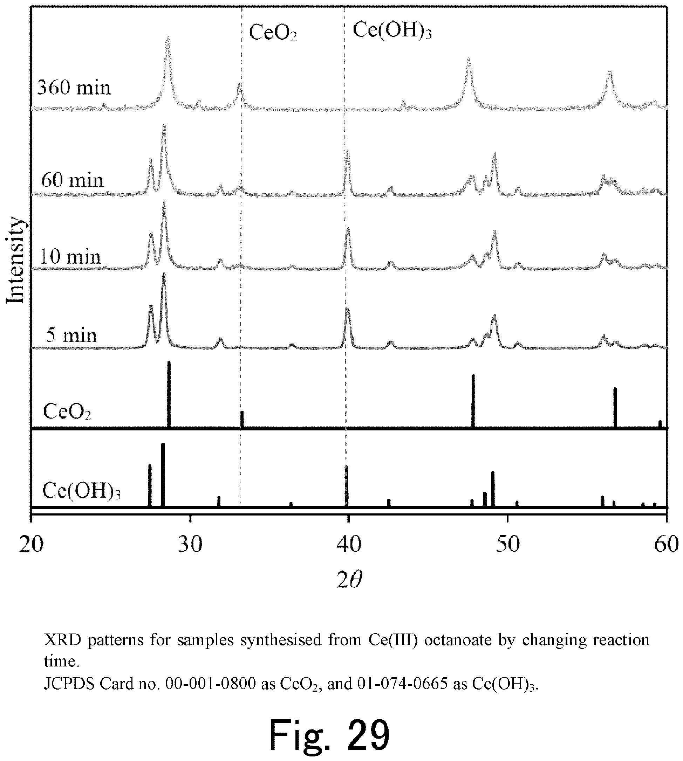

The XRD patterns of the products were recorded using a SmartLab 9MTP (Rigaku Corporation) equipped with a Cu K$\alpha$ ($\lambda$ = 1.5418 Å) source. The $2\theta$ scan speed was 3°/min. The XRD pattern of the precursor obtained in Test 5 is shown in Fig. 24B. The XRD pattern of the product synthesized from the precursor is shown in Fig. 25. The XRD patterns of the products synthesized from cerium (III) octanoate with different reaction times are shown in Fig. 29.

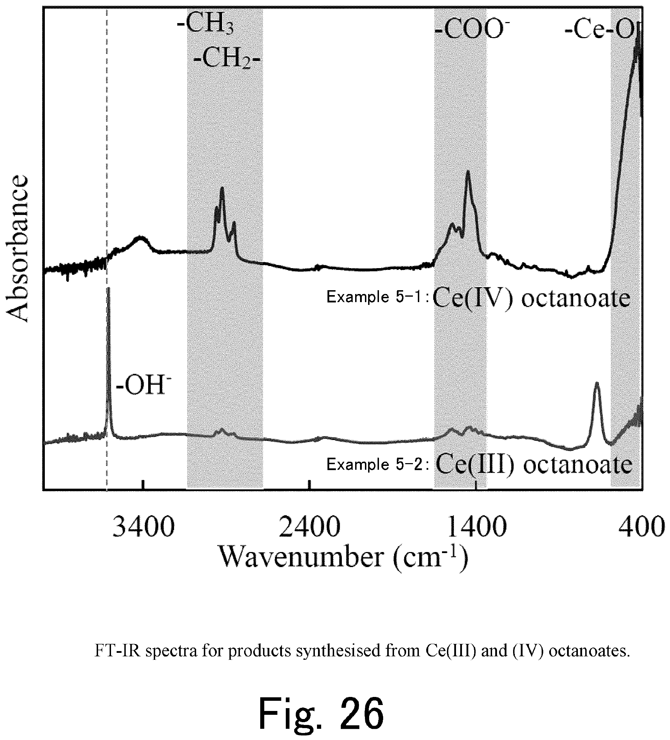

Fourier transform infrared (FT-IR) spectra were recorded using an infrared spectrometer (FT/IR-4200, Japan Spectroscopic Corporation). Analytical samples were prepared using the KBr method. The FT-IR spectra of the products synthesized from the precursor are shown in Fig. 26.

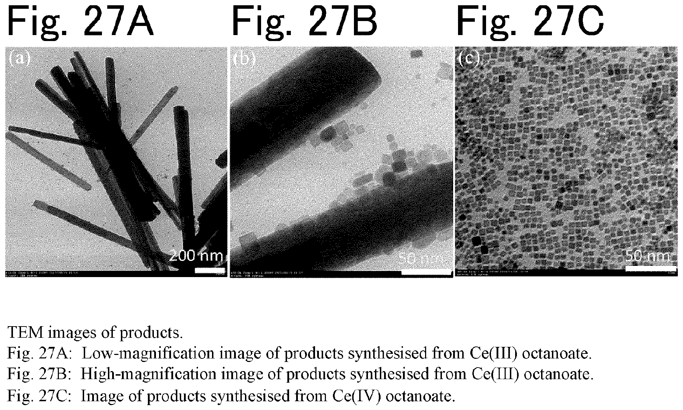

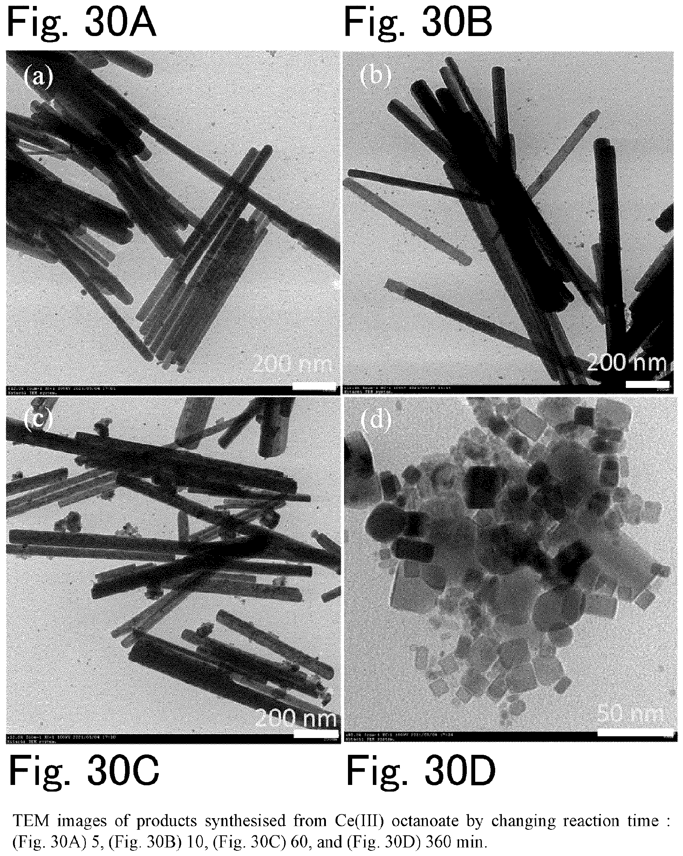

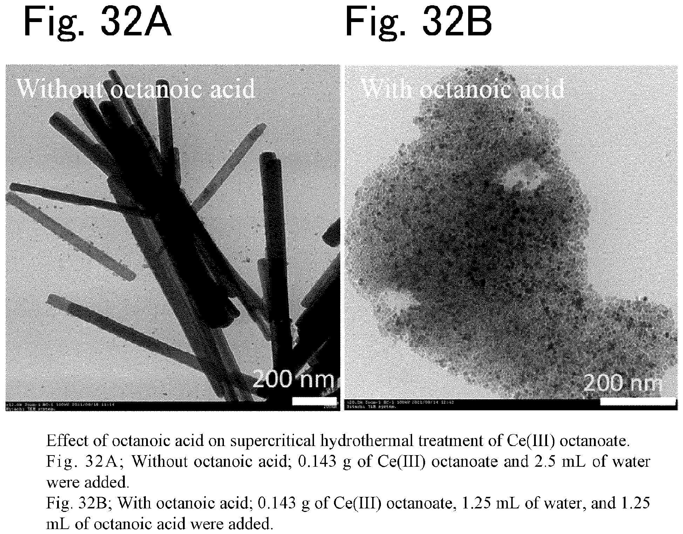

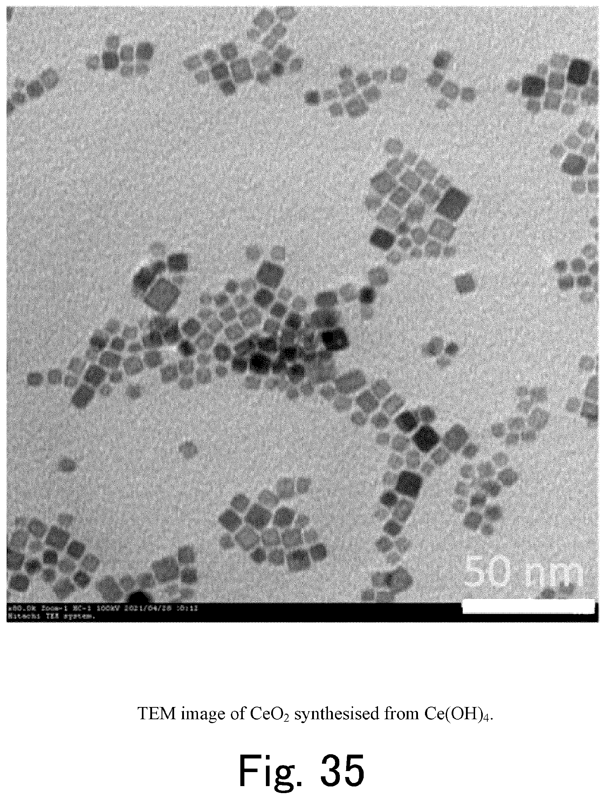

The morphology of each product was observed using TEM (H-7650, Hitachi High-Technologies Corporation). The morphology of the products synthesized from the precursor is shown in Fig. 27. The morphology of the product synthesized from cerium (III) octanoate at different reaction times is shown in Fig. 30. The morphology of the product synthesized from cerium (III) octanoate by changing the amount of octanoic acid added is shown in Fig. 32. The morphology of the product synthesized from $Ce(OH)_4$ is shown in Fig. 35. The TEM average primary particle diameter of the particles was measured from the diagonal of a square on one side.

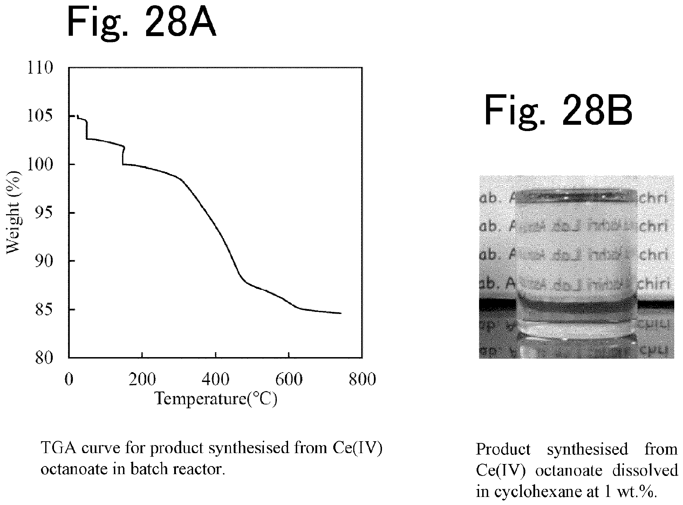

The grafting density of octanoic acid ligands onto the $CeO_2$ surface was calculated according to the method described in Non-patent Document 6 using information obtained from thermogravimetric analysis (TGA) weight loss measurements, particle volume, and TEM surface area, assuming that the particles were cubic in shape. The results are shown in Fig. 28. Here, TGA was performed using a thermogravimetric analyzer (DTG-60AH, Shimadzu Corporation) with N2 (99.9999%) flowing at 50 mL/min. The temperature was first increased to 150°C to remove residual adsorbed water and then increased to 750°C at 10°C/min.

[Result]

The valence of Ce in the precursor complexes was confirmed by Ce L3-edge X-ray absorption near edge structure (XANES) spectra (Fig. 24A). The spectrum of cerium octanoate (IV) derived from CAN showed no peak at 5724 eV but two peaks at 5730 eV and 5738 eV, suggesting that Ce is tetravalent (Non-patent Documents 7 and 8). In the synthesis of cerium octanoate, no $CeO_2$ was formed, which is supported by the absence of $CeO_2$-related peaks in the XRD pattern (Fig. 24B).

The crystal structure of the hydrothermally synthesized product was characterized by XRD (Fig. 25). When cerium (III) octanoate was used as a precursor, the XRD pattern of the product was almost identical to that of hexagonal $Ce(OH)_3$, and the weak peak at 33.1° was due to $CeO_2$. When cerium (IV) octanoate was used as a precursor, the XRD patterns of the products were consistent with those of pure $CeO_2$. These results suggest that pure $CeO_2$ nanoparticles can be synthesized from cerium octanoate (IV).

The FT-IR spectra results were consistent with the XRD pattern (Fig. 26). When cerium (III) octanoate was used as a precursor, the FT-IR spectrum showed a band at 3608 cm$^{-1}$, which was assigned to the stretching mode of the $Ce(OH)_3$ hydroxyl group. When cerium (IV) octanoate was used as a precursor, spectra corresponding to organically modified cerium (IV) oxide $CeO_2$ nanoparticles were obtained (Non-patent Document 9). The 2700-3100 cm$^{-1}$ range band was attributed to modified octanoate; the peak at 2955 cm$^{-1}$ was assigned to the asymmetric -CH$_3$ stretching mode; the peak at 2955 cm$^{-1}$ was assigned to the asymmetric -CH$_3$ stretching mode; and the peaks at 2922 cm$^{-1}$ and 2851 cm$^{-1}$ were assigned to asymmetric and symmetric -CH$_2$- stretching modes, respectively. A 1444 cm$^{-1}$ peak was assigned to the scissoring bending band of the -CH$_2$-group in the alkyl chain. In both samples, the band associated with the free carboxyl group (-COOH) of the original octanoic acid (usually detected at approximately 1700 cm$^{-1}$) was not observed, indicating that the unmodified octanoic acid was washed out of the product. The peaks at 1530 cm$^{-1}$ and 1408 cm$^{-1}$ are assigned to asymmetric and symmetric carboxylate (COO-) stretching modes, respectively, suggesting that organically modified $CeO_2$ from cerium (IV) octanoate is synthesized from cerium (IV) octanoate.

Fig. 27 shows a TEM image of the synthesized particles. Most of the particles synthesized from cerium octanoate (III) were rod-shaped, 20-100 nm in diameter, and 0.4-2 $\mu$m in length (Fig. 27A). The TEM image also shows traces of polygonal or cubic particles of 20 nm (average) diameter (Fig. 27B), suggesting that $CeO_2$ was formed as a byproduct, which was also confirmed by the corresponding XRD pattern (Fig. 25).

The nanoparticles synthesized from cerium octanoate (IV) were uniformly cubic and had an average diameter of 5.3 nm (Fig. 27C). The graft density of $CeO_2$ synthesized from cerium (IV) octanoate was calculated from TEM particle size and TGA weight loss data to be 2.8 nm$^{-2}$ (Fig. 28A), which is similar to previously reported $CeO_2$ synthesized from $Ce(OH)_4$ (Non-patent Document 9). The graft density was high enough to disperse $CeO_2$ nanoparticles in organic solvents (Fig. 28B). The valence of Ce in octanoate affected the products, and uniform $CeO_2$ nanoparticles were synthesized only from octanoate Ce (IV).

[Discussion]

[Mechanism of nanoparticle formation from cerium (III) octanoate.]

It has been reported that when trivalent cerium precursors are treated under hydrothermal/solvothermal conditions, the oxidizing power of the counter-anion changes the final product and particle shape (Non-patent Documents 10 and 11). $CeO_2$ was formed from $Ce(NO_3)_3$, which exhibited high anion oxidation power. However, $Ce(OH)_3$ was formed from cerium acetate ($Ce(Ac)_3 \cdot nH2O$), which showed low anion oxidation power in water at 200°C (Non-patent Document 11). The oxidation power of cerium (III) octanoate through the octanoate anion should be low because this anion is similar to the carboxylic acid anion of $Ce(Ac)_3$. However, since the oxidizing power of water increases with temperature, an increase in water temperature may shift the reaction equilibrium toward oxide formation (Non-patent Document 12), making it difficult to predict the reaction products. In this test case, the main product was found to be $Ce(OH)_3$ from cerium (III) octanoate, even after a 10-min reaction in supercritical water.

To investigate the possibility of the oxidation of $Ce(OH)_3$ by water, cerium (III) octanoate was treated with an extended reaction time. Fig. 29 shows the XRD patterns of the samples. The $CeO_2$ ratio increased with increasing reaction time. This may be due to the slow dissolution of $Ce(OH)_3$ intermediates while $CeO_2$ particles were synthesized and reprecipitated. This result indicates that $Ce(OH)_3$ crystals were formed during the heating period of the reaction system and transformed into thermodynamically more stable $CeO_2$ in the higher temperature range, where water can serve as an oxidant. These $CeO_2$ particles were larger than those obtained from cerium (IV) octanoate (Fig. 30), suggesting Ostwald ripening.

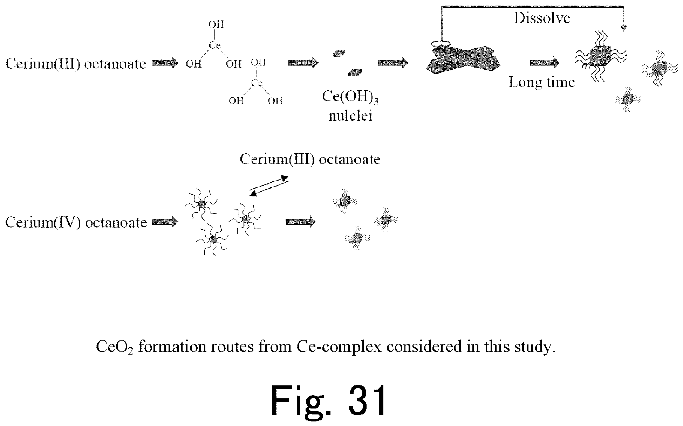

Fig. 31 shows the formation pathway of $CeO_2$ studied in this test case. When cerium (III) octanoate was hydrothermally treated, it was completely hydrolyzed to form $Ce(OH)_3$. Furthermore, $Ce(OH)_3$ rapidly crystalized into an intermediate and took a long time to redissolve. These results indicate that both the formation of $Ce(OH)_3$ intermediates and the necessary Ce oxidation prevented the formation of uniform $CeO_2$.

Previous studies on the hydrothermal treatment of $CeO_2$ with octanoic acid have reported that Ce ions on the surface bonded directly to carboxylic acids to form carboxylate Ce (III), which was later reprecipitated onto the $CeO_2$ surface (Non-patent Document 13). Under these experimental conditions, a significant amount of carboxylic acid remained in the reaction phase compared to the concentration of dissolved carboxylic acid Ce (III). The carboxylic acid may inhibit the formation of $Ce(OH)_3$. To investigate this effect, octanoic acid was added to the reactor. The products were observed using TEM (Fig. 32). When octanoic acid was added, only $CeO_2$ particles were synthesized, suggesting that octanoic acid suppressed the formation of $Ce(OH)_3$. However, these particles were relatively heterogeneous and larger than those synthesized from cerium (IV) octanoate (Fig. 27C). Both the reaction rate and the supersaturation of the raw material cerium octanoate (III) may decrease with increasing octanoic acid concentrations. In fact, the reaction yield decreased with increasing octanoic acid concentrations. Uniform nanoparticles could not be obtained from cerium (III) octanoate by changing the carboxylic acid concentration.

[Uniform CeO$_2$ nanoparticles obtained from cerium octanoate (IV)]

When cerium octanoate (IV) was hydrothermally treated, Ce(OH)$_4$ was not detected as an intermediate. This was thought to be due to the instantaneous dehydration of hydrolyzed Ce (IV) octanoate producing CeO$_2$ crystals, because unlike the reaction of Ce (III) complexes, the oxidation of Ce is not necessary to produce CeO$_2$ from Ce (IV) complexes. Because the complex precursor was homogeneous, uniform CeO$_2$ nanoparticles could be synthesized from cerium (IV) octanoate without forming bulk hydroxides.

Cerium (III) octanoate was synthesized as a byproduct of CeO$_2$ particle growth, even when cerium (IV) octanoate was used as a precursor. Cerium (III) octanoate was extracted with a benzene solution after the reaction.

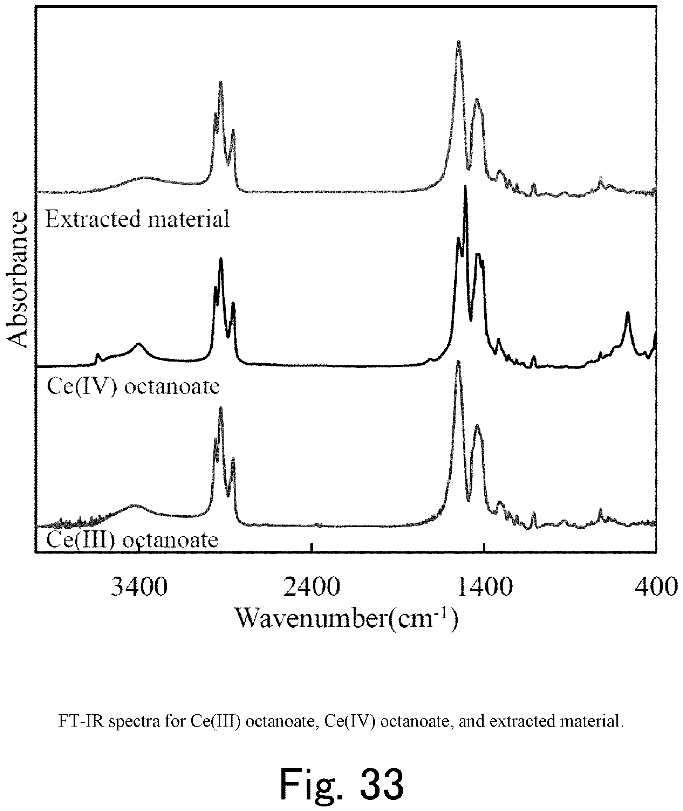

Extraction using a benzene solution was performed using the following method. A total of 112 mg of cerium octanoate (IV), 1.07 mL of octanoic acid, and 2.5 mL of water were placed in a reaction vessel and heated at 400°C for 20 min. After the reaction, the vessel was quenched in a water bath at room temperature. The product was recovered with benzene. After removing residual water, methanol was added to the benzene solution. The volume ratio of benzene to methanol was 1:1. The solution was centrifuged, and the supernatant was collected and evaporated, leaving residual octanoic acid and Ce (III) complexes. The residual octanoic acid was washed away with acetone. The cerium (III) octanoate was then dried at 70°C for 12 h. The FT-IR spectrum of the extract is shown in Fig. 33.

Cerium octanoate (III) is thermodynamically more stable than cerium octanoate (IV) and is present at higher temperatures. The carboxylate octanoate group may act as an electron donor during Ce dissolution and may reduce the concentration of Ce ions. Presumably, homogeneous CeO$_2$ particles were formed from cerium octanoate (IV) and grew with the dissolution and precipitation of cerium octanoate (III) (Fig. 31). The precipitation of cerium (III) octanoate may be coupled with the oxidation of Ce (III) ions by water (Non-patent Document 14). This finding provides new insight into the valence of Ce through the synthesis of CeO$_2$.

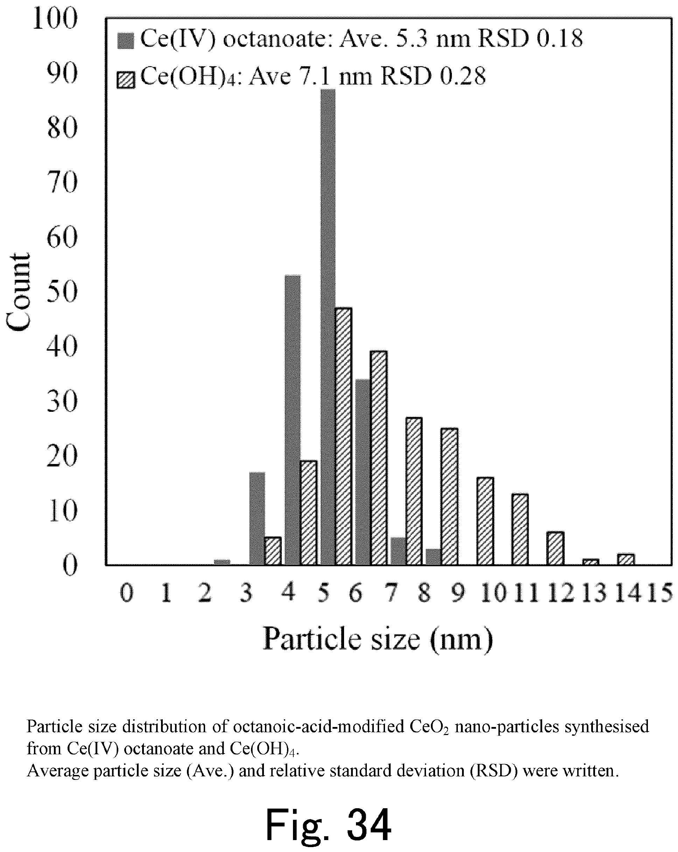

Fig. 34 compares the sizes of particles synthesized from cerium (IV) octanoate and Ce(OH)$_4$. The TEM image of CeO$_2$ derived from Ce(OH)$_4$ is shown in Fig. 35. When CeO$_2$ nanoparticles were synthesized from cerium (IV) octanoate, the average particle size was 5.3 nm, the relative standard deviation was 0.18, and the coefficient of variation was 0.034. When CeO$_2$ nanoparticles were synthesized from Ce(OH)$_4$, the average particle size was 7.1 nm, the relative standard deviation was 0.28, and the coefficient of variation was 0.039. Clearly, the CeO$_2$ particles synthesized from cerium (IV) octanoate were smaller and more uniform. In previous studies, the average particle size of octanoic acid-modified CeO$_2$ synthesized from Ce(OH)$_4$ was reported to be 9.5 nm (Non-patent Document 9) or 6.0 nm (Non-patent Document 15). The particles synthesized from cerium (IV) octanoate in this test case were smaller. When Ce(OH)$_4$ was used as the precursor, some of the precursor interacted with the organic modifier and shifted to the organic phase with increasing temperature, which may have resulted in a nonuniform precursor structure and a wider particle distribution. However, cerium octanoate (IV) became a homogeneous monomer with increasing temperature, inducing uniform nucleation and the formation of smaller particles.

[Conclusion]

The mechanism of CeO$_2$ formation in supercritical hydrothermal synthesis was elucidated by experiments using Ce precursors of different valences. Cerium octanoate (III) and octanoic acid (IV) were prepared as precursors and treated under supercritical hydrothermal conditions. Ce(OH)$_3$ nanorods were formed from cerium (III) octanoate, followed by its transformation to CeO$_2$. Uniform nanoparticles were not obtained because the formation of Ce(OH)$_3$ and the oxidation of Ce occurred during the process. On the other hand, when CeO$_2$ nanoparticles were synthesized directly from cerium (IV) octanoate, no hydroxide crystals were formed. The CeO$_2$ nanoparticles synthesized from cerium (IV) octanoate were smaller and more uniform than those synthesized from cerium (IV) hydroxide Ce(OH)$_4$.

<Test 6> Effect of modifier molecular chain length

In Tests 1-5, it was found that the particle size of homogeneously nucleated organically modified CeO$_2$ was affected by nonclassical nucleation. The modifier changed the tendency of particle growth in nonclassical nucleation, and it was suggested that the modifier suppressed the growth when the particle size exceeded 3 nm.

The effect of the layer created by the modifier may vary depending on the molecular chain length of the modifier. For example, the modifier's stability on the surface of an inorganic compound and its effect on particle dispersion may change depending on the molecular chain length. In this study, C6, C10, and C14 cerium-carboxylate complexes were synthesized in addition to C8 cerium-carboxylate complexes, and surface-modified CeO$_2$ nanoparticles were synthesized using these as precursors. The effect of modifier molecular chain length was clarified by measuring the difference in particle size at the same modifier concentration.

[Example 6-1] Cerium carboxylate complex of C6 (cerium hexanoate)

[Synthesis of cerium (IV) hexanoate]

The precursor of cerium hexanoate (IV) was obtained using the method described in Example 1, except that sodium hexanoate solution was used instead of sodium octanoate solution.

[Supercritical hydrothermal synthesis]

Supercritical hydrothermal synthesis was performed using the method described in Example 1, except that the precursor was cerium hexanoate (IV), the modifier was hexanoic acid, and the specified times were 0.04, 1, 3, 8, 95, and 380 s.

[Product recovery]

The product of cerium (IV) hexanoate nanoparticles was recovered using the method described in Example 1.

[Example 6-2] Cerium carboxylate complex of C8 (cerium octanoate)

[Synthesis of cerium (IV) octanoate]

The precursor of cerium (IV) octanoate was obtained using the method described in Example 1.

[Supercritical hydrothermal synthesis]

Supercritical hydrothermal synthesis was performed using the method described in Example 1, except that the specified times were 0.04, 1, 3, 8, 95, and 380 s.

[Product recovery]

The product of cerium (IV) octanoate nanoparticles was recovered using the method described in Example 1.

[Example 6-3] Cerium carboxylate complex of C10 (cerium decanoate)

[Synthesis of cerium (IV) decanoate]

The precursor of cerium (IV) decanoate was obtained using the method described in Example 1, except that aqueous sodium decanoate solution was used instead of aqueous sodium octanoate solution.

[Supercritical hydrothermal synthesis]

Supercritical hydrothermal synthesis was performed using the method described in Example 1, except that the precursor was cerium (IV) decanoate, the modifier was decanoic acid, and the specified times were 0.04, 1, 3, 8, 95, and 380 s.

[Product recovery]

The product of cerium (IV) decanoate nanoparticles was recovered using the method described in Example 1.

[Example 6-4] Cerium carboxylate complex of C14 (cerium myristate)

[Synthesis of cerium (IV) myristate]

The precursor of cerium myristate (IV) was obtained using the method described in Example 1, except that sodium myristate solution was used instead of sodium octanoate solution.

[Supercritical hydrothermal synthesis]

Supercritical hydrothermal synthesis was performed using the method described in Example 1, except that the precursor was cerium myristate (IV), the modifier was myristic acid, and the specified times were 0.04, 1, 3, 8, 95, and 380 s.

[Product recovery]

The product of cerium (IV) myristate nanoparticles was recovered using the method described in Example 1.

[Evaluation]

[Cerium carboxylate complex (IV) synthesized as a precursor]

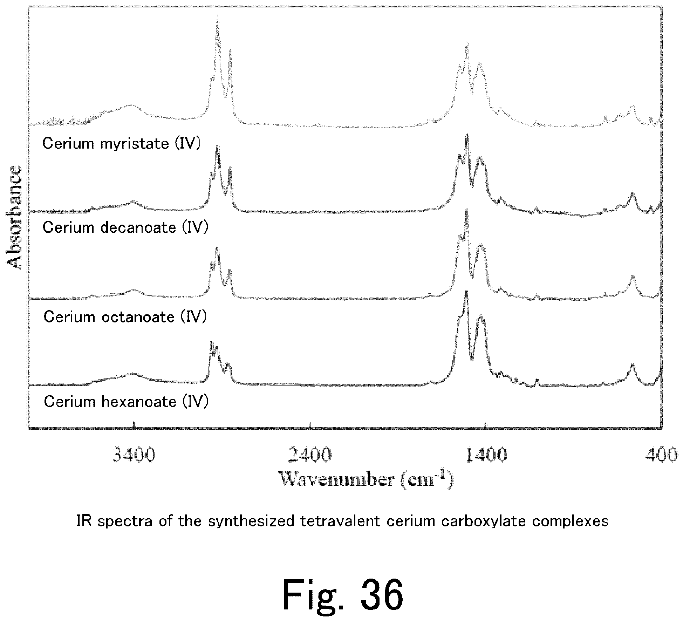

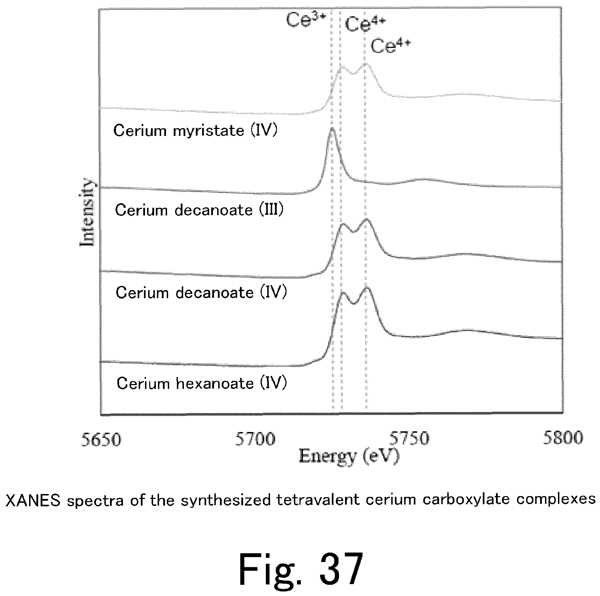

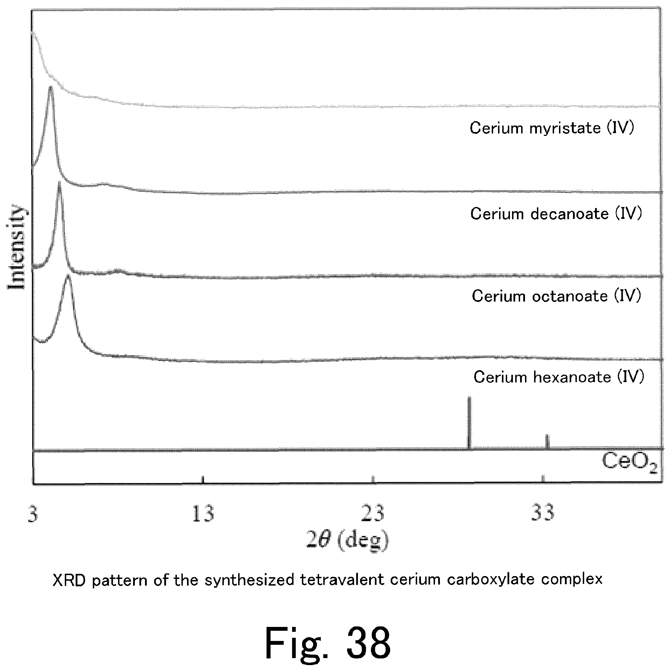

First, the cerium-carboxylate complex (IV) synthesized as a precursor was evaluated. Fig. 36 shows the IR spectra measurement results of the complexes. The peak shapes of the carboxylate bonding region from 1400 to 1600 $cm^{-1}$ were similar, even though the molecular chain lengths differed. On the other hand, the ethyl group peak intensity in the 2800-3200 $cm^{-1}$ region increased with increasing molecular chain length, indicating that complexes with different molecular chain lengths were obtained. The fact that a tetravalent Ce complex had been obtained was confirmed by comparing the CeL3 absorption edge XANES spectrum with that of the trivalent Ce complex (Fig. 37). The absence of $CeO_2$ formation during the precursor synthesis stage was confirmed by the XRD pattern (shown in Fig. 38). These analyses confirmed that C6 hexanoic acid Ce (IV), C10 decanoic acid Ce (IV), and C14 myristic acid Ce (IV) were obtained using the same synthetic method as octanoic acid Ce (IV).

[Ce-carboxylate complex (IV) after supercritical hydrothermal synthesis]

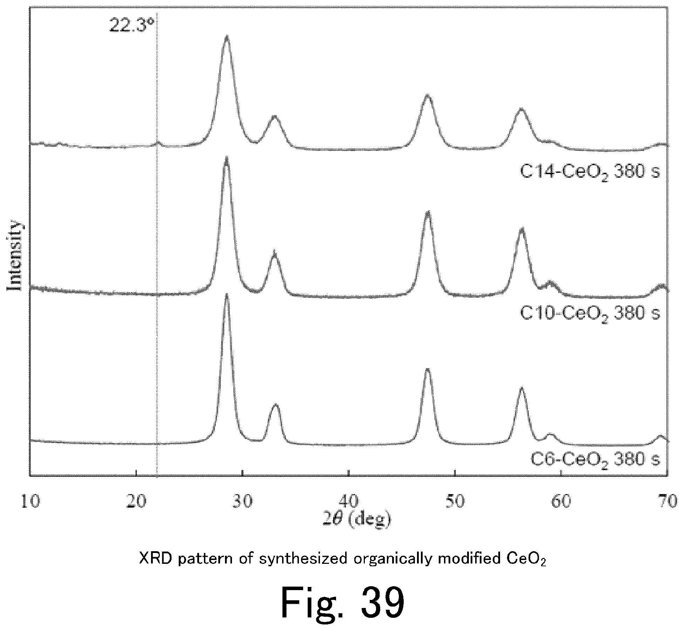

The XRD pattern for the synthesized organically modified $CeO_2$ at 380 s-the longest reaction time-is shown in Fig. 39. Hereafter, hexanoic acid-modified $CeO_2$ is denoted as $C6-CeO_2$, octanoic acid-modified $CeO_2$ is denoted as $C8-CeO_2$, decanoic acid-modified $CeO_2$ is denoted as $C10-CeO_2$, and myristic acid-modified $CeO_2$ is denoted as $C14-CeO_2$. The nanoparticles of C6 and $C10-CeO_2$ were synthesized without impurities, and $C8-CeO_2$ was present at 22.3°. As an attribution of this peak, we considered the possibility of a trivalent Ce complex, Ce (III) myristate. As clarified in Test 5, even when $CeO_2$ is synthesized using a tetravalent Ce complex as a precursor, it grows while repeatedly dissolving and precipitating as a trivalent Ce complex during particle growth. Therefore, the trivalent Ce complexes formed during distribution-type synthesis can remain and be recovered. In the case of $C8-CeO_2$ synthesis, even if trivalent octanoic acid Ce (III) is formed, it can be removed by dissolution in a benzene-methanol solution. On the other hand, the Ce (III) myristate is not dissolved in this solution because of its different molecular structure. The solubility of C6, C8, and C10 complexes in benzene-methanol solution was examined by synthesizing various trivalent Ce complexes, and it was found that C6, C8, and C10 complexes were soluble in the benzene-methanol solvent, while the C14 complex had low solubility in that solvent.

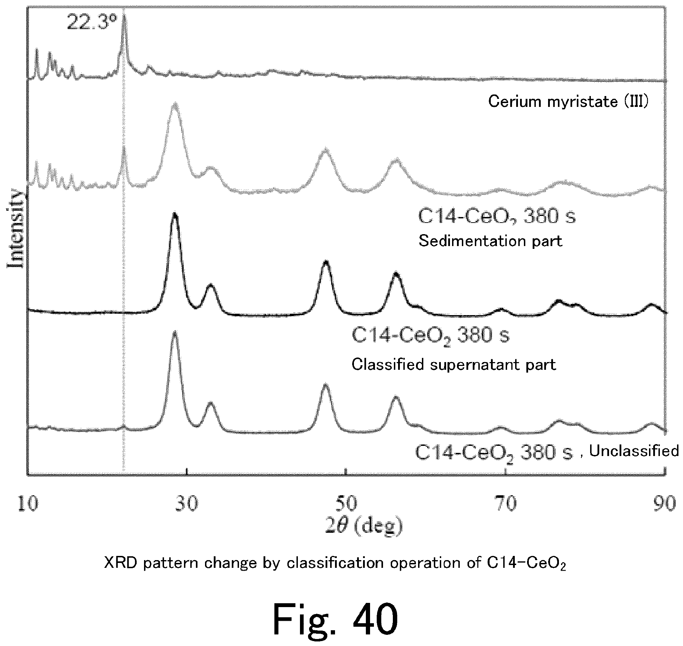

We considered that the Ce (III) myristate remained in the $C14-CeO_2$ synthesis, and we classified the complexes to confirm this and to separate impurities. Ce (III) myristate precipitates in cyclohexane. In contrast, $C14-CeO_2$ nanoparticles with a high modification density are dispersed in cyclohexane. Therefore, impurities were separated by standing the cyclohexane for more than 12 h and collecting the supernatant with a pipette. The XRD pattern was confirmed, and the results are shown in Fig. 40. The cyclohexane precipitate clearly contained many impurities, and the pattern of the impurity portion was found to be in good agreement with that of the Ce (III) myristate. In contrast, the classified supernatant no longer had an impurity peak. These results indicate that Ce (III) myristate was formed during the synthesis of $C14-CeO_2$ and could not be washed out using the same method as for $C8-CeO_2$. There was no solvent in this study that dissolved Ce (III) myristate well, and impurity-free $C14-CeO_2$ was obtained by collecting the supernatant of the fractionation. In this study, XRD crystallite size measurements were performed on the unfractionated sample. TEM images were taken from the unclassified samples that reflected the overall shape of the synthesized particles.

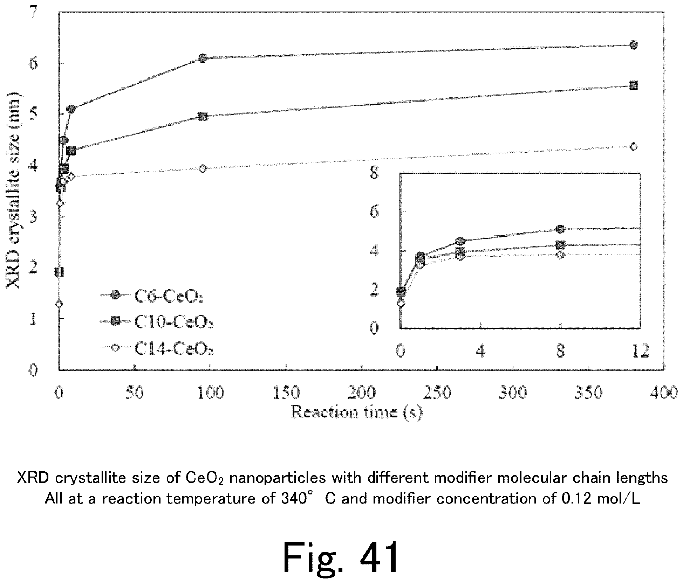

Fig. 41 shows the change in the synthesized $CeO_2$ nanoparticles versus the reaction time. As with $C8-CeO_2$, particles of about 2 nm were obtained in the initial stage of nucleation, and the crystallite diameter increased rapidly in the initial stage of formation within 8 s of residence time. Thereafter, the growth rate of the crystallite diameter decreased. The longer the modifier, the more the crystallite size decreased during the same reaction time. This indicates that the nanoparticles grew due to a nonclassical nucleation mechanism, and the longer the modifier, the lower the frequency of aggregation and coalescence, resulting in a decrease in particle size.

We believe that the stability of the modified molecular chains on the surface is one of the reasons for the lower aggregation and coalescence frequencies with longer modifiers. In the surface modification of metal oxides with linear carboxylic acids, it is known that the longer the hydrocarbon molecular chain is, the more stable the modified molecule becomes due to intermolecular interactions between the modified molecular chains (Non-patent Documents 16 and 17). It is known that the melting point of linear carboxylic acid molecules increases as the molecular chain length increases and that their mobility and crystallinity change at around room temperature (Non-patent Document 18). Although this experiment was conducted at a high temperature of 340°C, we believe that the kinetics of the modifier will be different. The modifier's stability may have changed its rate of carboxylic acid desorption, which is necessary for particle coalescence.

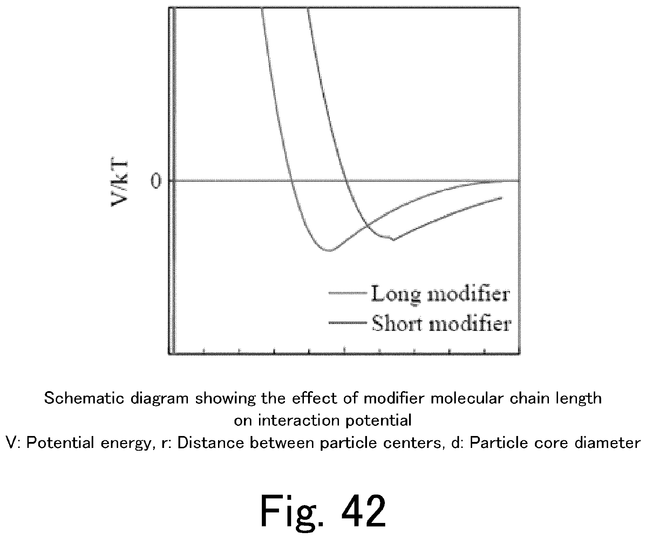

Another factor could be that the modifier's attractive action may have varied with its length. When the reaction field relationship for organically modified nanoparticles is a poor solvent, the potential energy, which represents the interaction between particles, can have valleys that exhibit an attractive effect (Non-patent Documents 19 and 20). As shown schematically in Fig. 42, the range and strength of the attractive effect depend on the length of the modifier (Non-patent Documents 19 and 20). The longer the molecular chain, the wider the range of the attractive action, but the valley of the attractive action is located over a longer distance, which may have further reduced the probability of coalescence.

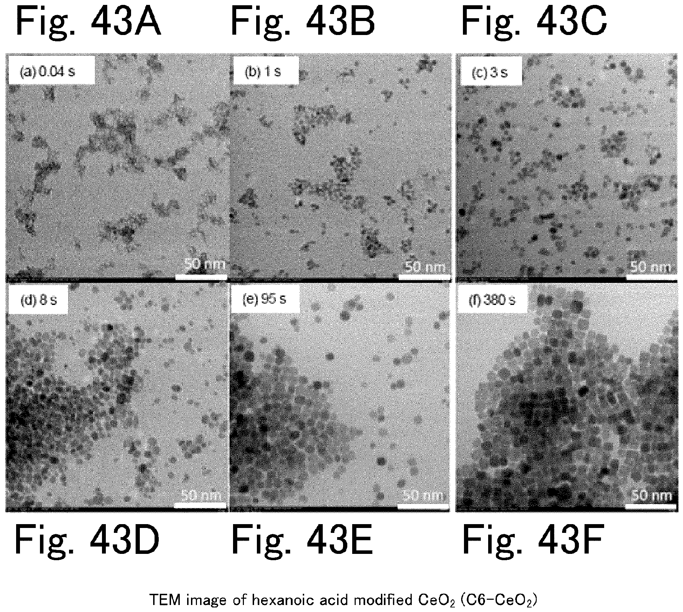

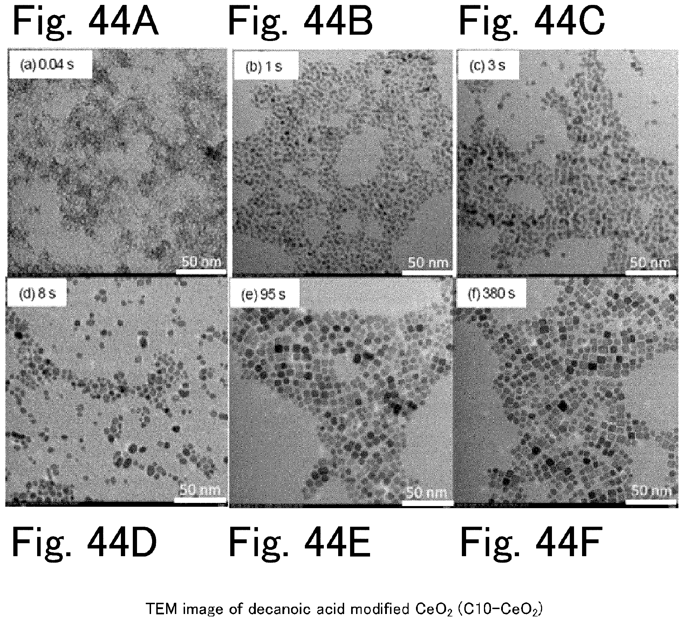

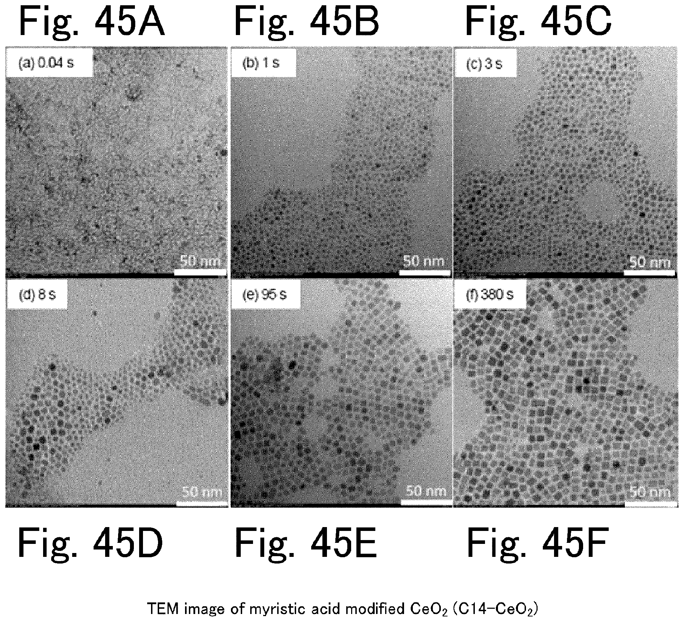

Electron microscope images of the obtained $CeO_2$ nanoparticles are shown in Fig. 43 (C6-$CeO_2$), Fig. 44 (C10-$CeO_2$), and Fig. 45 (C14-$CeO_2$). The nanoparticles 8 s after the start of synthesis were spherical or ellipsoidal in shape, while those after more than 95 s approached a cubic shape. In this experiment, shape-controlled nanoparticles with different modified molecular chain lengths could be synthesized at will using the distribution formula. We believe that by changing the concentration of these modifiers, it is possible to synthesize nanoparticles with freely controllable particle sizes.

Research on nanoparticle dispersion is necessary for nanofluids in which nanoparticles are dispersed at high concentrations. In the dispersion of organically modified nanoparticles, the modification density and modifier molecular chain length have a large impact. Therefore, it is necessary to continuously synthesize nanoparticles with varying modifier chain lengths and controlled sizes using a distribution method. This experiment clarified the particle formation process and provided guidelines for the synthesis of cubic $CeO_2$ nanoparticles with freely controllable modifier chain lengths and sizes in a distribution system.

<Test 7> Effect of precursor concentration

The formation-to-growth mechanism of $CeO_2$ was clarified by the distribution-type reaction. It was found that particle size changes due to nonclassical nucleation following homogeneous nucleation. Here, we attempted to control the particle size by controlling nonclassical nucleation through synthesis conditions other than reaction temperature and modifier. This allowed us to control the particle size from a smaller size without using a large amount of modifier, even for solvents other than benzene, which form homogeneous phases only at higher temperatures.

If nanoparticles are growing through the aggregation and coalescence of micronuclei, it is expected that the particle concentration in the reaction field will become thinner, the collision frequency will decrease, and particle growth will be suppressed. Therefore, we hypothesized that the $CeO_2$ particle size would decrease by decreasing the Ce concentration in the precursor. The effect of the precursor Ce concentration was examined in octanoic acid-modified C8-$CeO_2$.

[Example 7].

[Synthesis of cerium (IV) octanoate]

The precursor of cerium (IV) octanoate was obtained using the method described in Example 1.

[Supercritical hydrothermal synthesis]

The cerium concentrations of the cerium (IV) octanoate precursor were 0.0075 mol/L (Example 7-1), 0.0025 mol/L (Example 7-2), 0.0013 mol/L (Example 7-3), and 0.0005 mol/L (Example 7-4), and the reaction times were 0.04 s and 95 s.

[Recovery of products]

The product of cerium (IV) octanoate nanoparticles was recovered using the method described in Example 1.

[Evaluation]

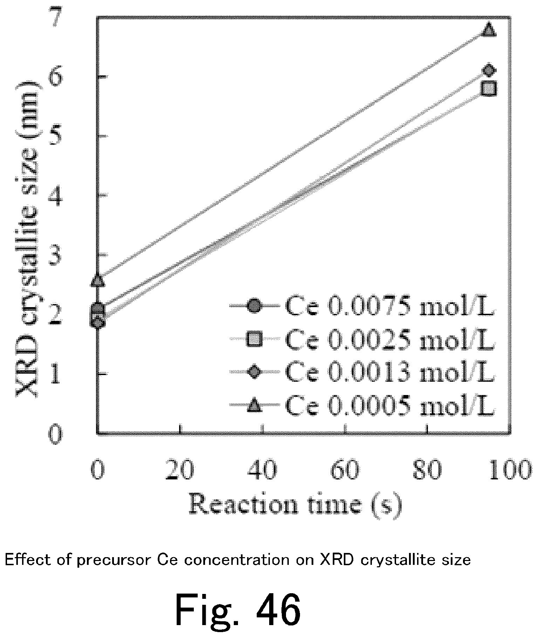

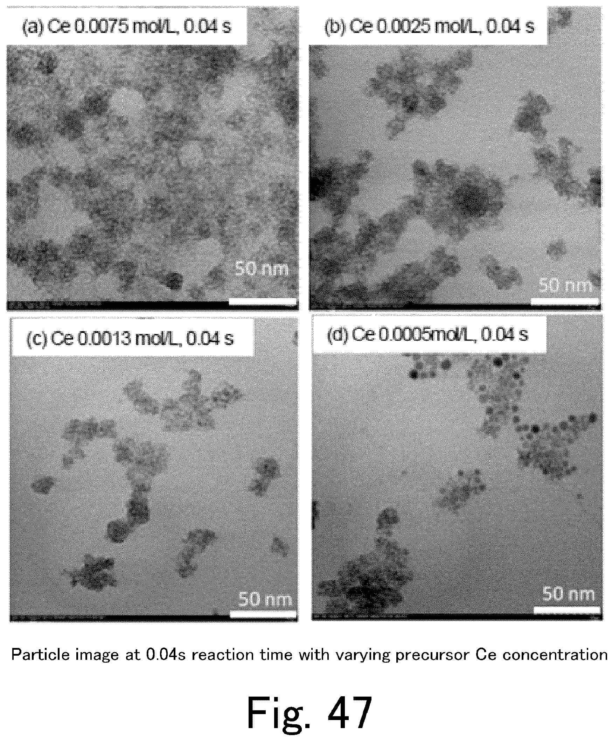

Fig. 46 shows the results of XRD analysis of the crystallite diameter of cerium (IV) octanoate nanoparticles, which did not decrease with decreasing Ce concentration but rather increased when the Ce concentration was reduced to 0.0005 mol/L. In this case, the crystallite diameter was large from the initial, 0.04 s, stage of formation. Fig. 47 shows a TEM image of nanoparticles at a reaction time of 0.04 s. When the Ce concentration was 0.0005 mol/L, coarse particles formed during the initial stage. We believe that large particles were formed in the early stages of formation, and that this was reflected in the final particle size.

We believe that the initial particle size increase was due to a change in the nucleation stage rather than the aggregation stage of particles when the concentration was lowered. According to homogeneous nucleation theory, the critical nucleation radius and nucleation rate decrease as the degree of supersaturation decreases. From Table 2, it can be considered that if the OA concentration is low, more than 99% of the precursor will react at a residence time of 0.04 s. Therefore, when the precursor concentration decreased from 0.0075 mol/L to 0.0005 mol/L, the degree of supersaturation became about 1/15. This is considered to cause an increase in the nucleus radius and a decrease in the nucleation rate, resulting in the formation of a large nucleus.

<Example 8> Effect of the Reynolds number

[0226] To control nonclassical nucleation, we focused on the Re number during synthesis because we considered it necessary to change the synthesis conditions that would not affect the homogeneous nucleation stage but would affect the aggregation frequency of micronuclei. When the theoretical aggregation frequency was calculated, the diffusion coefficient was computed assuming Brownian motion in a flowless field. In reality, the diffusion coefficient of particles increases with the distribution field. The diffusion coefficient of particles is considered larger when the flow is highly turbulent (i.e., when the Re number is high).

Denis et al. examined the effect of the Re number on particle size in the distribution-type synthesis of Co3O4 (Non-patent Document 21). They reported that a high Re number and turbulent flow resulted in a higher coalescence rate between particles and an increase in particle size. The same may also occur in the nonclassical nucleation of organically modified $CeO_2$, and it was thought that the particle size could be controlled by the Re number.

[Example 8]

The reaction Re numbers for supercritical hydrothermal synthesis were 11,300 (Example 8-1), 7100 (Example 8-2), 3100 (Example 8-3), 1500 (Example 8-4), and 180. The cerium (IV) octanoate nanoparticles were obtained using the method described in Example 1-6, except that the number of particles was 11,300 (Example 8-1), 7100 (Example 8-2), 3100 (Example 8-3), 1500 (Example 8-4), and 180.5 (Example 8-5).

[Evaluation]

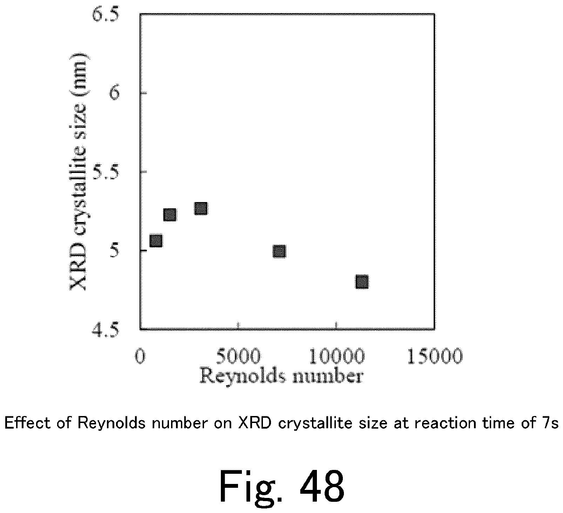

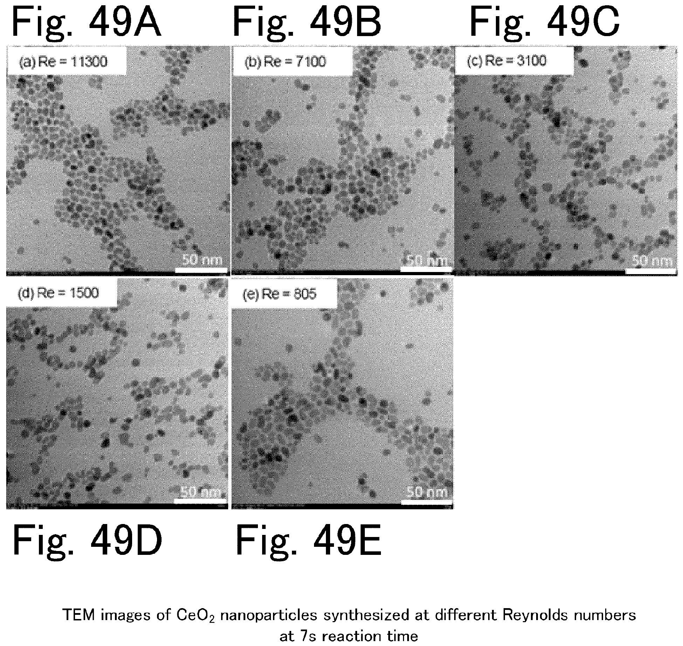

Fig. 48 summarizes the results of the XRD evaluation of the crystallite diameter at a reaction time of 6.8 s when the Re number during the reaction was varied. TEM images of the synthesized particles are shown in Fig. 49. The crystallite diameter did not decrease when the Re number decreased. In contrast, the crystallite diameter decreased slightly when the tube diameter was narrower and the Re number was higher. The flow in the reaction field becomes laminar as the tube diameter becomes larger, and the Re number decreases. It was thought that this would decrease the diffusion coefficient of the particles, thereby reducing the collision frequency and decreasing the particle size. The actual results were contrary to expectations. By increasing the Re number, the diffusion coefficient of the particles increases, and the number of simple particle collisions increases. However, for complete collisional coalescence to occur when particles are in close proximity to each other, the energy barrier to coalescence must be exceeded. Changing the Re number within the range of this experiment did not change the frequency with which the energy barrier was exceeded. Rather, we believe that the particle diameter decreased because the Re number increased; adhesion to the wall surface, flow velocity gradient, and retention at the pipe diameter change were eliminated; and uniform distribution was achieved.

Through Tests 1 through 8, the mechanism of the formation of organically modified $CeO_2$ by the supercritical hydrothermal method was clarified. In the formation of homogeneous nanoparticles, it was clarified that after homogeneous nucleation, a nonclassical nucleation stage was followed by the aggregation and coalescence of micronuclei. The particle size increase rate during the nonclassical nucleation stage varied depending on the reaction temperature and modifier. On the other hand, the effect on the nonclassical nucleation stage of the precursor concentration and Re number, which varied assuming aggregation growth, was not clear. For the synthesis conditions, such as the precursor concentration and Re number, which have a significant effect on particle diffusion and contact frequency, the effect on particle size was small. On the other hand, synthesis conditions such as reaction temperature and modifier, which can significantly affect the probability of coalescence of particles in contact, had a large effect on particle size.

<Test 9> Crystal structure change with the growth of nanoparticles

In the course of clarifying the formation process of organically modified nanoparticles, we succeeded in synthesizing $CeO_2$ nanoparticles in a controlled manner from a minimum of 1.5 nm by changing only the reaction time. This method made it possible to obtain particles of any size without the need to remove coarse particles. In the synthesis of nanoparticles by pyrolysis, the growth of nanoparticles during the process can be confirmed only by taking a small amount of nanoparticles during the process (Non-patent Documents 22 and 23). In contrast, by crystallizing nanoparticles using hydrothermal treatment in the order of seconds and cooling them rapidly, we were able to synthesize particles with a high yield that preserved the structure of the initial stage of formation. We now analyze the changes in physical properties associated with growth and clarify the relationship with the growth mechanism of nanoparticles.

[Example 9-1] Reaction temperature: 340°C, octanoic acid concentration: 0.12 mol/L, OA/Ce ratio: 16

[Synthesis of cerium (IV) octanoate]

The product of cerium octanoate (IV) was recovered using the method described in Example 1.

[Supercritical hydrothermal synthesis]

In addition to cerium (IV) octanoate and water, octanoic acid was added as the composition of the feed solution supplied to the distribution-type hydrothermal apparatus. The ratio (molar ratio) of octanoic acid to cerium (IV) was 16 (octanoic acid concentration of 0.12 mol/L). The reaction temperature was set at 340°C, and the specified times were 0.04, 0.1, 0.2, 1, 3, 8, and 95 s. Otherwise, supercritical hydrothermal synthesis was performed using the method described in Example 1.

[Recovery of products]

Cerium (IV) oxide nanoparticles were recovered using the method described in Example 1.

[Example 9-2] Reaction temperature: 300°C, octanoic acid concentration: 0.12 mol/L, OA/Ce ratio: 16

Cerium (IV) oxide nanoparticles were recovered using the method described in Example 9-1, except that the reaction temperature in the supercritical hydrothermal synthesis was 300°C.

[Example 9-3] Reaction temperature: 340°C, octanoic acid concentration: 0.52 mol/L, OA/Ce ratio: 64

The ratio (molar ratio) of octanoic acid to cerium (IV) in the supercritical hydrothermal synthesis was set to 64 (octanoic acid concentration 0.52 mol/L), and the specified times were 0.04, 0.1, 0.2, 1.1, 3.3, 9, and 106 s. The cerium (IV) oxide nanoparticles were recovered using the method described in Example 9-1, except that the specified time was set to 0.04 s.

[Evaluation]

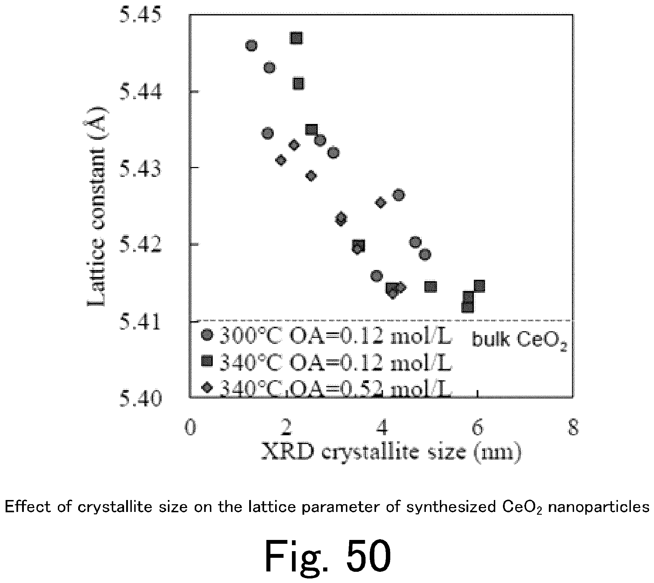

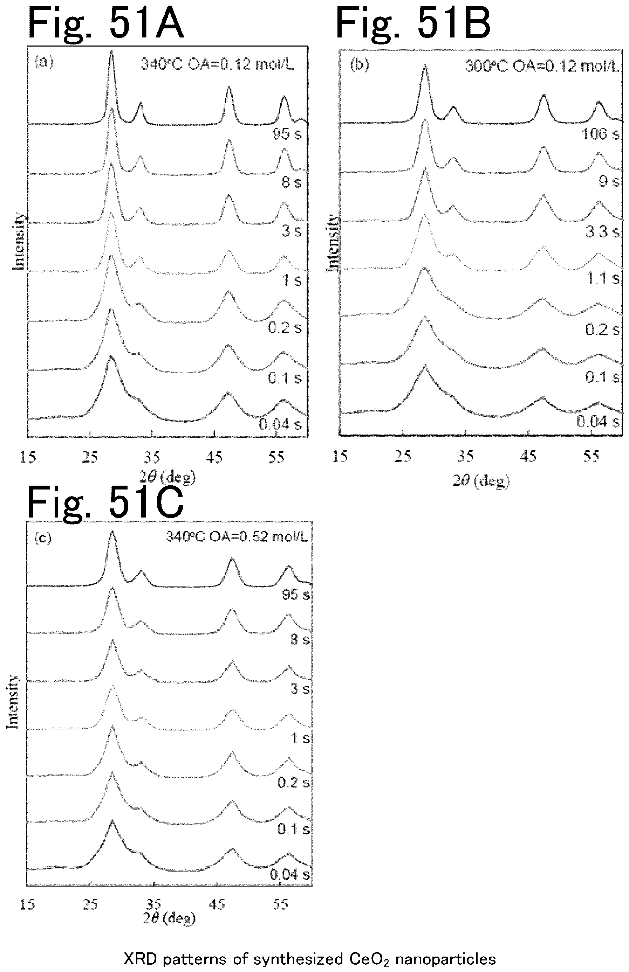

Fig. 50 shows the relationship between the lattice constant and particle size. The obtained XRD pattern is shown in Fig. 51. Fig. 51A shows the XRD patterns in Example 9-1 (340°C, OA = 0.12 mol/L), Fig. 51B shows those in Example 9-2 (300°C, OA= 0.12 mol/L), and Fig. 51C shows those in Example 9-3 (340°C, OA= 0.5 2 mol/L). The lattice constant was higher for smaller crystallite diameters. In particular, it increased sharply in the region where the particle size was less than 4 nm, suggesting that during nucleation of $CeO_2$, the lattice expanded more than in the bulk due to the smaller size of $CeO_2$ (NP 24-27) and that this state was preserved. During the process of particle growth after the reaction time, the lattice constant became smaller and approached the bulk lattice constant (5.410 Å). The relationship between the lattice constant and particle size was not significantly different under different synthesis conditions.

There was an increase in the lattice constant with decreasing crystallite diameter. Lattice expansion in $CeO_2$ is related to an increase in oxygen vacancies with decreasing particle size. Therefore, the change in cerium valence was analyzed using XPS spectra.

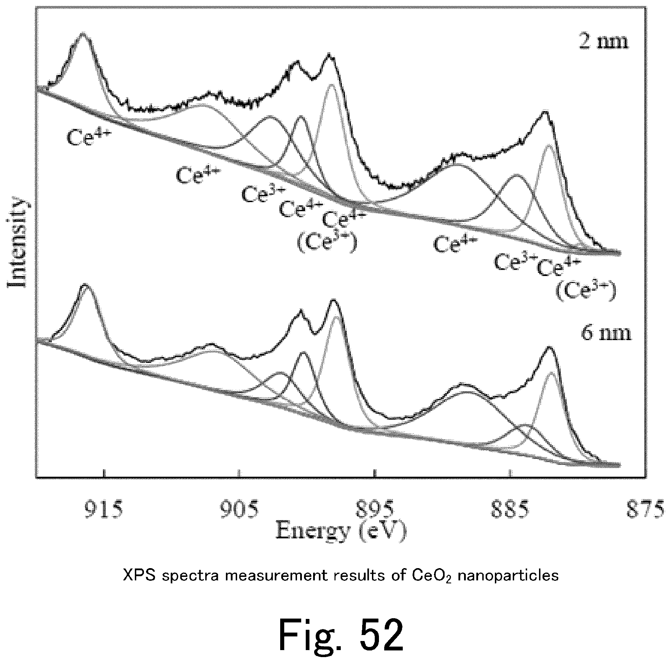

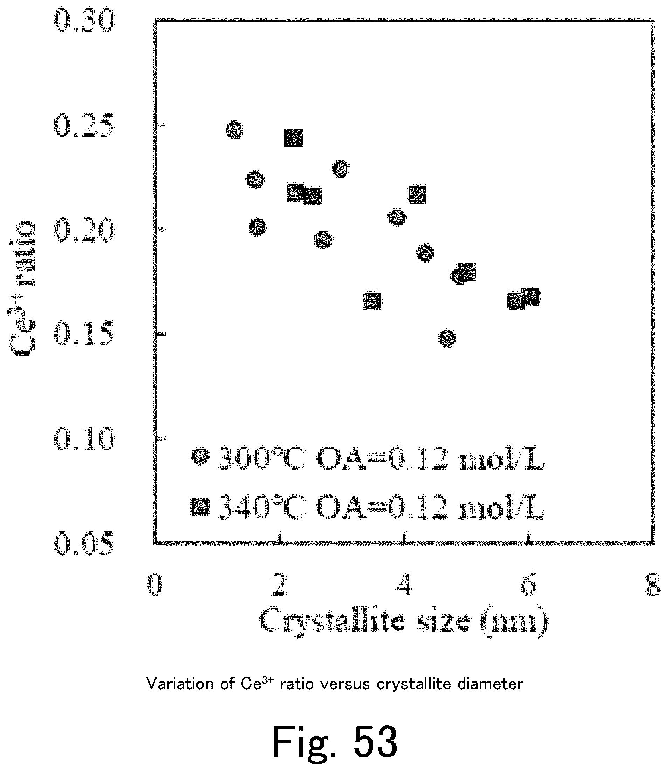

Representative XPS spectra are shown in Fig. 52, where 916.4, 907.1, 900.7, 898.1, 888.7, and 882.4 eV in the Ce3d region peaks are attributed to Ce4+ (Non-patent Document 28). The peaks at 902.5, 899.1, 884.4, and 880.9 eV are from Ce3+, and the peaks at 899.1 and 880.9 eV have low intensity. The $Ce^{3+}$ ratio can be quantified by taking the ratio of $Ce^{3+}$-derived peak intensity to all Ce-derived peak areas. Therefore, the measurements were performed immediately after transfer to the sample chamber, with the Ce spectrum measurement performed first. Fig. 53 shows the relationship between the ratio of $Ce^{3+}$ determined from XPS and the XRD crystallite diameter, where the ratio of $Ce^{3+}$ increased as the crystallite diameter decreased, but the increase was only small, at less than 10%.

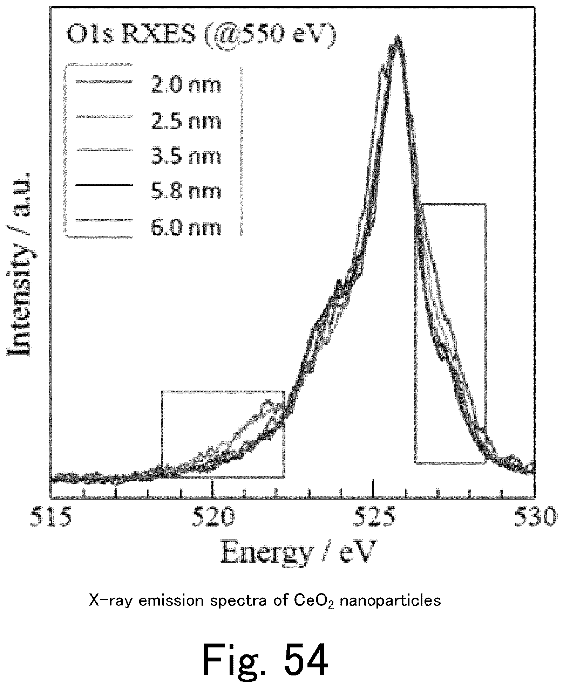

Fig. 54 shows the effect of the crystallite diameter on the X-ray emission spectrum in the O1s region. We observed the appearance of shoulder peaks at 521.5 eV and 527 eV for particles with a crystallite diameter of 3 nm or less. This suggests the presence of unstable O-Ce bonds in addition to stable O-Ce bonds in the early formation sample with a small particle size. Thus, there was a difference in the Ce-O bonding state between the early formation and postgrowth stages.

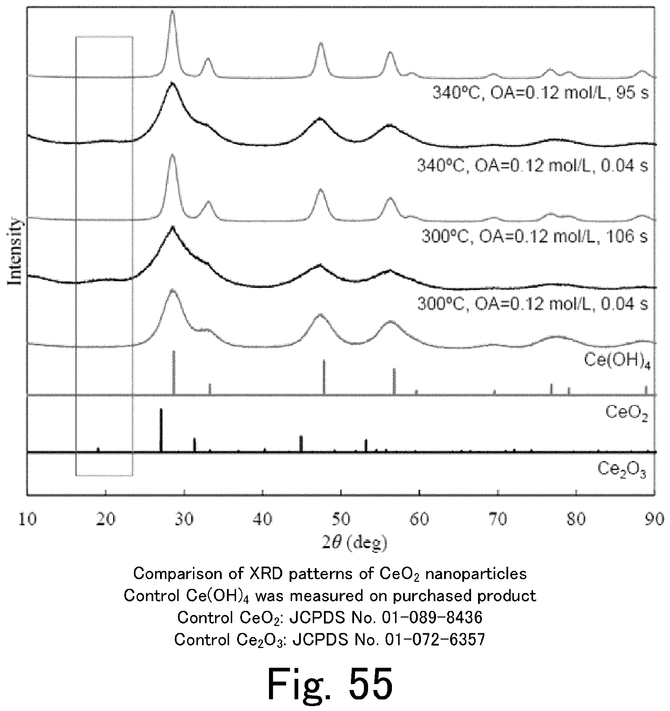

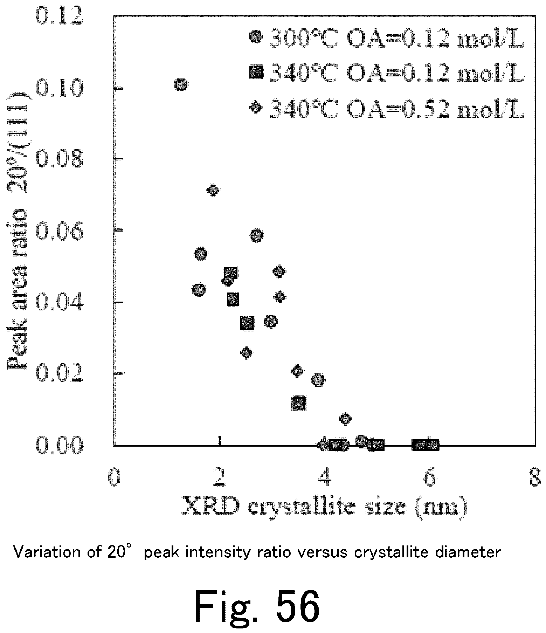

While the change in the ratio of $Ce^{3+}$ was slight in the early formation particles with a small crystal size, there was a change in the Ce-O bonding. We believe that this phenomenon indicates traces of nonclassical nucleation of $CeO_2$. Fig. 55 again shows the XRD patterns at the beginning of formation and after growth. The nanoparticles with a residence time of 0.04 s have a peak around 20° that is not present in bulk $CeO_2$. Even $Ce(OH)_4$, which can exist as an intermediate in the transformation from the complex to $CeO_2$, does not have a peak at 20°. This peak has not thus far been reported for $CeO_2$ nanoparticles synthesized. Fig. 56 shows the ratio of the peak area at 20° to the peak area at 28.3° due to the (111) plane of $CeO_2$ versus the crystallite diameter. The 20° peak intensity increases as the crystallite diameter decreases; in particular, the intensity increases rapidly below 4 nm.

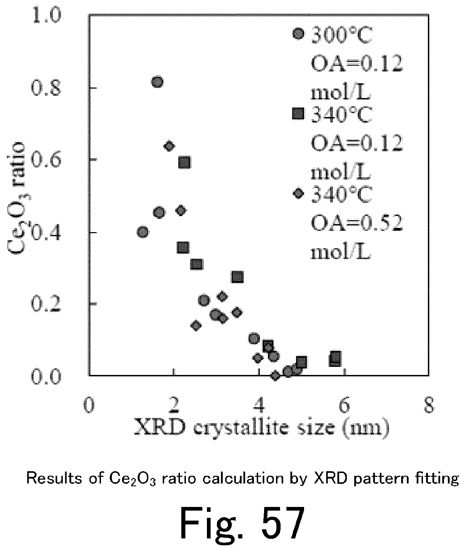

The origin of this characteristic peak is now discussed. As shown in the reference peak in Fig. 55, $CeO_2$ does not show a peak at 20°, while $Ce_2O_3$ shows a peak at around 20°. This suggests that the $CeO_2$ nanoparticles may have a distorted structure as $Ce_2O_3$ in the early stage of formation. However, XPS shows that the ratio of $Ce^{3+}$ increases only slightly, even when the crystallite diameter becomes less than 4 nm. The ratio change of $Ce_2O_3$ determined by XRD peak analysis was compared with the ratio change of $Ce^{3+}$ determined by XPS. The reference pattern for $CeO_2$ was JCPDS No. 01-089-8436, and that for $Ce_2O_3$ was JCPDS No. 01-072-6357. The relationship between $Ce_2O_3$ ratio and crystallite size determined by XRD pattern analysis is shown in Fig. 57. The $Ce_2O_3$ ratio increases sharply when the crystallite size is below 4 nm and exceeds 50% when it is below 2 nm, which is not consistent with the XPS results showing a $Ce^{3+}$ increase ratio of less than 10%. From this result, we considered that the 20° peak observed in the XRD was not caused by $Ce_2O_3$.

We believe that the 20° peak in XRD is not due to the $Ce_2O_3$ structure but to stacking faults. Stacking faults are a type of lattice plane defect, which is a defect stacked on atomic planes in a different order than it should be. It has been reported that stacking faults can exist in $CeO_2$ (Non-patent Documents 30 and 31). When stacking faults exist, a peak at a lower angle near the main peak has been reported in SiC and $BaTiO_3$ (Non-patent Documents 32 and 33). We believe that a similar phenomenon occurred in $CeO_2$. The stacking defects were probably generated during the initial stage of particle growth and during agglomeration and coalescence growth, and it then remained. The stacking defects were then eliminated by the oriented attachment of crystals to each other to eliminate the defects. In other words, the XRD peaks obtained in this study show traces of nonclassical nucleation in the early stages of nanoparticle formation.

<Test 10> Nanoparticles other than cerium octanoate ($ZrO_2$, $Ce_xZr_{1-x}O_2$)

[Preparation of raw material solution]

[Zr (IV) octanoate benzene solution]

Zr octanoate and octanoic acid (OA) were added to benzene to prepare a 0.020 M benzene solution of Zr octanoate. Two solutions with different Zr-atom-to-octanoic-acid ratios were prepared (OA/Zr ratio: 16, 40).

[Octanoic acid Ce (IV)]

First, 0.10 M cerium diammonium nitrate and 0.30 M sodium octanoate solutions were prepared. These were mixed and centrifuged at 4700 rpm for 20 min. Acetone was added to the precipitate, and washing and centrifugation were repeated twice at 4700 rpm for 20 min. The mixture was then dried at 70°C for 24 h.

[Ce octanoate and Zr octanoate benzene solution]

The prepared Ce octanoate, Zr octanoate, and octanoic acid were added to benzene to prepare a $Ce_xZr_{1-x}$ (x = 0.1, 0.9) solution with a metal concentration of 0.20 M (OA/(Ce+Zr) = 16).

[Hydrothermal synthesis using a distribution-type apparatus]

[Synthesis of $ZrO_2$, $Ce_xZr_{1-x}O_2$ (residence time: 0.029-12 s)

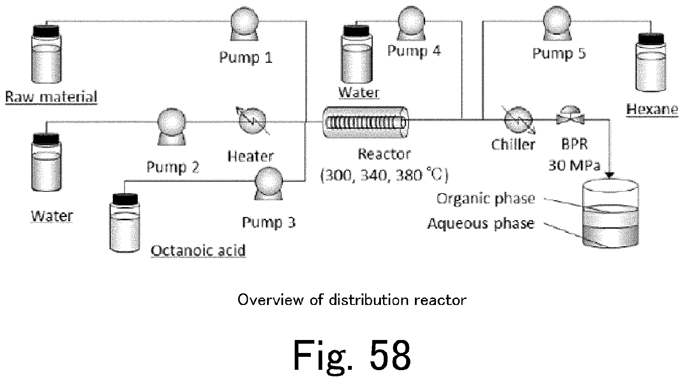

Fig. 58 shows an overview of the apparatus used for the synthesis. The raw material solution was pumped using multi-channel high-pressure feed pump 1, and the preheated water was pumped using high-pressure feed pump 2 at a predetermined rate. By manipulating the size and flow rate of the reaction tube, the residence time of the mixed solution was set to between 0.029 and 12 s. The reaction tube was wrapped with a ribbon heater and heated to 300, 340, and 380°C for $ZrO_2$ synthesis and 340°C for $Ce_xZr_{1-x}O_2$ synthesis in the mixing and inlet sections. In the synthesis, where the residence time was 0.20 s or less, pure water was pumped using high-pressure feed pump 4 to the outlet section to rapidly cool the fluid after the reaction. The particle dispersion fluid was collected after synthesis using a double-tube heat exchanger, with cooling water circulated using a small cooling water circulator in the outer tube.

[Particle washing and freeze-drying]

The generated particles existed at the interface between the organic and aqueous phases of the particle dispersion solution. Therefore, the organic and aqueous phases were separated using a multi-tubing pump, and the particles were dispersed in the organic phase. An equal volume of ethanol was added to the particle dispersion and centrifuged at 12,000 rpm for 60 min. A total of 30 mL of cyclohexane and 300 mL of acetone were added to the precipitate, and centrifugation was repeated twice at 4700 rpm for 60 min. After centrifugation, the precipitate was dispersed in cyclohexane and lyophilized to obtain powder particles.

[Analysis equipment]

The equipment used for the analysis of the synthesized particles is described below.

- Powder X-ray diffraction analyzer (XRD) (RIGAKU, SmartLab, Cu tube sphere, X-ray source CuK$\alpha$ 0.154056 nm) to identify synthetic particles, crystallite size, lattice distortion, lattice volume, etc.
- Transmission electron microscope (TEM) (HITACHI, H-7650, 100 kV, 20 $\mu$A) to observe the shape and particle size of the synthesized particles.
- Plasma emission spectrometer (ICP) (SPECTRO, ARCOS) to measure the concentration of metal ions in the solution and calculate the reaction rate.
- Multifunctional scanning X-ray photoelectron spectrometer (XPS) (ULVAC-PHI, PHI-5000VersaProbeII) to evaluate the valence of metal ions.
- Fourier transform infrared spectrophotometer (FT-IR) (Japan Spectroscopic Corporation, FT/IR-4200) to analyze the bonding state of the particle surfaces. Samples for analysis were prepared using the KBr method.
- Thermogravimetric analyzer (TGA) (Shimadzu Corporation, DTG-60AH) to determine the surface modification rate of the octanoic acid from the thermogravimetric reduction rate. N2 was distributed at 50 mL/min as a carrier gas, and the following temperature program was used for the measurement:

    1. Room temperature $\rightarrow$ 60°C (10°C/min)
    2. 60°C for 30 min (for removal of adhered water)
    3. 60°C $\rightarrow$ 150°C (10°C/min)
    4. 150°C for 1 h (for removal of bound water)
    5. 150°C $\rightarrow$ 900°C (10°C/min)

[Results and discussion]

[Synthesis of octanoic acid-modified $ZrO_2$]

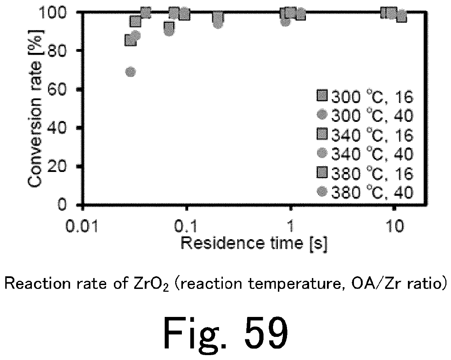

Fig. 59 shows the reaction rate in $ZrO_2$ synthesis obtained from plasma atomic emission spectrometry (ICP). Samples synthesized at 380°C or 340°C from raw materials with low octanoic acid concentrations showed a reaction rate of over 95% from the initial stage of the reaction. On the other hand, in samples where the reaction temperature was 300°C and samples were synthesized at 340°C from raw materials with high octanoic acid concentrations, unreacted raw materials remained.

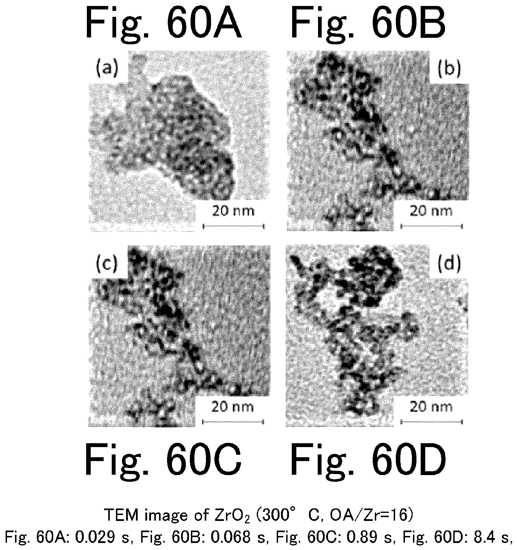

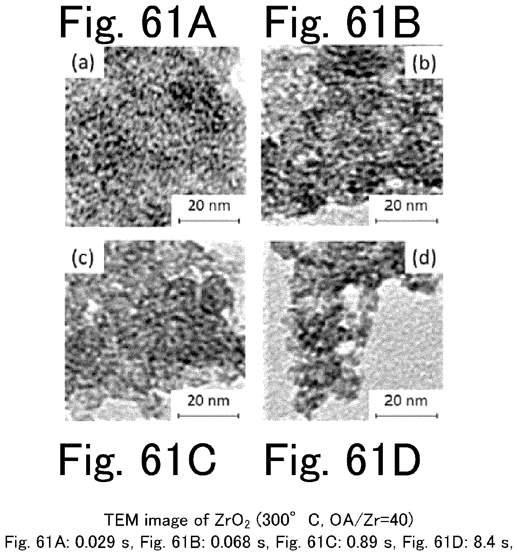

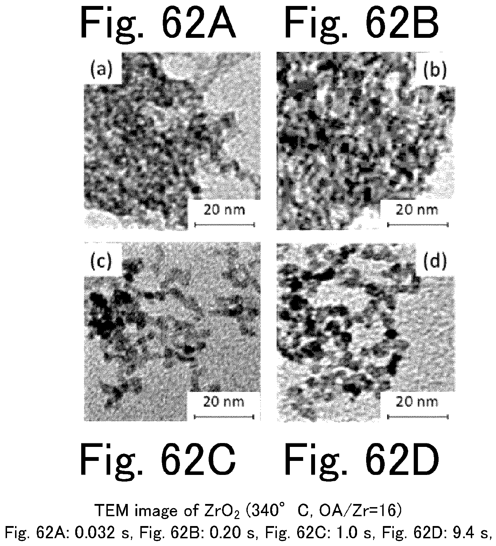

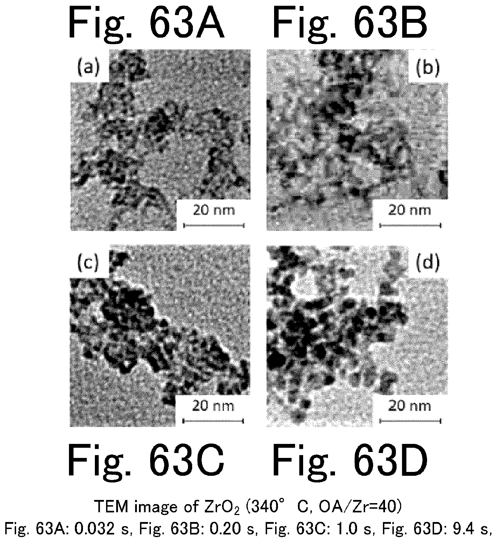

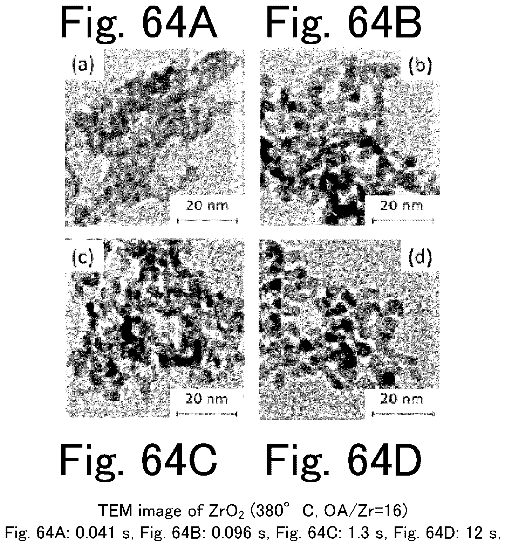

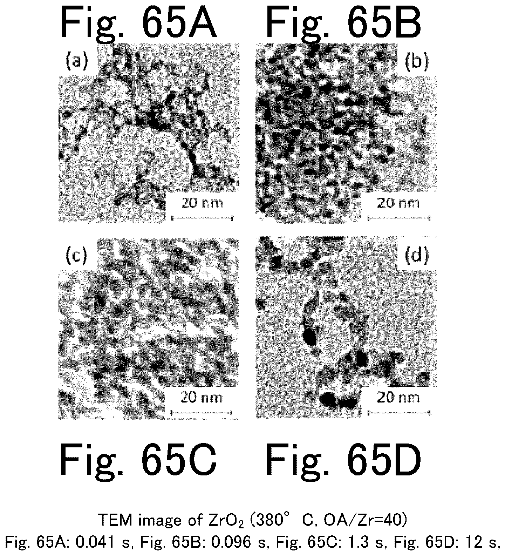

Figs. 60-65 show TEM images of the synthesized particles. The particles were agglomerated, there were fewer cubic particles than the $CeO_2$ obtained in Tests 1-9, and more elliptical and circular particles were observed.

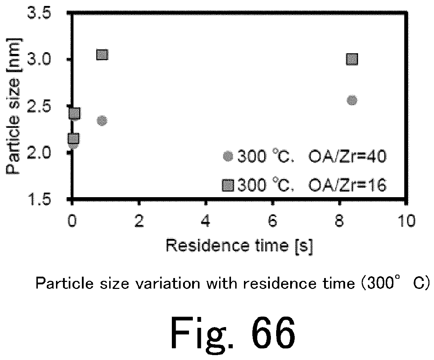

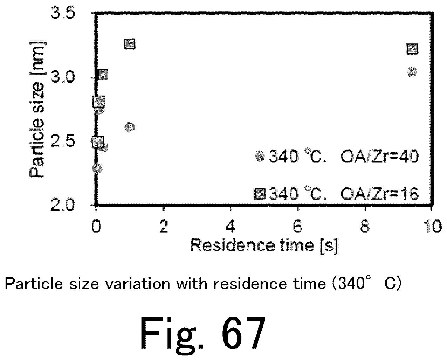

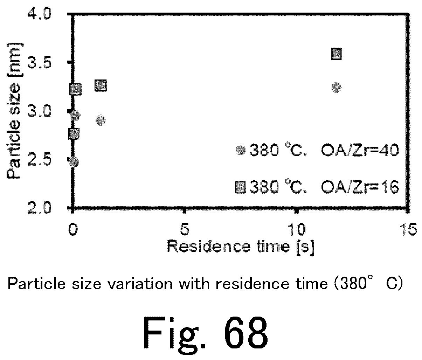

Figs. 66-68 show the change in particle size versus residence time. The particle size decreased with decreasing

# EP 4 484 378 A1

reaction temperature and increasing octanoic acid concentration in the raw material, even at the same residence time.

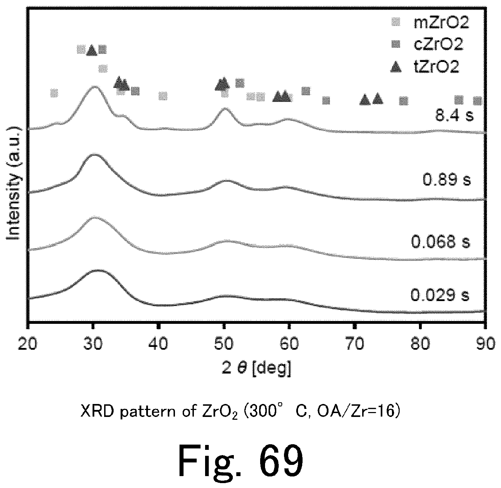

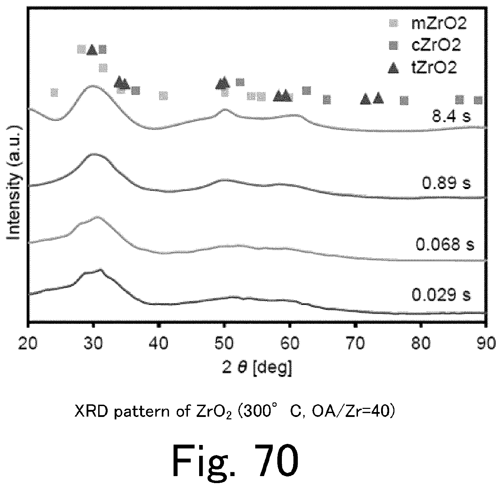

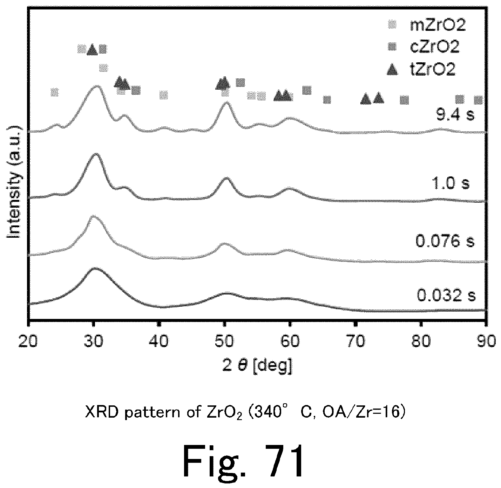

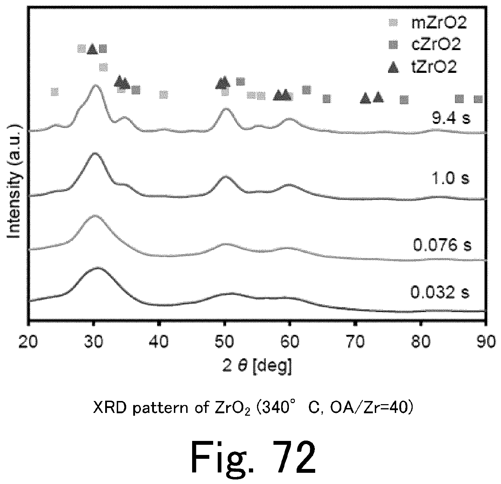

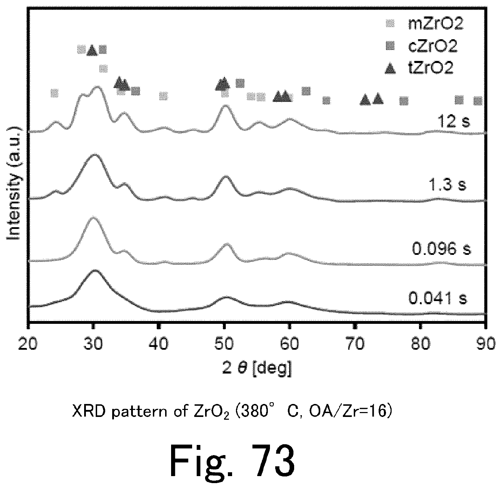

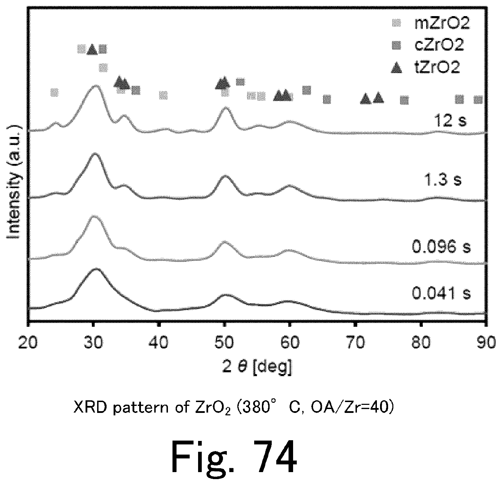

Figs. 69-74 show the XRD patterns of $ZrO_2$ under different experimental conditions. Similar to the $CeO_2$ obtained in test cases 1-9, the broadening of the peaks with the shortening of the residence time was confirmed. Peaks originating from the three crystalline phases of $ZrO_2$ (monoclinic [$mZrO_2$], cubic [$cZrO_2$], and tetragonal [$tZrO_2$]) were also observed. Rietveld analysis is a method for estimating the crystal structure from the measured XRD patterns. Rietveld analysis was performed for the combination of $mZrO_2$ and $tZrO_2$, which are stable phases at room temperature, and the three phases of $mZrO_2$, $tZrO_2$, and $cZrO_2$. The accuracy of the analysis is expressed, for example, by the $R_{wp}$ value and the S value. When each value is low, it can be said that the analysis was performed with high accuracy. Table 3 shows the $R_{wp}$ and S values for each condition and the percentage of crystalline phases analyzed in the three phases.

TABLE 3

| Temperature | Residence time | $R_{wp}$ | | S | | t/(m+t+c) | c/(m+t+c) |
|---|---|---|---|---|---|---|---|
| | | m and t | 3 phase | m and t | 3 phase | | |
| 300 °C | 0.029 s | 9.7 | 10.1 | 1.07 | 1.11 | 66% | 16% |
| | 8.4 s | 8.8 | 8.9 | 1.10 | 1.12 | 47% | 0.50% |
| 340 °C | 0.032 s | 10.1 | 9.6 | 1.11 | 1.07 | 56% | 25% |
| | 9.4 s | 8.3 | 7.9 | 1.12 | 1.06 | 31% | 6.0% |
| 380 °C | 0.041 s | 11.4 | 11.3 | 1.10 | 1.09 | 83% | 3.9% |
| | 12 s | 8.9 | 8.8 | 1.13 | 1.12 | 30% | 5.0% |
| $R_{wp}$ and S values in each condition, and the ratio of tZrO2 and cZrO2 when analyzed in three phases (OA/Zr = 16) | | | | | | | |

The accuracy of the analysis was approximately the same, although it was somewhat larger or smaller, depending on the conditions. The percentage of the $cZrO_2$ phase obtained by the analysis was smaller than that of the $tZrO_2$ phase in all cases, and in some samples, it was less than 1% of the total. From this, we believe that $mZrO_2$ and $tZrO_2$ accounted for the majority of the samples synthesized in this study, and we will discuss the results for the combination of these two phases in the future.

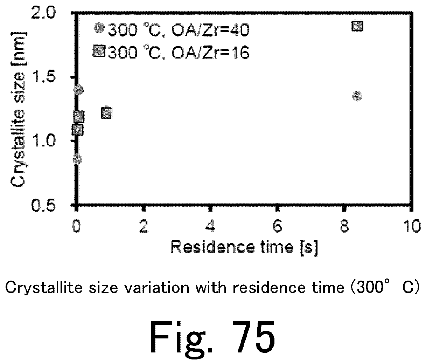

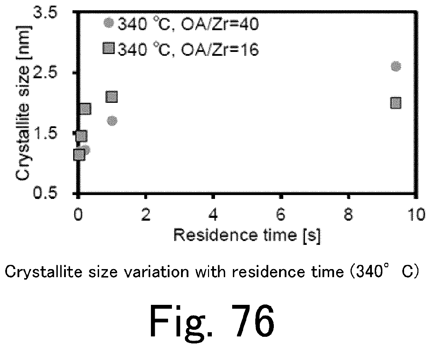

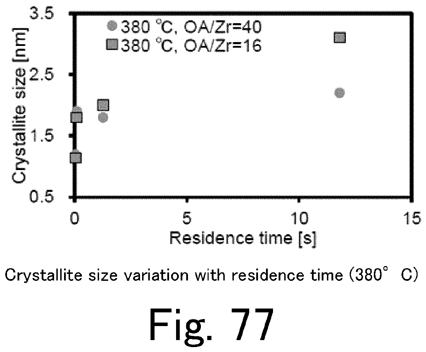

Figs. 75-77 show the crystallite size change of $ZrO_2$ versus the residence time. As the reaction temperature decreased and the octanoic acid concentration in the raw material increased, the crystallite size became smaller for the same residence time, showing the same trend as the change in grain size with respect to residence time. The cause of the size change depending on the synthesis condition is thought to be the suppression of the Brownian motion of the particles due to the decrease in temperature and the increase in solvent viscosity due to the decrease in temperature and increase in modifier, which suppressed the movement and collision of the particles in the solvent.

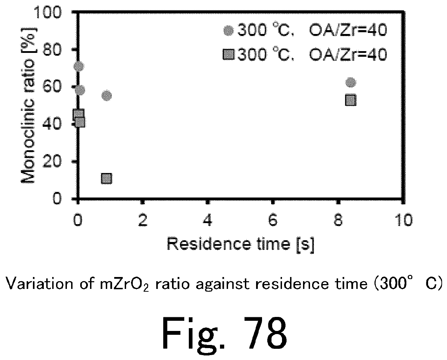

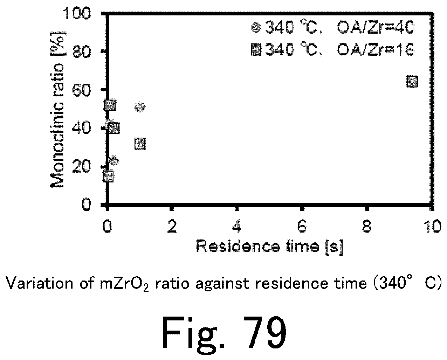

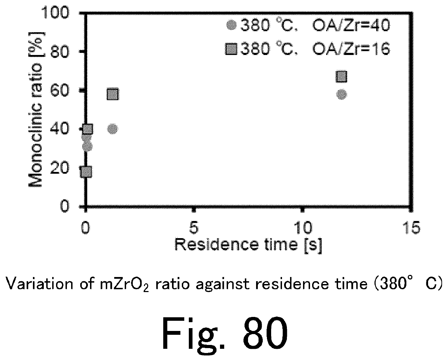

. Figs. 78-80 show the percentage of $mZrO_2$ phase in the synthesized particles versus the residence time. In the sample with a reaction temperature of 300°C, there was no significant correlation between the residence time and the percentage of $mZrO_2$, but in the samples with a reaction temperature of 340°C and 380°C, the percentage of $mZrO_2$ increased as the residence time was extended.

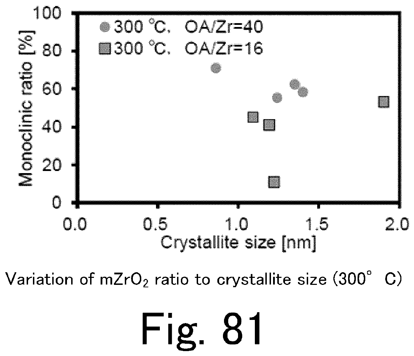

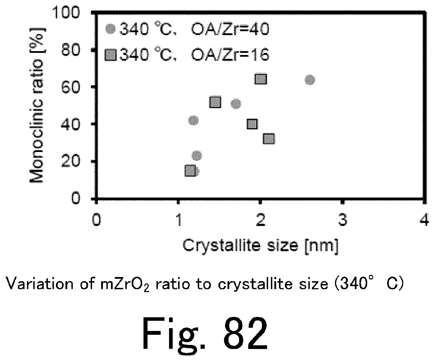

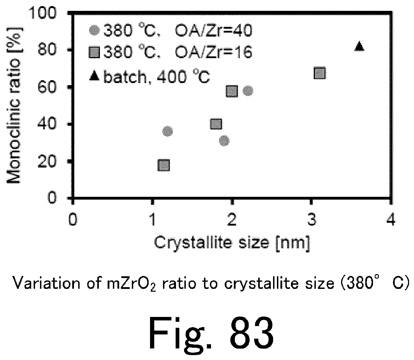

Figs. 81-83 show the percentage of $mZrO_2$ phase in the synthetic particles versus crystallite size. Fig. 83 also shows data for $ZrO_2$ synthesized from Zr nitrate at 400°C for 10 min using a batch tube reactor (Non-patent Document 34). The percentage of $mZrO_2$ increased with particle growth in the samples at reaction temperatures of 340°C and 380°C. This increasing trend is similar to previous reports on the phase transition from $tZrO_2$ to $mZrO_2$ with the change in the size of nanoparticles. However, in the newly synthesized $ZrO_2$, the crystalline phase transition occurred in the region of 5 nm or less, which is smaller than previously reported.

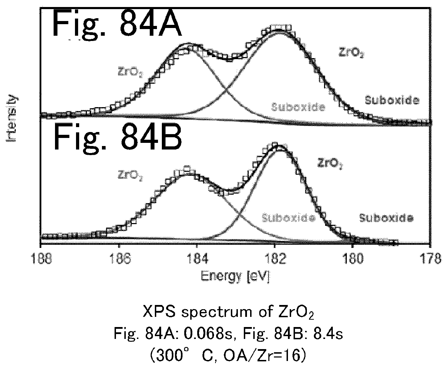

Fig. 84 shows the XPS spectrum of Zr3d. The XPS spectrum of $ZrO_2$ is derived from a suboxide composed of $ZrO_2$ and ions with low Zr valence (e.g., $Zr^{2+}$). In the particles synthesized in this study, no suboxide-derived peaks were observed, and unlike Ce, the Zr ions on the particle surface underwent almost no valence change.

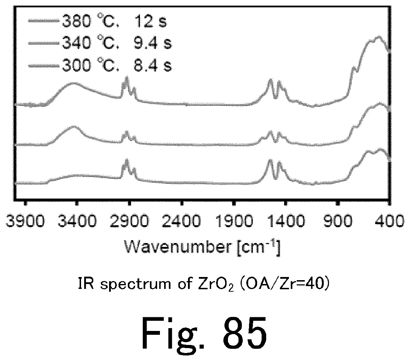

Fig. 85 shows the IR spectrum of $ZrO_2$. In Fig. 85, the peaks at 400-500 $cm^{-1}$ are Zr-O stretching vibrations, the peak at 1462 $cm^{-1}$ is an asymmetric vibration of the carboxylate bond, the peak at 1567 $cm^{-1}$ peak is a symmetric stretching vibration of the carboxylate bond, and the peak at 2700-3100 $cm^{-1}$ originates from the organically modified octanate molecular chain. Therefore, octanate chains were organically modified on the particle surface in hydrothermally synthesized $ZrO_2$.

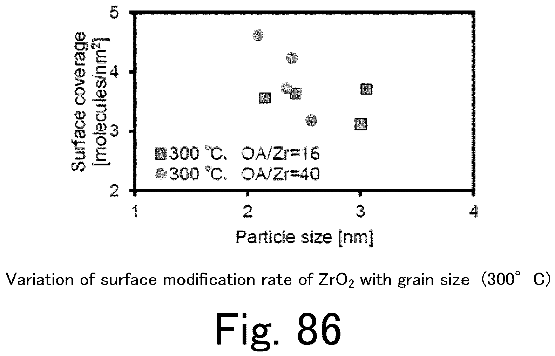

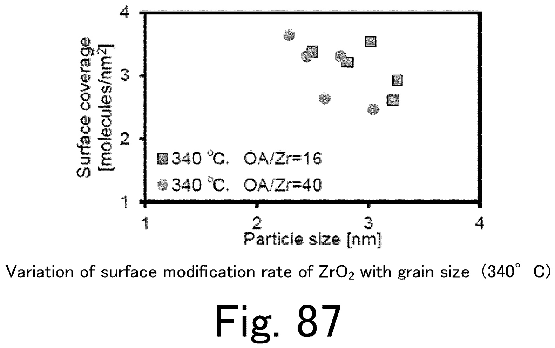

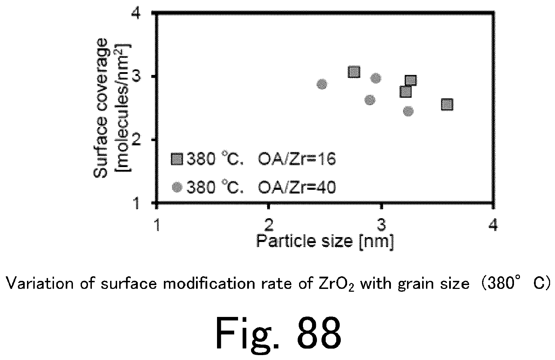

Figs. 86-88 show the change in the surface modification ratio of $ZrO_2$ with particle size. As with $CeO_2$, the surface modification ratio was large for small particles, and the value decreased with particle growth. The effect of the octanoic acid concentration in the raw material was not significant, while the modification ratio increased with decreasing reaction temperature.

31

[Synthesis of octanoic acid-modified $Ce_xZr_{1-x}O_2$]

The reaction rate of each particle in the synthesis of $Ce_{0.9}Zr_{0.1}O_2$ was shown to be more than 95% from the beginning, while that of $Ce_{0.1}Zr_{0.9}O_2$ with a residence time of 0.032 s was shown to be 83%, indicating that the precursor was not completely consumed in the early stage of the reaction.

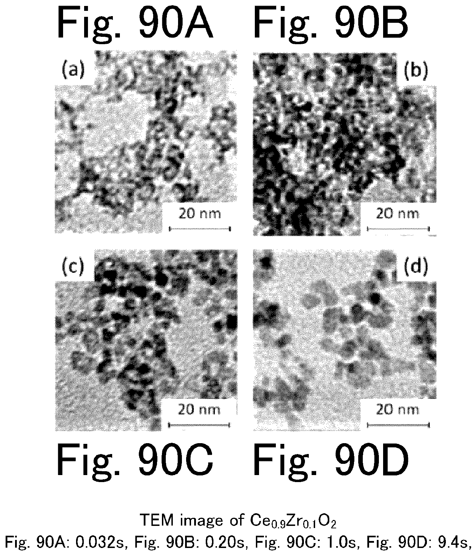

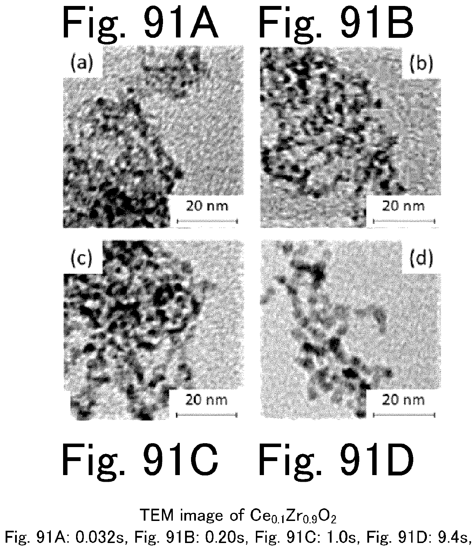

Figs. 90 and 91 show TEM images of the synthesized particles. Both $Ce_{0.9}Zr_{0.1}O_2$ and $Ce_{0.1}Zr_{0.9}O_2$ showed few cubic and many elliptical and spherical particles.

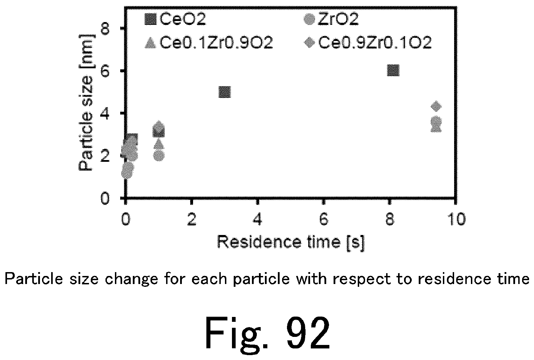

Fig. 92 shows the change in particle size of each particle with respect to residence time. $Ce_{0.1}Zr_{0.9}O_2$ showed the same growth trend as $ZrO_2$, and there was almost no difference in particle size at the same residence time, while the particle size of $Ce_{0.9}Zr_{0.1}O_2$ decreased more than that of $CeO_2$.

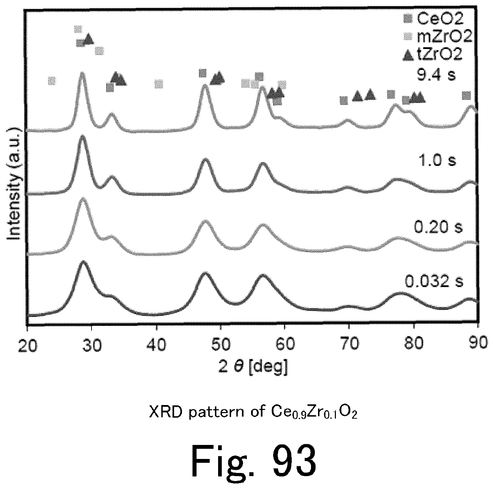

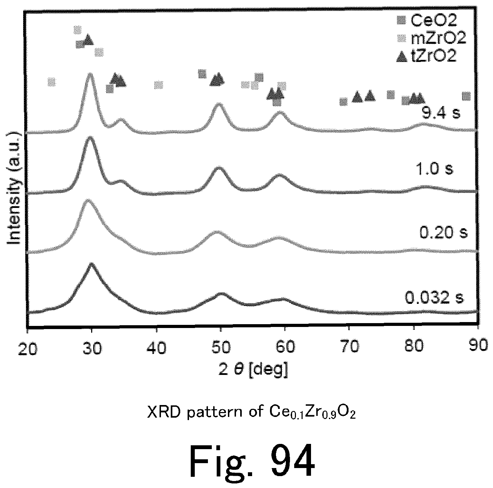

Figs. 93 and 94 show the XRD patterns of $Ce_{0.9}Zr_{0.1}O_2$ and $Ce_{0.1}Zr_{0.9}O_2$. In $Ce_{0.9}Zr_{0.1}O_2$, a peak derived from $CeO_2$ was observed. On the other hand, peaks originating from $mZrO_2$ and $tZrO_2$ were observed in $Ce_{0.1}Zr_{0.9}O_2$.

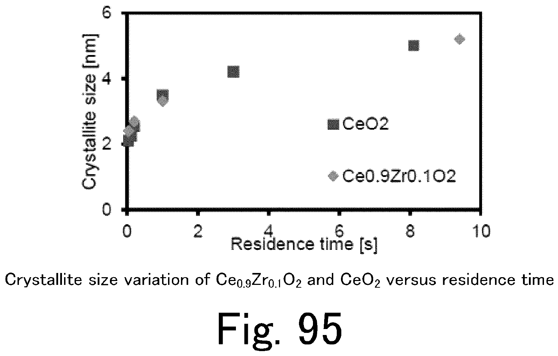

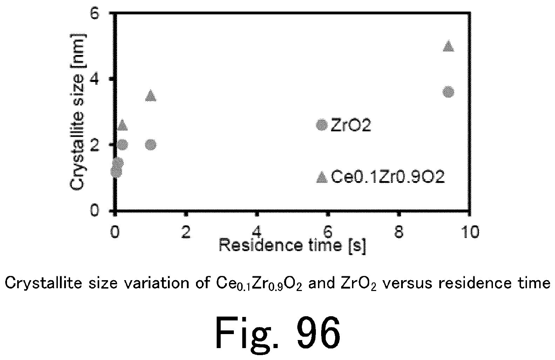

Figs. 95 and 96 show the crystallite size versus the residence time for each particle. As a comparison with pure materials, Fig. 95 shows data for $CeO_2$ synthesized at 340°C from Ce octanoate prepared previously in Tests 1-1 to 1-9, and Fig. 96 shows data for $ZrO_2$ synthesized at 340°C. As with the particle sizes obtained from TEM image observations, $Ce_{0.9}Zr_{0.1}O_2$ showed the same growth trend as $CeO_2$, and there was almost no difference in crystallite size at the same residence time. On the other hand, in $Ce_{0.1}Zr_{0.9}O_2$, the crystallite size increased more than in $ZrO_2$ due to Ce doping.

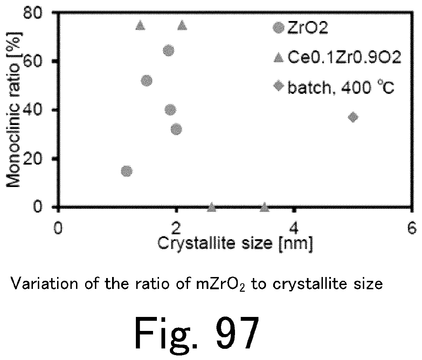

Fig. 97 shows the percentage of $mZrO_2$ within $Ce_{0.1}Zr_{0.9}O_2$. Data for $Ce_{0.1}Zr_{0.9}O_2$ synthesized from Ce and Zr nitrates at 400°C for 10 min using a batch tube reactor (Non-patent Document 34) are also shown. The $mZrO_2$ in $Ce_{0.1}Zr_{0.9}O_2$ did not increase with the crystallite size increase observed in $ZrO_2$. However, in samples with residence time less than 0.2 s, the XRD pattern broadened, partly due to the addition of Ce, and the actual $mZrO_2$ may be less than the measured value. In samples with a residence time of 1.0 s or longer, $tZrO_2$ peaks of sharp origin were observed. This suggests that even particles larger than $ZrO_2$ may be stabilized in the tZrO2 phase in $Ce_{0.1}Zr_{0.9}O_2$. However, this is not necessarily the case, since the percentage of $mZrO_2$ phase was 75% for samples smaller than 2.1 nm.

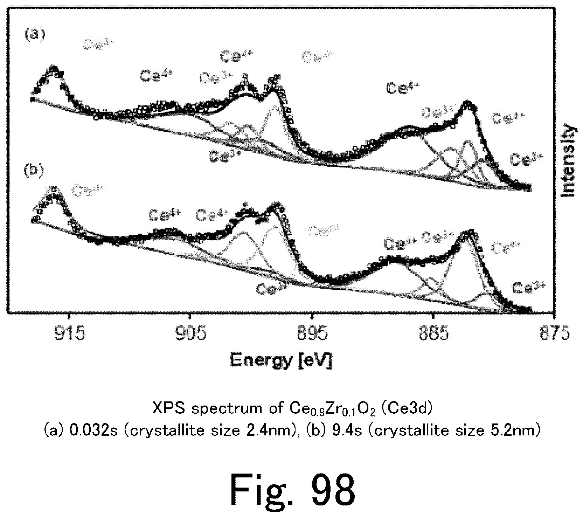

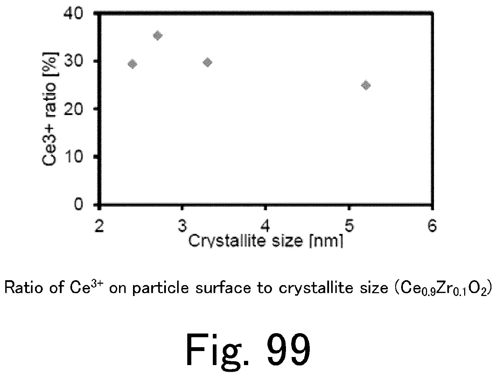

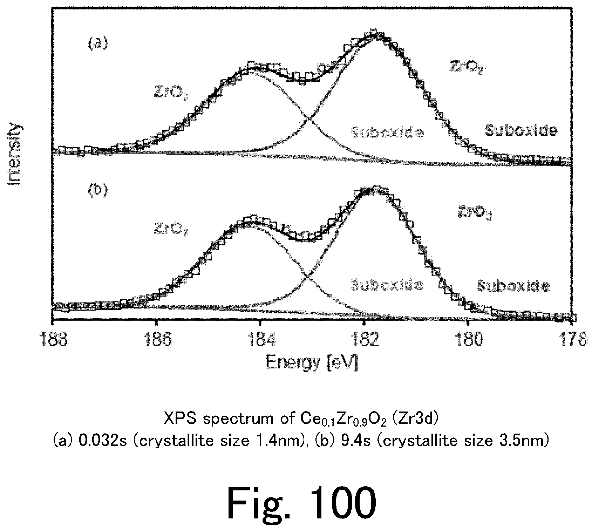

Fig. 98 shows the XPS spectrum of $Ce_{0.9}Zr_{0.1}O_2$ (Ce3d), Fig. 99 shows the percentage of $Ce^{3+}$ on the surface of $Ce_{0.9}Zr_{0.1}O_2$ particles, and Fig. 100 shows the XPS spectrum of $Ce_{0.1}Zr_{0.9}O_2$ (Zr3d). As with $CeO_2$, the residence time in $Ce_{0.9}Zr_{0.1}O_2$ was reduced (particle miniaturization) and the $Ce^{3+}$-derived peak increased. On the other hand, in $Ce_{0.1}Zr_{0.9}O_2$, no change in Zr valence occurred on the particle surface, as in $ZrO_2$.

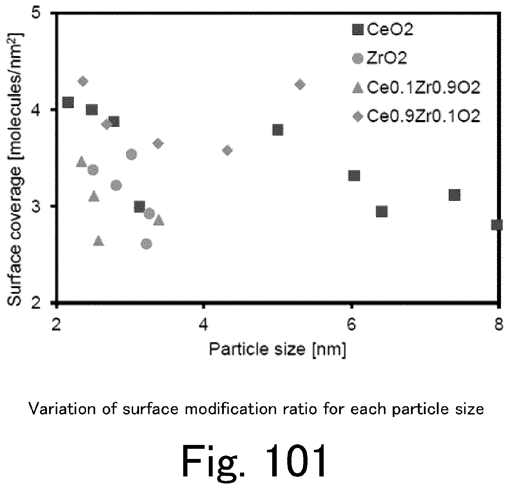

Fig. 101 shows the surface modification ratio of each particle with respect to the change in particle diameter. The surface modification ratio increased with decreasing particle size for both $Ce_{0.9}Zr_{0.1}O_2$ and $Ce_{0.1}Zr_{0.9}O_2$. The values for $Ce_{0.9}Zr_{0.1}O_2$ and $Ce_{0.1}Zr_{0.9}O_2$ were equal to or slightly lower than those for $CeO_2$ and ZrO2, respectively.

<Test 11> Investigation of the growth and crystallization process in the initial stage of nanoparticle synthesis reaction

In Tests 1-10, the effects of the process factors of the supercritical hydrothermal synthesis method on the size, crystalline state, and surface modification rate of the particles produced were confirmed. In this study, the reaction mechanism and lattice expansion of each particle are investigated and examined to understand the relationship between the growth and crystallization processes in the early reaction phase of particle synthesis.

[Reagents]

The following nanoparticles synthesized in Tests 1-10 were investigated. For $ZrO_2$ and $Ce_xZr_{1-x}O_2$, we also compared the data of particles synthesized from nitrate at 400°C (Non-Patent Document 34).

- Octanoic acid-modified $CeO_2$
- Octanoic acid-modified $ZrO_2$
- Octanoic acid-modified $Ce_xZr_{1-x}O_2$ (x=0.1, 0.9)

[Powder X-ray diffraction analyzer]

- Powder X-ray diffraction analyzer (XRD) (RIGAKU, SmartLab, Cu tube sphere, CuK$\alpha$ 0.154056 nm X-ray source)
- Plasma emission spectrometer (ICP) (SPECTRO, ARCOS)
- Multifunctional scanning X-ray photoelectron spectrometer (XPS) (ULVAC-PHI, PHI-5000 VersaProbeII)

[Results and discussion]

[Growth process of $CeO_2$ nanoparticle synthesis in the initial stage of the reaction.]

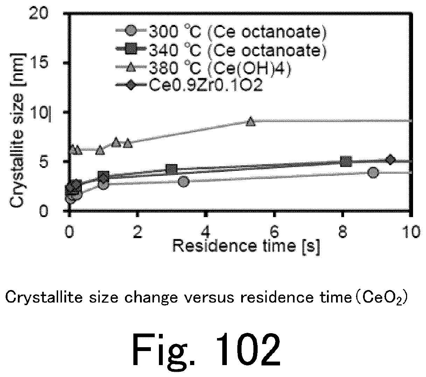

Fig. 102 shows the change in the crystallite size of $CeO_2$ in the initial stage of the reaction. The crystallite size increased in the initial stage of the reaction at all reaction temperatures and then changed to a gradual increase trend. As shown in Tests 1-9, reaction rates of more than 95% were obtained from the initial process. This suggests that all precursors were consumed at the time of homogeneous nucleation, and thereafter, the particles themselves became monomers of growth.

The growth mechanism in the initial stage of the reaction is discussed in terms of the frequency of collisions and coalescence. When the growth mechanism of particles encompasses collision and coalescence, the time variation of the number of particles N $[1/m^3]$ is expressed as follows:

$$\frac{dN}{dt} = -\frac{1}{2}\beta N^2$$

$$(14)$$

Here, $\beta$ $[m^3/s]$ is the collision/coalescence frequency factor, and by plotting dN/dt against $N^2$, the collision/coalescence frequency factor $\beta$ can be obtained for each reaction time (crystallite size).

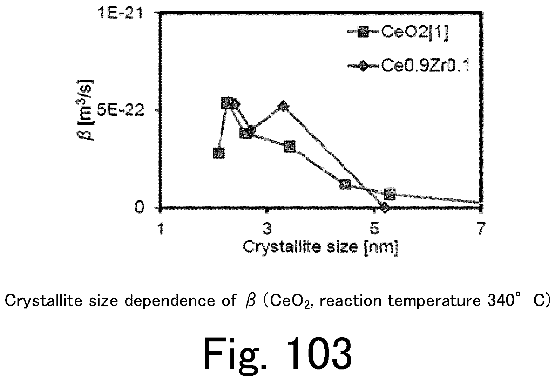

Fig. 103 shows the relationship between crystallite size and $\beta$ for each particle at a reaction temperature of 340°C. The value of $\beta$ was high in the initial stage of the reaction when the crystallite size was small, and the value decreased with particle growth. There was no clear difference in $\beta$ between $CeO_2$ and $Ce_{0.9}Zr_{0.1}O_2$.

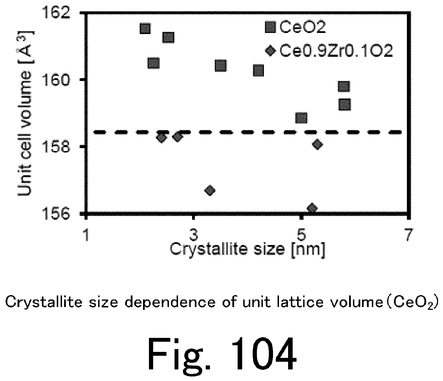

Here, we focus on the volume of the unit lattice as a change in crystallinity with particle growth. Fig. 104 shows the relationship between crystallite size and the unit lattice volume of $CeO_2$. The plot represents the measured unit lattice volume of the nanoparticles, and the dotted line represents the unit lattice volume in the bulk. In $CeO_2$, the unit lattice expands with nanosizing, and lattice expansion is suppressed with particle growth. In $Ce_{0.9}Zr_{0.1}O_2$, the unit lattice volume in the bulk is smaller than in $CeO_2$ due to $ZrO_2$. The volume of $Ce_{0.9}Zr_{0.1}O_2$ is lower than that of $CeO_2$ in the bulk, but the trend of volume decrease with particle growth is similar to that of $CeO_2$.

XPS measurements showed that the proportion of $Ce^{3+}$ on the particle surface was large for small particles with larger expansion, oxygen vacancies were generated, and the proportion of $Ce^{3+}$ decreased with particle growth. These results suggest that there may be a correlation between $\beta$ and changes in surface properties, lattice expansion, and valence change (oxygen vacancy formation) due to particle growth.

[Investigation of the growth process of $ZrO_2$ and $Ce_{0.1}Zr_{0.9}O_2$ nanoparticle synthesis in the initial stage of the reaction]

For $ZrO_2$, the same investigation as for $CeO_2$ was carried out without a valence change, and the mechanism of the decrease of $\beta$ and the appearance of crystallite-size-dependent physical properties are discussed here in comparison with $CeO_2$.

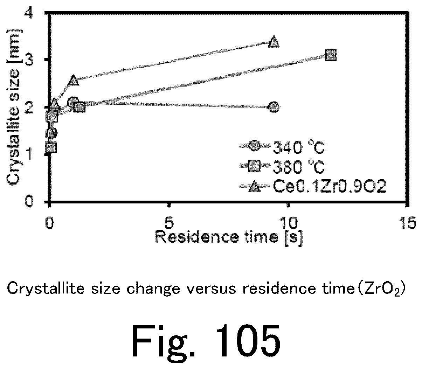

Fig. 105 shows the crystallite size change of $ZrO_2$ in the initial stage of the reaction. Similar to $CeO_2$, shown in Fig. 102, the crystallite size of $ZrO_2$ increased in the initial stage of the reaction and then changed to a gradual increase trend. In addition, in the synthesis of $ZrO_2$, a reaction rate of more than 95% was obtained from the initial process, suggesting that the growth process in the initial stage of the reaction was due to the collision and coalescence of particles.

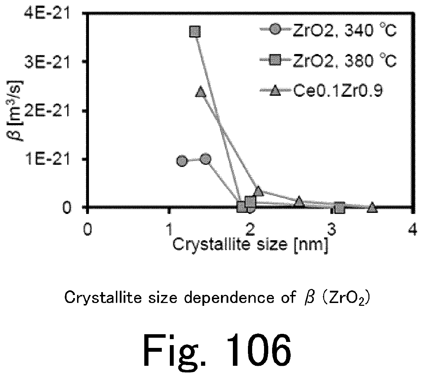

Fig. 106 shows the relationship between the crystallite size of each particle and $\beta$ calculated from Equation (14). $\beta$ decreases with increasing crystallite size in $ZrO_2$ and $Ce_{0.1}Zr_{0.9}O_2$, indicating that not only $CeO_2$ but also the hydrothermal synthesis of $ZrO_2$-based nanoparticles using a distribution-type reactor suppressed the collision and coalescence growth associated with particle growth. As shown in Test 10, $\beta$ depends on other factors, since no valence change was observed in Zr ions on the particle surface in $ZrO_2$ and $Ce_{0.1}Zr_{0.9}O_2$ from XPS measurements.

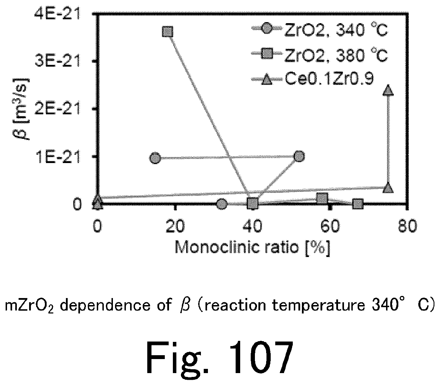

Next, we discuss the relationship between crystal structure and $\beta$. As described in Test 10, we observed a phase transition from monoclinic ($mZrO_2$) to tetragonal ($tZrO_2$) with particle growth in $ZrO_2$. Fig. 107 shows the relationship between the percentage of $mZrO_2$ and $\beta$ for each particle. No correlation was obtained between the percentage of $mZrO_2$ and $\beta$.

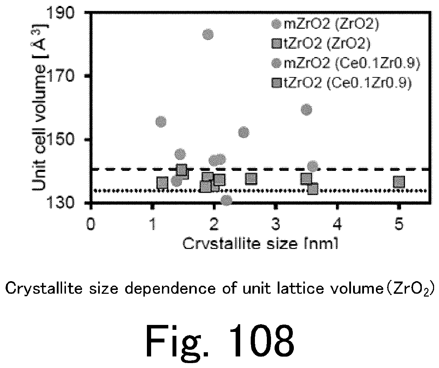

Lattice expansion is discussed next. Fig. 108 shows the relationship between crystallite size and the unit lattice volume of $ZrO_2$. The plot shows the measured unit lattice volume of nanoparticles; the dotted and dashed lines represent the unit lattice volume in the bulk of $tZrO_2$ and $mZrO_2$, respectively. In comparison, for $tZrO_2$, the measured and bulk values (66.97 Å3) of nanoparticles doubled.

In both $ZrO_2$ and $Ce_{0.1}Zr_{0.9}O_2$, the unit lattice of both phases expanded with nanosizing. However, the variation was large, and no clear change with respect to crystallite size was observed.

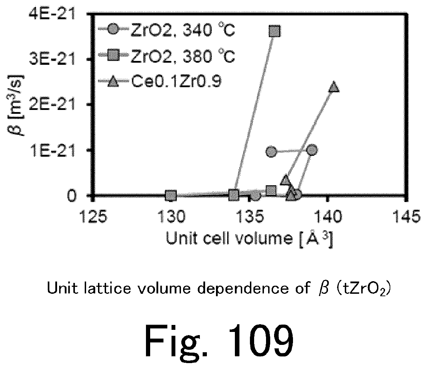

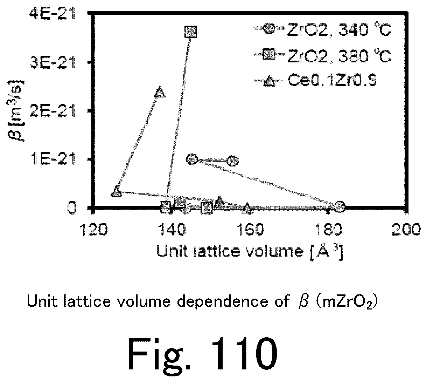

The relationship between unit lattice volume and $\beta$ is shown in Fig. 109 for $tZrO_2$ and Fig. 110 for the $mZrO_2$

phase. No significant correlation was observed between the unit lattice volume and β for each phase due to the effect of variation in unit lattice volume with respect to crystallite size.

These results suggest that the collision and coalescence frequency factors depend not only on the crystal structure and lattice expansion but also on the particle size and size-dependent surface properties.

[Summary]

In this study, the reaction mechanism and lattice expansion of each particle were investigated to understand the relationship between the growth process and the crystallization process in the early reaction phase of particle synthesis. The fact that precursors were consumed for homogeneous nucleation in the early stage of the reaction in each particle indicates that the growth mechanism thereafter is based on the collision and coalescence of particles. In addition, the unit lattice of each particle was expanded by nanosizing.

In the above, the so-called nonclassical nucleation process, which is the growth of particles by collisional coalescence and fusion immediately after homogeneous nucleation, can be achieved by maintaining a high reaction rate of 99% by precisely controlling the mixing rate and reaction time in a distribution-type supercritical hydrothermal synthesis reaction system, while achieving the continuous synthesis of ultrafine nanoparticles with a particle diameter of less than a single nanometer with a narrow particle size distribution. The system has demonstrated that ultrafine nanoparticles with a particle size distribution narrower than a single nanometer can be continuously synthesized while maintaining a high reaction rate of 99% through precise control of the mixing rate and the reaction time.

<Non-patent Documents>

Non-patent Document 1: A. Yoko, Y Tanaka, G. Seong, D. Hojo, T. Tomai, and T. Adschiri, Mixing and Solvent Effects on Kinetics of Supercritical Hydrothermal ynthesis: Reaction of Nickel Nitrate to Nickel Oxide, J. Phys. Chem. C, 2020, 124, 4772-4780.
Non-patent Document 2: N. Aoki, A. Sato, H. Sasaki, A. Litwinowicz, G. Seong, T. Aida, D. Hojo, S. Takami, and T. Adschiri, Kinetics study to identify reaction-controlled conditions forsupercritical hydrothermal nanoparticle synthesis with flow-typereactors, J. of Supercritical Fluids 2016, 110, 161-166.
Non-patent Document 3: M. Smoluchowski, Phys. Z., 1916, 17, 557-585.
Non-patent Document 4: M. Kobayashi, Y Adachi, Journal of the Agricultural Engineering Society, Japan, 1998, 66, 959-964.
Non-patent Document 5: S. Taniguchi, A. Kikuchi, Tetsu-to-Hagane., 1992, 78, 527-535.
Non-patent Document 6: T. Fujii, S. Kawasaki, A. Suzuki and T. Adschiri, HighSpeed Morphology Control of Boehmite Nanoparticles by Supercritical Hydrothermal Treatment with Carboxylic Acids, Cryst. Growth Des., 2016, 16, 1996. DOI: 10.1021/acs.cgd.5b01584.
Non-patent Document 7: Y. Takahashi, H. Shimizu, A. Usui, H. Kagi and M. Nomura, Direct observation of tetravalent cerium in ferromanganese nodules and crusts by X-ray-absorption near-edge structure (XANES), Geochim. Cosmochim. Acta, 2000, 64, 2929. DOI: 10.1016/S0016-7037(00)00403-8.
Non-patent Document 8: A. Bianconi, A. Marcelli, H. Dexpert, R. Kamatak, A. Kotani, T. Jo and J. Petiau, Specific intermediate-valence state of insulating 4f compounds detected by L3 x-ray absorption, Phys. Rev. B, 1987, 35, 806. DOI: 10.1103/PhysRevB.35.806.
Non-patent Document 9: M. Taguchi, N. Yamamoto, D. Hojo, S. Takami, T. Adschiri, T. Funazukuri and T. Naka, Synthesis of monocarboxylic acid-modified CeO2 nanoparticles using supercritical water, RSC Adv., 2014, 4, 49605. DOI: 10.1039/C4RA06936F.
Non-patent Document 10: M. K. Devaraju, S. Yin and T. Sato, Morphology Control of Cerium Oxide Particles Synthesized via a Supercritical Solvothermal Method, ACS Appl. Mater. Interfaces, 2009, 1, 2694. DOI: 10.1021/am900574m.
Non-patent Document 11: H. He, P. Yang, J. Li, R. Shi, L. Chen, A. Zhang and Y. Zhu, Controllable synthesis, characterization, and CO oxidation activity of CeO2 nanostructures with various morphologies, Ceram. Int., 2016, 42, 7810. DOI: 10.1016/j.ceramint.2016.02.005.
Non-patent Document 12: A. Yoko, G. Seong, T. Tomai and T. Adschiri, Continuous Flow Synthesis of Nanoparticles Using Supercritical Water: Process Design, Surface Control, and Nanohybrid Materials, KONA, 2020, 37, 28. DOI: 10.14356/kona.2020002.
Non-patent Document 13: Y. Omura, A. Yoko, G. Seong, T. Tomai and T. Adschiri, CrystEngComm., Mechanisms of the surface reaction and crystal growth of cerium oxide by supercritical hydrothermal treatment with carboxylic acids, 2021, 23, 5353. DOI: 10.1039/D1CE00720C.

Non-patent Document 14: G. Seong, M Dejhosseini, T Adschiri, A kinetic study of catalytic hydrothermal reactions of acetaldehyde with cubic CeO2 nanoparticles, Appl. Catal. A-Gen., 2018, 550, 284 DOI: 10.1016/j.apcata.2017.11.023.

Non-patent Document 15: M. Z. Hossain, D. Hojo, A. Yoko, G. Seong, N. Aoki, T. Tomai, S. Takami and T. Adschiri, Dispersion and rheology of nanofluids with various concentrations of organic modified nanoparticles: Modifier and solvent effects, Colloids Surf. A, 2019, 583, 123876. DOI: 10.1016/j.colsurfa.2019.123876.

Non-patent Document 16: A. Y Fadeev, R. Helmy, S. Marcinko, Langmuir, 2002, 18, 7521-7529.

Non-patent Document 17: M. Taguchi, S. Takami, T. Naka, T. Adschiri, Cryst. Growth Des., 2009, 9, 5297-5303.

Non-patent Document 18: S. Pawsey, K. Yach, J. Halla, L. Reven, Langmuir, 2000, 16, 3294-3303.

Non-patent Document 19: S. Usune, M. Ando, M. Kubo, T. Tsukada, K.-I. Sugioka, O. Koike, R. Tatsumi, M. Fujita, S. Takami, T. Adschiri, J. Chem. Eng. Japan, 2018, 51, 492-500.

Non-patent Document 20: S. J. Khan, F. Pierce, C. M. Sorensen, A. Chakrabarti, Langmuir, 2009, 25, 13861-13868.

Non-patent Document 21: C. J. Denis, C. J. Tighe, R. I. Gruar, N. M. Makwana, J. A. Darr, Cryst. Growth Des., 2015, 15, 4256-4265.

Non-patent Document 22: S. G. Kwon, Y Piao, J. Park, S. Angappane, Y Jo, N.-M. Hwang, J.-G. Park, T. Hyeon, J. Am. Chem. Soc., 2007, 129, 12571-12584.

Non-patent Document 23: B. H. Kim, K. Shin, S. G. Kwon, Y Jang, H.-S. Lee, H. Lee, S. W. Jun, J. Lee, S. Y Han, Y-H. Yim, D.-H. Kim, T. Hyeon, J. Am. Chem. Soc., 2013, 135, 2407-2410.

Non-patent Document 24: J. Zhang, T. Naka, S. Ohara, K. Kaneko, T. Trevethan, A. Shluger, T. Adschiri, Phys. Rev. B, 2011, 84, 045411.

Non-patent Document 25: L. Wu, H. J. Wiesmann, A. R. Moodenbaugh, R. F. Klie, Y Zhu, D. O. Welch, M. Suenaga Phys. Rev. B, 2004, 69, 125415.

Non-patent Document 26: S. Tsunekawa, S. Ito, Y Kawazoe, Appl. Phys. Lett., 2004, 85, 3845-3847.

Non-patent Document 27: F. Zhang, O. Jin, S.-W. Chan, J. Appl. Phys., 2004, 95, 4319-4326.

Non-patent Document 28: E. Beche, P. Charvin, D. Perarnau, S. Abanades, G. Flamant, Surf. Interface Anal., 2008, 40, 264-267.

Non-patent Document 29: T. Naganuma and E. Traversa, Nanoscale, 2012, 4, 4950-4953.

Non-patent Document 30: K. Yasunaga, K. Yasuda, S. Matsumura and T. Sonoda, Nucl. Instrum. Methods Phys. Res. B, 2008, 266, 2877-2881.

Non-patent Document 31: R. D. Singh, P. B. Koli, B. S. Jagdale and A. V. Patil, SN Applied Sciences., 2019, 1, 315.

Non-patent Document 32: V. V. Pujar and J. D. Cawley, J. Am. Ceram. Soc., 1995, 78, 774-782.

Non-patent Document 33: W.-S. Cho, E. Hamada and K. Takayanagi, J. Appl. Phys., 1997, 81, 3000-3002.

Non-Patent Document 34: A. Yoko, et al., Chem. Nano. Mat., 2022, 8, 4.

**Claims**

1. A method of producing metal oxide nanoparticles comprising:

   a mixing process for obtaining metal oxide nanoparticles by mixing a supercritical, subcritical, or gas phase aqueous material and an organometallic complex solution,
   wherein the mixing time is controllable within a range from 0.015 seconds to 380 seconds, and
   at least one of the average primary particle diameter or the crystallite diameter of the nanoparticles can be controlled within the range of 1.0 nm to 9.0 nm, and the coefficient of variation of the diameter can be controlled at 0.5 nm or less by controlling the mixing time.

2. The method according to claim 1,
   wherein the diameter and the coefficient of variation of the nanoparticles can be controlled by controlling the molar ratio of the organometallic complex to the organic material with respect to the metal constituting the organometallic complex.

3. The method according to claim 1 or 2,

   wherein the mixing temperature is controllable within a range from 300°C to 450°C, and
   the diameter and the said coefficient of variation of the nanoparticles can be controlled by controlling the mixing temperature.

4. The method according to any one of claims 1 to 3,

wherein when the metal elements comprising the organometallic complexes are metal elements that can take on multiple types of valence(s), the valence(s) of the metal elements comprising the organometallic complexes in said solution is controlled to be the same as that of the metal elements of the product.

5. The method according to any one of claims 1 to 4,

wherein the mixing process is a mixing process using a continuous reactor, and
the mixing time can be controlled within a range of 1 second or less by setting the Reynolds number (Re) to 3000 or more.

6. The method according to claim 5,

wherein a mixing speed $k_{mix}$ is obtained using Kolmogorov's theory,
the true reaction rate k is obtained from $1/k = 1/k_{app} - 1/k_{mix}$ using the mixing rate $k_{mix}$ and the apparent reaction rate $k_{app}$, and
the mixing time can be controlled within the range of 1 second or less by setting the Damkeller number $Da = k/k_{mix} \ll 1$ and the Re to 3000 or more.

7. The method according to claim 5 or 6,

the method further comprising a synthesis process for synthesizing the organometallic complexes,
wherein the synthesis process and the mixing process are continuous processes.

8. The method according to any one of claims 1 to 7,
the method further comprising a washing process in which the organometallic complexes are removed by washing the mixed product using the mixing process.

9. A metal oxide nanoparticle comprising:

a diameter of at least one of the average primary particle diameter or the crystallite diameter being between 1.0 nm and 9.0 nm, and the coefficient of variation of the diameter being 0.5 or less, and
the metal element constituting the metal oxide being a metal element capable of forming an organometallic complex.

10. An organically modified metal oxide nanoparticle comprising:

a diameter of at least one of the average primary particle diameter or the crystallite diameter being between 1.0 nm and 9.0 nm, and the coefficient of variation of the diameter being 0.5 or less, and
organic molecules being strongly bound to the most unstable surface.

Solution of metal-organic
complexes

Contact area
(Metal oxide nucleation)

Degassing and
pressurization

Increasing
pressure

Preheat

Reactor

Aqueous material

Schematic diagram of this method when realized in a continuous reaction system

# Fig. 1

【Cerium (IV) octanoate】

Fig. 2

# Fig. 3A

Piping from raw material solution

Contact area
(Metal oxide nucleation)

ノズル

Piping from aqueous material

# Fig. 3B

Contact area
(Metal oxide nucleation)

Piping from raw material solution

Piping from aqueous material

# Fig. 3C

Piping from aqueous material

Contact area
(Metal oxide nucleation)

Piping from raw material solution

# Fig. 3D

Piping from aqueous material

Piping from raw material solution

Contact area
(Metal oxide nucleation)

Add equal volume of methanol to benzene and wash (remove unreacted complexes)

# Fig. 4

Example 1-1
0.038s

Example 1-2
0.1s

Example 1-3
0.2s

Example 1-4
1s

Example 1-5
3s

Example 1-6
6.8s

Example 1-7
95s

Example 1-8
380s

TEM images of cerium oxide $(CeO_2)$ nanoparticles for Examples 1-1 to 1-8

# Fig. 5

Comparative Example 1-1 $(NH_4)_2Ce(NO_3)_6$ Comparative Example 1-2

Octanoic acid/Ce : 16  Octanoic acid/Ce : 69

TEM images of cerium oxide $(CeO_2)$ nanoparticles for Comparative Examples 1-1 and 1-2

Fig. 6

Variation of crystallite size of synthesized $CeO_2$ nanoparticles with reaction time

The inset shows a magnified view of the reaction time up to 12 seconds.

# Fig. 7

Volume change of synthesized CeO₂ nanoparticles with reaction time

# Fig. 8

Fig. 9A Example 1-1

5 nm

Fig. 9B Example 1-4

10 nm

Fig. 9C Example 1-5

10 nm

Fig. 9D Example 1-6

20 nm

Fig. 9E Example 1-7

20 nm

Fig 9F. Example 1-8

20 nm

HR-TEM images of synthesized $CeO_2$ nanoparticles

Illustration of method for calculating particle aspect ratio from HR-TEM images

# Fig. 10

Relation between particle length diameter and aspect ratio at different reaction times

# Fig. 11

0.04–1 s        1–3 s

3–i 6.8 s        6.8–380 s

Schematic diagram of the change in the mechanism of particle size increase with reaction time

Fig. 12

# Fig. 13A

Calculation results of the number of collisions
Relation between particle size and theoretical
collision time

# Fig. 13B

Calculation results of the number of collisions
Relationship between reaction time and
theoretical number of collisions in real data

Relationship between reaction time and particle coefficient of variation

# Fig. 14

Relationship between the molar ratio of octanoic acid to cerium and the average primary particle size of cerium oxide ($CeO_2$) nanoparticles produced.

# Fig. 15

Example 3-1: 340°C

Octanoic acid/Ce : 16     Octanoic acid/Ce : 69

Example 3-2: 300°C

Octanoic acid/Ce : 16     Octanoic acid/Ce : 69

TEM images of cerium oxide (CeO$_2$) nanoparticles for Examples 3-1 and 3-2

# Fig. 16

# Fig. 17A  Fig. 17B  Fig. 17C

(a) 340°C OA=0.12 mol/L

(b) 340°C OA=0.52 mol/L

(C) 300°C OA=0.12 mol/L

Effect of modifier concentration and reaction temperature on organically modified $CeO_2$

TEM images at reaction times of 95s (340° C) and 106s (300° C)

Effect of modifier concentration and reaction temperature on the crystallite size of organically modified $CeO_2$

# Fig. 18

## Fig. 19A   Fig. 19B   Fig. 19C

## Fig. 19D   Fig. 19E   Fig. 19F

TEM image of CeO$_2$ nanoparticles at 340° C, OA=0.52 mol/L

# Fig. 20A  Fig. 20B  Fig. 20C

# Fig. 20D  Fig. 20E  Fig. 20F

TEM image of $CeO_2$ nanoparticles at 300° C, OA=0.12 mol/L

# Fig. 21A

# Fig. 21B

Variation of collision frequency factor $\beta$
with synthesis conditions and particle size

Schematic diagram of dN/dt
calculation method

Fig. 22A

Fig. 22B

Estimation of Activation Energy

Calculation of $\beta$ at 3 nm

Arrhenius blot with calculated $\beta$

Relationship between reaction time to obtain cerium oxide ($CeO_2$) nanoparticles and conversion rate to $CeO_2$ nanoparticles

# Fig. 23

# Fig. 24A

# Fig. 24B

Characterisation of precursors.

Fig 24A: Ce $L_3$-Edge XANES spectra for Ce(III) octanoate, Ce(IV) octanoate, and $CeO_2$. $CeO_2$ was purchased from ITEC Corp.

Fig 24B: XRD patterns for Ce(III) octanoate, Ce(IV) octanoate, and $CeO_2$

XRD patterns for products synthesised from Ce(III) and (IV) octanoates.
JCPDS Card no. 00-001-0800 as $CeO_2$, and 01-074-0665 as $Ce(OH)_3$.

# Fig. 25

FT-IR spectra for products synthesised from Ce(III) and (IV) octanoates.

# Fig. 26

# Fig. 27A    Fig. 27B    Fig. 27C

TEM images of products.

Fig. 27A: Low-magnification image of products synthesised from Ce(III) octanoate.

Fig. 27B: High-magnification image of products synthesised from Ce(III) octanoate.

Fig. 27C: Image of products synthesised from Ce(IV) octanoate.

# Fig. 28A

# Fig. 28B

TGA curve for product synthesised from Ce(IV) octanoate in batch reactor.

Product synthesised from Ce(IV) octanoate dissolved in cyclohexane at 1 wt.%.

XRD patterns for samples synthesised from Ce(III) octanoate by changing reaction time.
JCPDS Card no. 00-001-0800 as $CeO_2$, and 01-074-0665 as $Ce(OH)_3$.

# Fig. 29

## Fig. 30A

## Fig. 30B

## Fig. 30C

## Fig. 30D

TEM images of products synthesised from Ce(III) octanoate by changing reaction time : (Fig. 30A) 5, (Fig. 30B) 10, (Fig. 30C) 60, and (Fig. 30D) 360 min.

CeO$_2$ formation routes from Ce-complex considered in this study.

# Fig. 31

# Fig. 32A  Fig. 32B

Effect of octanoic acid on supercritical hydrothermal treatment of Ce(III) octanoate.
Fig. 32A; Without octanoic acid; 0.143 g of Ce(III) octanoate and 2.5 mL of water were added.
Fig. 32B; With octanoic acid; 0.143 g of Ce(III) octanoate, 1.25 mL of water, and 1.25 mL of octanoic acid were added.

FT-IR spectra for Ce(III) octanoate, Ce(IV) octanoate, and extracted material.

# Fig. 33

Particle size distribution of octanoic-acid-modified $CeO_2$ nano-particles synthesised from Ce(IV) octanoate and $Ce(OH)_4$.
Average particle size (Ave.) and relative standard deviation (RSD) were written.

# Fig. 34

TEM image of CeO$_2$ synthesised from Ce(OH)$_4$.

# Fig. 35

IR spectra of the synthesized tetravalent cerium carboxylate complexes

Fig. 36

XANES spectra of the synthesized tetravalent cerium carboxylate complexes

# Fig. 37

XRD pattern of the synthesized tetravalent cerium carboxylate complex

# Fig. 38

XRD pattern of synthesized organically modified $CeO_2$

# Fig. 39

XRD pattern change by classification operation of C14-CeO$_2$

Fig. 40

XRD crystallite size of $CeO_2$ nanoparticles with different modifier molecular chain lengths
All at a reaction temperature of 340° C and modifier concentration of 0.12 mol/L

# Fig. 41

Schematic diagram showing the effect of modifier molecular chain length
on interaction potential
V: Potential energy, r: Distance between particle centers, d: Particle core diameter

Fig. 42

Fig. 43A    Fig. 43B    Fig. 43C

(a) 0.04 s

50 nm

(b) 1 s

50 nm

(c) 3 s

50 nm

(d) 8 s

50 nm

(e) 95 s

50 nm

(f) 380 s

50 nm

Fig. 43D    Fig. 43E    Fig. 43F

TEM image of hexanoic acid modified CeO$_2$ (C6-CeO$_2$)

Fig. 44A    Fig. 44B    Fig. 44C

Fig. 44D    Fig. 44E    Fig. 44F

TEM image of decanoic acid modified $CeO_2$ ($C10-CeO_2$)

# Fig. 45A　Fig. 45B　Fig. 45C

(a) 0.04 s　50 nm

(b) 1 s　50 nm

(c) 3 s　50 nm

(d) 8 s　50 nm

(e) 95 s　50 nm

(f) 380 s　50 nm

# Fig. 45D　Fig. 45E　Fig. 45F

TEM image of myristic acid modified $CeO_2$ ($C14-CeO_2$)

Effect of precursor Ce concentration on XRD crystallite size

# Fig. 46

(a) Ce 0.0075 mol/L, 0.04 s

(b) Ce 0.0025 mol/L, 0.04 s

(c) Ce 0.0013 mol/L, 0.04 s

(d) Ce 0.0005mol/L, 0.04 s

50 nm

Particle image at 0.04s reaction time with varying precursor Ce concentration

# Fig. 47

Effect of Reynolds number on XRD crystallite size at reaction time of 7s

# Fig. 48

# Fig. 49A    Fig. 49B    Fig. 49C

(a) Re = 11300

(b) Re = 7100

(c) Re = 3100

(d) Re = 1500

(e) Re = 805

# Fig. 49D    Fig. 49E

TEM images of $CeO_2$ nanoparticles synthesized at different Reynolds numbers at 7s reaction time

Effect of crystallite size on the lattice parameter of synthesized CeO$_2$ nanoparticles

# Fig. 50

# Fig. 51A

(a)      340°C OA=0.12 mol/L

95 s

8 s

3 s

1 s

0.2 s

0.1 s

0.04 s

Intensity

$2\theta$ (deg)

# Fig. 51B

(b)      300°C OA=0.12 mol/L

106 s

9 s

3.3 s

1.1 s

0.2 s

0.1 s

0.04 s

Intensity

$2\theta$ (deg)

# Fig. 51C

(c)      340°C OA=0.52 mol/L

95 s

8 s

3 s

1 s

0.2 s

0.1 s

0.04 s

Intensity

$2\theta$ (deg)

XRD patterns of synthesized $CeO_2$ nanoparticles

XPS spectra measurement results of CeO$_2$ nanoparticles

# Fig. 52

Variation of Ce$^{3+}$ ratio versus crystallite diameter

# Fig. 53

X-ray emission spectra of CeO₂ nanoparticles

# Fig. 54

Comparison of XRD patterns of $CeO_2$ nanoparticles
Control $Ce(OH)_4$ was measured on purchased product
Control $CeO_2$: JCPDS No. 01-089-8436
Control $Ce_2O_3$: JCPDS No. 01-072-6357

# Fig. 55

Variation of 20° peak intensity ratio versus crystallite diameter

# Fig. 56

Results of $Ce_2O_3$ ratio calculation by XRD pattern fitting

# Fig. 57

Overview of distribution reactor

# Fig. 58

Reaction rate of $ZrO_2$ (reaction temperature, OA/Zr ratio)

# Fig. 59

# Fig. 60A  Fig. 60B

# Fig. 60C  Fig. 60D

TEM image of $ZrO_2$ (300° C, OA/Zr=16)
Fig. 60A: 0.029 s, Fig. 60B: 0.068 s, Fig. 60C: 0.89 s, Fig. 60D: 8.4 s,

Fig. 61A    Fig. 61B

Fig. 61C    Fig. 61D

TEM image of $ZrO_2$ (300° C, OA/Zr=40)
Fig. 61A: 0.029 s, Fig. 61B: 0.068 s, Fig. 61C: 0.89 s, Fig. 61D: 8.4 s,

# Fig. 62A    Fig. 62B

# Fig. 62C    Fig. 62D

TEM image of $ZrO_2$ (340° C, OA/Zr=16)
Fig. 62A: 0.032 s, Fig. 62B: 0.20 s, Fig. 62C: 1.0 s, Fig. 62D: 9.4 s,

# Fig. 63A  Fig. 63B

# Fig. 63C  Fig. 63D

TEM image of $ZrO_2$ (340° C, OA/Zr=40)
Fig. 63A: 0.032 s, Fig. 63B: 0.20 s, Fig. 63C: 1.0 s, Fig. 63D: 9.4 s,

# Fig. 64A  Fig. 64B

# Fig. 64C  Fig. 64D

TEM image of $ZrO_2$ (380° C, OA/Zr=16)
Fig. 64A: 0.041 s, Fig. 64B: 0.096 s, Fig. 64C: 1.3 s, Fig. 64D: 12 s,

# Fig. 65A  Fig. 65B

(a)

20 nm

(b)

20 nm

(c)

20 nm

(d)

20 nm

# Fig. 65C  Fig. 65D

TEM image of $ZrO_2$ (380° C, OA/Zr=40)
Fig. 65A: 0.041 s, Fig. 65B: 0.096 s, Fig. 65C: 1.3 s, Fig. 65D: 12 s,

Particle size variation with residence time (300° C)

# Fig. 66

Particle size variation with residence time (340° C)

# Fig. 67

Particle size variation with residence time (380° C)

# Fig. 68

XRD pattern of $ZrO_2$ (300° C, OA/Zr=16)

# Fig. 69

XRD pattern of ZrO$_2$ (300° C, OA/Zr=40)

# Fig. 70

XRD pattern of $ZrO_2$ (340° C, OA/Zr=16)

# Fig. 71

XRD pattern of $ZrO_2$ (340° C, OA/Zr=40)

# Fig. 72

XRD pattern of $ZrO_2$ (380° C, OA/Zr=16)

# Fig. 73

XRD pattern of ZrO$_2$ (380° C, OA/Zr=40)

# Fig. 74

Crystallite size variation with residence time (300° C)

# Fig. 75

Crystallite size variation with residence time (340° C)

# Fig. 76

Crystallite size variation with residence time (380° C)

# Fig. 77

Variation of mZrO$_2$ ratio against residence time (300° C)

# Fig. 78

Variation of mZrO$_2$ ratio against residence time (340° C)

Fig. 79

Variation of mZrO$_2$ ratio against residence time (380° C)

# Fig. 80

Variation of mZrO$_2$ ratio to crystallite size (300° C)

# Fig. 81

Variation of $mZrO_2$ ratio to crystallite size (340° C)

# Fig. 82

Variation of $mZrO_2$ ratio to crystallite size (380° C)

# Fig. 83

XPS spectrum of ZrO$_2$
Fig. 84A: 0.068s, Fig. 84B: 8.4s
(300° C, OA/Zr=16)

IR spectrum of $ZrO_2$ (OA/Zr=40)

# Fig. 85

Variation of surface modification rate of $ZrO_2$ with grain size （300° C）

# Fig. 86

Variation of surface modification rate of $ZrO_2$ with grain size (340° C)

# Fig. 87

Variation of surface modification rate of $ZrO_2$ with grain size （380° C）

# Fig. 88

Reaction rate for each particle synthesis

# Fig. 89

# Fig. 90A  Fig. 90B

# Fig. 90C  Fig. 90D

TEM image of $Ce_{0.9}Zr_{0.1}O_2$
Fig. 90A: 0.032s, Fig. 90B: 0.20s, Fig. 90C: 1.0s, Fig. 90D: 9.4s,

Fig. 91A  Fig. 91B

Fig. 91C  Fig. 91D

TEM image of $Ce_{0.1}Zr_{0.9}O_2$
Fig. 91A: 0.032s, Fig. 91B: 0.20s, Fig. 91C: 1.0s, Fig. 91D: 9.4s,

Particle size change for each particle with respect to residence time

# Fig. 92

XRD pattern of $Ce_{0.9}Zr_{0.1}O_2$

# Fig. 93

XRD pattern of $Ce_{0.1}Zr_{0.9}O_2$

# Fig. 94

Crystallite size variation of $Ce_{0.9}Zr_{0.1}O_2$ and $CeO_2$ versus residence time

# Fig. 95

Crystallite size variation of $Ce_{0.1}Zr_{0.9}O_2$ and $ZrO_2$ versus residence time

# Fig. 96

Variation of the ratio of $mZrO_2$ to crystallite size

# Fig. 97

XPS spectrum of $Ce_{0.9}Zr_{0.1}O_2$ (Ce3d)
(a) 0.032s (crystallite size 2.4nm), (b) 9.4s (crystallite size 5.2nm)

# Fig. 98

Ratio of Ce$^{3+}$ on particle surface to crystallite size ($Ce_{0.9}Zr_{0.1}O_2$)

# Fig. 99

XPS spectrum of $Ce_{0.1}Zr_{0.9}O_2$ (Zr3d)
(a) 0.032s (crystallite size 1.4nm), (b) 9.4s (crystallite size 3.5nm)

# Fig. 100

Variation of surface modification ratio for each particle size

# Fig. 101

Crystallite size change versus residence time $(CeO_2)$

# Fig. 102

Crystallite size dependence of $\beta$ (CeO$_2$, reaction temperature 340° C)

# Fig. 103

Crystallite size dependence of unit lattice volume $(CeO_2)$

# Fig. 104

Crystallite size change versus residence time $(ZrO_2)$

# Fig. 105

Crystallite size dependence of $\beta$ ($ZrO_2$)

# Fig. 106

mZrO$_2$ dependence of $\beta$ (reaction temperature 340° C)

# Fig. 107

Crystallite size dependence of unit lattice volume$(ZrO_2)$

# Fig. 108

Unit lattice volume dependence of $\beta$ (tZrO$_2$)

# Fig. 109

Unit lattice volume dependence of $\beta$ (mZrO$_2$)

# Fig. 110

## INTERNATIONAL SEARCH REPORT

| International application No. |
| --- |
| **PCT/JP2023/005326** |

**A. CLASSIFICATION OF SUBJECT MATTER**

*C01F 17/235*(2020.01)i; *B82Y 30/00*(2011.01)i; *B82Y 40/00*(2011.01)i
FI: C01F17/235; B82Y30/00; B82Y40/00

According to International Patent Classification (IPC) or to both national classification and IPC

**B. FIELDS SEARCHED**

Minimum documentation searched (classification system followed by classification symbols)

C01F17/235; B82Y30/00; B82Y40/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2011-224558 A (SEOUL NATIONAL UNIVERSITY INDUSTRY FOUNDATION) 10 November 2011 (2011-11-10) claims 1-12, paragraphs [0087]-[0089], fig. 20 | 9-10 |
| A | | 1-8 |
| A | JP 2012-197195 A (TOHOKU UNIVERSITY) 18 October 2012 (2012-10-18) claims 1-3, paragraphs [0046]-[0064] | 1-10 |
| A | WO 2015/046496 A1 (FUJIFILM CORP.) 02 April 2015 (2015-04-02) entire text, all drawings | 1-10 |
| A | JP 2006-525930 A (EIDGENOESSISCHE TECHNISCHE HOCHSCHULE ZUERICH) 16 November 2006 (2006-11-16) entire text, all drawings | 1-10 |

☐ Further documents are listed in the continuation of Box C.    ☑ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **11 April 2023** | **25 April 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/005326**

| Patent document cited in search report | | | Publication date (day/month/year) | Patent family member(s) | | | Publication date (day/month/year) |
|---|---|---|---|---|---|---|---|
| JP | 2011-224558 | A | 10 November 2011 | US 2006/0133990 A1 claims 1-12, paragraphs [0107]-[0109], fig. 20 WO 2006/057467 A1 EP 1661649 A1 CN 101090785 A KR 10-2007-0102672 A | | | |
| JP | 2012-197195 | A | 18 October 2012 | (Family: none) | | | |
| WO | 2015/046496 | A1 | 02 April 2015 | US 2016/0203894 A1 entire text, all drawings CN 105593169 A KR 10-2016-0047510 A | | | |
| JP | 2006-525930 | A | 16 November 2006 | US 2006/0229197 A1 entire text, all drawings WO 2004/103900 A1 EP 1626929 A1 CN 1787964 A | | | |

Form PCT/ISA/210 (patent family annex) (January 2015)

## REFERENCES CITED IN THE DESCRIPTION

### Patent documents cited in the description

- JP 3925936 B

### Non-patent literature cited in the description

- **A. YOKO** ; **Y TANAKA** ; **G. SEONG** ; **D. HOJO** ; **T. TOMAI** ; **T. ADSCHIRI**. Mixing and Solvent Effects on Kinetics of Supercritical Hydrothermal ynthesis: Reaction of Nickel Nitrate to Nickel Oxide. *J. Phys. Chem. C*, 2020, vol. 124, 4772-4780
- **N. AOKI** ; **A. SATO** ; **H. SASAKI** ; **A. LITWINOWICZ** ; **G. SEONG** ; **T. AIDA** ; **D. HOJO** ; **S. TAKAMI** ; **T. ADSCHIRI**. Kinetics study to identify reaction-controlled conditions forsupercritical hydrothermal nanoparticle synthesis with flow-typereactors. *J. of Supercritical Fluids*, 2016, vol. 110, 161-166
- **M. SMOLUCHOWSKI**. *Phys. Z.*, 1916, vol. 17, 557-585
- **M. KOBAYASHI** ; **Y ADACHI**. *Journal of the Agricultural Engineering Society, Japan*, 1998, vol. 66, 959-964
- **S. TANIGUCHI** ; **A. KIKUCHI**. *Tetsu-to-Hagane*, 1992, vol. 78, 527-535
- **T. FUJII** ; **S. KAWASAKI** ; **A. SUZUKI** ; **T. ADSCHIRI**. HighSpeed Morphology Control of Boehmite Nanoparticles by Supercritical Hydrothermal Treatment with Carboxylic Acids. *Cryst. Growth Des.*, 2016, vol. 16, 1996
- **Y. TAKAHASHI** ; **H. SHIMIZU** ; **A. USUI** ; **H. KAGI** ; **M. NOMURA**. Direct observation of tetravalent cerium in ferromanganese nodules and crusts by X-ray-absorption near-edge structure (XANES). *Geochim. Cosmochim. Acta*, 2000, vol. 64, 2929
- **A. BIANCONI** ; **A. MARCELLI** ; **H. DEXPERT** ; **R. KAMATAK** ; **A. KOTANI** ; **T. JO** ; **J. PETIAU**. Specific intermediate-valence state of insulating 4f compounds detected by L3 x-ray absorption. *Phys. Rev. B*, 1987, vol. 35, 806
- **M. TAGUCHI** ; **N. YAMAMOTO** ; **D. HOJO** ; **S. TAKAMI** ; **T. ADSCHIRI** ; **T. FUNAZUKURI** ; **T. NAKA**. Synthesis of monocarboxylic acid-modified CeO2 nanoparticles using supercritical water. *RSC Adv.*, 2014, vol. 4, 49605
- **M. K. DEVARAJU** ; **S. YIN** ; **T. SATO**. Morphology Control of Cerium Oxide Particles Synthesized via a Supercritical Solvothermal Method. *ACS Appl. Mater. Interfaces*, 2009, vol. 1, 2694

- **H. HE** ; **P. YANG** ; **J. LI** ; **R. SHI** ; **L. CHEN** ; **A. ZHANG** ; **Y. ZHU**. Controllable synthesis, characterization, and CO oxidation activity of CeO2 nanostructures with various morphologies. *Ceram. Int.*, 2016, vol. 42, 7810
- **A. YOKO** ; **G. SEONG** ; **T. TOMAI** ; **T. ADSCHIRI**. Continuous Flow Synthesis of Nanoparticles Using Supercritical Water: Process Design, Surface Control, and Nanohybrid Materials. *KONA*, 2020, vol. 37, 28
- **Y. OMURA** ; **A. YOKO** ; **G. SEONG** ; **T. TOMAI** ; **T. ADSCHIRI**. Mechanisms of the surface reaction and crystal growth of cerium oxide by supercritical hydrothermal treatment with carboxylic acids. *CrystEngComm.*, 2021, vol. 23, 5353
- **G. SEONG** ; **M DEJHOSSEINI** ; **T ADSCHIRI**. A kinetic study of catalytic hydrothermal reactions of acetaldehyde with cubic CeO2 nanoparticles. *Appl. Catal. A-Gen.*, 2018, vol. 550, 284
- **M. Z. HOSSAIN** ; **D. HOJO** ; **A. YOKO** ; **G. SEONG** ; **N. AOKI** ; **T. TOMAI** ; **S. TAKAMI** ; **T. ADSCHIRI**. Dispersion and rheology of nanofluids with various concentrations of organic modified nanoparticles: Modifier and solvent effects. *Colloids Surf. A*, 2019, vol. 583, 123876
- **A. Y FADEEV** ; **R. HELMY** ; **S. MARCINKO**. *Langmuir*, 2002, vol. 18, 7521-7529
- **M. TAGUCHI** ; **S. TAKAMI** ; **T. NAKA** ; **T. ADSCHIRI**. *Cryst. Growth Des.*, 2009, vol. 9, 5297-5303
- **S. PAWSEY** ; **K. YACH** ; **J. HALLA** ; **L. REVEN**. *Langmuir*, 2000, vol. 16, 3294-3303
- **S. USUNE** ; **M. ANDO** ; **M. KUBO** ; **T. TSUKADA** ; **K.-I. SUGIOKA** ; **O. KOIKE** ; **R. TATSUMI** ; **M. FUJITA** ; **S. TAKAMI** ; **T. ADSCHIRI**. *J. Chem. Eng. Japan*, 2018, vol. 51, 492-500
- **S. J. KHAN** ; **F. PIERCE** ; **C. M. SORENSEN** ; **A. CHAKRABARTI**. *Langmuir*, 2009, vol. 25, 13861-13868
- **C. J. DENIS** ; **C. J. TIGHE** ; **R. I. GRUAR** ; **N. M. MAKWANA** ; **J. A. DARR**. *Cryst. Growth Des.*, 2015, vol. 15, 4256-4265
- **S. G. KWON** ; **Y PIAO** ; **J. PARK** ; **S. ANGAPPANE** ; **Y JO** ; **N.-M. HWANG** ; **J.-G. PARK** ; **T. HYEON**. *J. Am. Chem. Soc.*, 2007, vol. 129, 12571-12584

- **B. H. KIM** ; **K. SHIN** ; **S. G. KWON** ; **Y JANG** ; **H.-S. LEE** ; **H. LEE** ; **S. W. JUN** ; **J. LEE** ; **S. Y HAN** ; **Y-H. YIM**. *J. Am. Chem. Soc.*, 2013, vol. 135, 2407-2410
- **J. ZHANG** ; **T. NAKA** ; **S. OHARA** ; **K. KANEKO** ; **T. TREVETHAN** ; **A. SHLUGER** ; **T. ADSCHIRI**. *Phys. Rev. B*, 2011, vol. 84, 045411
- **L. WU** ; **H. J. WIESMANN** ; **A. R. MOODENBAUGH** ; **R. F. KLIE** ; **Y ZHU** ; **D. O. WELCH** ; **M. SUENAGA**. *Phys. Rev. B*, 2004, vol. 69, 125415
- **S. TSUNEKAWA** ; **S. ITO** ; **Y KAWAZOE**. *Appl. Phys. Lett.*, 2004, vol. 85, 3845-3847
- **F. ZHANG** ; **O. JIN** ; **S.-W. CHAN**. *J. Appl. Phys.*, 2004, vol. 95, 4319-4326
- **E. BECHE** ; **P. CHARVIN** ; **D. PERARNAU** ; **S. ABANADES** ; **G. FLAMANT**. *Surf. Interface Anal.*, 2008, vol. 40, 264-267
- **T. NAGANUMA** ; **E. TRAVERSA**. *Nanoscale*, 2012, vol. 4, 4950-4953
- **K. YASUNAGA** ; **K. YASUDA** ; **S. MATSUMURA** ; **T. SONODA**. *Nucl. Instrum. Methods Phys. Res. B*, 2008, vol. 266, 2877-2881
- **R. D. SINGH** ; **P. B. KOLI** ; **B. S. JAGDALE** ; **A. V. PATIL**. *SN Applied Sciences.*, 2019, vol. 1, 315
- **V. V. PUJAR** ; **J. D. CAWLEY**. *J. Am. Ceram. Soc.*, 1995, vol. 78, 774-782
- **W.-S. CHO** ; **E. HAMADA** ; **K. TAKAYANAGI**. *J. Appl. Phys.*, 1997, vol. 81, 3000-3002
- **A. YOKO et al.** *Chem. Nano. Mat.*, 2022, vol. 8, 4